(19) Europäisches Patentamt
European Patent Office
Office européen des brevets

(11) EP 2 030 971 B1

(12) **EUROPEAN PATENT SPECIFICATION**

(45) Date of publication and mention of the grant of the patent:
**12.10.2011 Bulletin 2011/41**

(21) Application number: **07767294.7**

(22) Date of filing: **20.06.2007**

(51) Int Cl.:
***C07D 213/74*** (2006.01) ***A01N 43/40*** (2006.01)
***A01N 43/42*** (2006.01) ***A01N 43/54*** (2006.01)
***A01N 43/56*** (2006.01) ***A01N 43/60*** (2006.01)
***A01N 43/78*** (2006.01) ***A01N 43/80*** (2006.01)
***A01N 43/836*** (2006.01) ***A01N 43/84*** (2006.01)
***A01N 43/90*** (2006.01)

(86) International application number:
**PCT/JP2007/062456**

(87) International publication number:
**WO 2007/148738 (27.12.2007 Gazette 2007/52)**

(54) **PEST CONTROL AGENT CONTAINING NOVEL PYRIDYL-METHANAMINE DERIVATIVE OR SALT THEREOF**

SCHÄDLINGSBEKÄMPFUNGSMITTEL MIT NEUEM PYRIDYL-METHANAMIN-DERIVAT ODER SALZ DARAUS

AGENT DE LUTTE ANTIPARASITE CONTENANT UN NOUVEAU DÉRIVÉ DE PYRIDYL-MÉTHANAMINE OU UN SEL DE CELUI-CI

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **20.06.2006 JP 2006170283**

(43) Date of publication of application:
**04.03.2009 Bulletin 2009/10**

(73) Proprietor: **ISHIHARA SANGYO KAISHA, LTD.**
**Osaka-shi**
**Osaka 550-0002 (JP)**

(72) Inventors:
- **KIMURA, Hirohiko**
  **Kusatsu-shi**
  **Shiga 525-0025 (JP)**
- **MORITA, Masayuki**
  **Kusatsu-shi**
  **Shiga 525-0025 (JP)**
- **YAMAMOTO, Kazuhiro**
  **Kusatsu-shi**
  **Shiga 525-0025 (JP)**
- **UEKI, Toshihiko**
  **Kusatsu-shi**
  **Shiga 525-0025 (JP)**
- **AZUMA, Kumiko**
  **Kusatsu-shi**
  **Shiga 525-0025 (JP)**
- **HAMAMOTO, Taku**
  **Kusatsu-shi**
  **Shiga 525-0025 (JP)**

(74) Representative: **Hartz, Nikolai**
**Wächtershäuser & Hartz**
**Patentanwaltspartnerschaft**
**Weinstrasse 8**
**80333 München (DE)**

(56) References cited:
**WO-A1-02/066470 JP-A- 03 197 460**
**JP-A- 05 504 569**

- **MATSUMOTO K. ET AL.: 'Synthesis of functionalized pyridines by aminolysis of 2,6-dichloropyridines' CHEMISTRY EXPRESS vol. 7, no. 6, 1992, pages 473 - 476, XP008102436**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

TECHNICAL FIELD

The present invention relates to a novel pyridyl-methanamine derivative or its salt, and a pesticide containing it as an active ingredient.

BACKGROUND ART

Patent Documents 1 to 3 disclose pyridine derivatives having specific chemical structures respectively. However, the compound disclosed in Patent Document 1 and the compound disclosed in Patent Document 2 are different from the pyridyl-methanamine derivative of the present invention in the $R^4$ moiety and the $R^5$ moiety in the after-mentioned formula (I), respectively. Whereas, Patent Document 3 does not specifically disclose the pyridyl-methanamine derivative of the present invention. Further, the compounds disclosed in Patent Documents 1 to 3 are all compounds for medical or pharmaceutical uses and not compounds for pesticides.
Patent Document 4 relates to arthropodicidal trichloromethylbenzylamines. Patent Document 5 relates to nitro-substituted heterocyclic compounds, processes for their preparation, and their use as insecticides.
Non-Patent Document 1 discloses N-(2-pyridylmethyl)-3,5,6-trichloro-4-(trifluoromethyl)-2-pyridylamine which is a compound contained in the after-mentioned formula (I), but discloses no pesticide containing such a compound as an active ingredient.

Patent Document 1: WO01/62233
Patent Document 2: WO02/66470
Patent Document 3: WO04/91518
Patent Document 4: WO91/12228
Patent Document 5: JP3-197460 A
Non-Patent Document 1: Chemistry Express 7, 473-476 (1992)

DISCLOSURE OF THE INVENTION

PROBLEMS TO BE SOLVED BY THE INVENTION

For many years, many pesticides have been used, but many of them have various problems such that the effects are inadequate, their use is restricted as pests have acquired resistance, etc. Accordingly, it is desired to develop a novel pesticide substantially free from such problems, for example, a pesticide capable of controlling various pests which create problems in agricultural and horticultural fields or a pesticide capable of controlling pests parasitic on animals.

MEANS TO SOLVE THE PROBLEMS

The present inventors have conducted various studies on pyridyl-methanamine derivative in an effort to find a superior pesticide. As a result, they have found that a novel pyridyl-methanamine derivative has an extremely high pesticidal effect against pests at a low dose and at the same time has safety to crop plants, the natural enemy to pests, or mammals, and have accomplished the present invention.
Namely, the present invention relates to a pyridyl-methanamine derivative represented by the formula (I) or its salt:

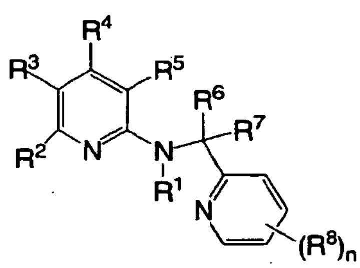

( I )

wherein $R^1$ is hydrogen, alkyl which may be substituted by $R^b$, alkenyl which may be substituted by $R^b$, alkynyl which

may be substituted by $R^b$, aryl, cyano, N=CH$R^c$, O$R^c$, $S(O)_pR^c$, COS$R^c$, COO$R^c$, CO$R^c$, or a heterocyclic group which may be substituted by alkyl or haloalkyl; each of $R^2$ and $R^3$ which are independent of each other, is hydrogen, halogen, cyano, nitro, alkyl which may be substituted by $R^8$, cycloalkyl, alkenyl, alkynyl, aryl, a heterocyclic group, $NR^aR^c$, $OR^a$, $SR^a$, $COR^a$, $COOR^a$, $CONR^aR^c$, CH=NO$R^a$, $SO_2R^a$ or $SOR^a$; $R^4$ is trifluoromethyl or chlorodifluoromethyl; $R^5$ is hydrogen, halogen, cyano, nitro, alkyl which may be substituted by $R^8$, alkenyl which may be substituted by $R^8$, alkynyl which may be substituted by $R^8$, $OR^a$, $SR^a$, $NR^aR^c$, $COOR^a$ or $COR^a$; each of $R^6$ and $R^7$ which are independent of each other, is hydrogen, cyano, alkyl, haloalkyl or cycloalkyl, or $R^6$ and $R^7$ may together form $C_{3-6}$ cycloalkyl which may be substituted by halogen; $R^8$ is alkyl, cycloalkyl, alkoxyalkyl, alkoxyalkoxyalkyl, hydroxyalkyl, halogen, haloalkyl, cyano, nitro, aryl which may be substituted by halogen, a heterocyclic group which may be substituted by halogen, heterocyclic oxy which may be substituted by halogen, $CONR^aR^c$, $COR^c$, $COOR^c$, $NR^aR^c$ or $OR^a$; $R^a$ is hydrogen, alkyl, cycloalkyl, haloalkyl or heterocyclic alkyl; $R^b$ is halogen, aryl which may be substituted by $R^8$, a heterocyclic group which may be substituted by $R^8$, heterocyclic oxy which may be substituted by $R^8$, heterocyclic thio which may be substituted by $R^8$, cyano, $NR^aR^c$, NHCOO$R^a$, $COR^c$, $COOR^c$, $CONR^aR^c$, alkoxyalkoxy, $OR^a$ or $S(O)_pR^a$; $R^c$ is hydrogen, alkyl, haloalkyl, cycloalkyl, alkoxyalkyl, hydroxyalkyl, aryl which may be substituted by halogen, or a heterocyclic group which may be substituted by haloalkyl; n is an integer of from 0 to 4, p is an integer of from 0 to 2, in the $NR^aR^c$ moiety in each of the above substituents, $R^a$ and $R^c$ may together form a 5- or 6-membered heterocyclic ring together with the nitrogen atom to which they are bonded as defined in claim 1. The present invention also relates to a method for producing a pyridyl-methanamine derivative or its salt, as defined in claim 5; a pesticide comprising, as an active ingredient, a pyridyl-methanamine derivative or its salt, as defined in claim 6; and a use of a pyridyl-methanamine for controlling a pest, as defined in claim 10

EFFECTS OF THE INVENTION

A pesticide containing the pyridyl-methanamine derivative of the above formula (I) as an active ingredient, has a very high pesticidal effect against pets at a low dose.

BEST MODE FOR CARRYING OUT THE INVENTION

As the halogen or halogen as the substituent in the formula (I), an atom of fluorine, chlorine, bromine or iodine may be mentioned. The number of halogens as the substituents may be 1 or more, and if more, the respective halogens may be the same or different. Further, the positions for substitution of such halogens may be any positions:

The alkyl or an alkyl moiety in the alkoxy in the formula (I) may be linear or branched. As its specific example, $C_{1-6}$ alkyl such as methyl, ethyl, propyl, isopropyl, butyl, tert-butyl, pentyl or hexyl may be mentioned.

As the cycloalkyl in the formula (I), $C_{3-6}$ cycloalkyl such as cyclopropyl, cyclobutyl, cyclopentyl or cyclohexyl may, for example, be mentioned.

The alkenyl in the formula (I) may be linear or branched. As its specific example, $C_{2-6}$ alkenyl such as vinyl, 1-propenyl, allyl, isopropenyl, 1-butenyl, 1,3-butadienyl or 1-hexenyl may be mentioned.

The alkynyl in the formula (I) may be linear or branched. As its specific example, $C_{2-6}$ alkynyl such as ethynyl, 2-butynyl, 2-pentynyl, 3-methyl-1-butynyl, 2-penten-4-ynyl or 3-hexynyl may be mentioned.

As the aryl in the formula (I), $C_{6-10}$ aryl such as phenyl or naphthyl may, for example, be mentioned.

The heterocyclic group or a heterocyclic moiety in the heterocyclic alkyl, the heterocyclic oxy or the heterocyclic thio in the formula (I) includes a fused heterocyclic group in addition to a monocyclic heterocyclic group. The monocyclic heterocyclic group may, for example, be a 3-membered heterocyclic group such as oxiranyl; a 5-membered heterocyclic group such as furyl, tetrahydrofuryl, thienyl, pyrrolyl, pyrrolinyl, pyrrolidinyl, dioxolanyl, oxazolyl, isoxazolyl, dihydroisoxazolyl, thiazolyl, isothiazolyl, imidazolyl, imidazolinyl, imidazolidinyl, pyrazolyl, pyrazolinyl, pyrazolidinyl, triazolyl, oxadiazolyl, thiadiazolyl, tetrazolyl, 1,3-dioxolanyl, 1,3-oxathiolanyl or 1,3-oxathiolanyl-3-oxide; or a 6-membered heterocyclic group such as pyranyl, pyridyl, piperidinyl, dioxanyl, oxazinyl, morpholinyl, thiazinyl, pyridazinyl, pyrimidinyl, pyrazinyl, piperazinyl, triazinyl, 1,3-dioxanyl, tetrahydropyranyl, 2H-1,4-oxathiinyl or 1,3-dithioranyl. Among such monocyclic heterocyclic groups, preferred is a 5- or 6-membered monocyclic heterocyclic group containing from 1 to 4 atoms of at least one type selected from the group consisting of O, S and N. The fused heterocyclic group may, for example, be benzofuranyl, isobenzofuranyl, dihydrobenzofuranyl, dihydroisobenzofuranyl, benzothienyl, isobenzothienyl, dihydrobenzothienyl, dihydroisobenzothienyl, tetrahydrobenzothienyl, indolyl, isoindolyl, benzoxazolyl, benzothiazolyl, indazolyl, benzimidazolyl, benzodioxolanyl, benzodioxanyl, chromenyl, chromanyl, isochromanyl, chromonyl, chromanonyl, quinolyl, isoquinolyl, cinnolinyl, phthalazinyl, quinazolinyl, quinoxalinyl, indolizinyl, quinolizinyl, imidazopyridyl, naphthyridinyl, pteridinyl, dihydrobenzoxazinyl, dihydrobenzoxazolinonyl, dihydrobenzoxazinonyl, benzothioxanyl or imidazopyridinyl. Among such fused heterocyclic groups, preferred is a 8- to 10-membered fused heterocyclic group containing from 1 to 4 atoms of at least one type selected from the group consisting of O, S and N.

In the $NR^aR^c$ moiety in each of the substituents in the formula (I), $R^a$ and $R^c$ may together form a 5- or 6-membered heterocyclic ring together with the nitrogen atom to which they are bonded. Such a 5- or 6-membered

heterocyclic ring may further contain, in addition to the nitrogen atom to which $R^a$ and $R^c$ are bonded, at least one hetero atom. Such a heterocyclic ring may, for example, be pyrrolidinyl, pyrazolidinyl, piperazinyl or morpholinyl. Further, the $C_{3-6}$ cycloalkyl to be formed by $R^6$ and $R^7$ in the formula (I) may be cycloalkyl such as cyclopropyl, cyclobutyl, cyclopentyl or cyclohexyl, which may be substituted by a halogen atom.

The salt of the pyridyl-methanamine derivative represented by the above formula (I) includes all kinds so long as they are agriculturally acceptable. For example, an alkali metal salt such as a sodium salt or a potassium salt; an alkaline earth metal salt such as a magnesium salt or a calcium salt; an ammonium salt such as a dimethylammonium salt or a triethylammonium salt; an inorganic acid salt such as a hydrochloride, a perchlorate, a sulfate or a nitrate; or an organic salt such as an acetate or a methanesulfonate., may be mentioned.

The pyridyl-methanamine derivative represented by the above formula (I) may have optical isomers or geometrical isomers, and such isomers and mixtures thereof are both included in the present invention. In the present description, isomers are disclosed as mixtures, unless otherwise specified. Further, in the present invention, various isomers other than those mentioned above, may be included within the scope of the common knowledge in this technical field. Further, depending upon the type of such an isomer, the chemical structure may be different from the above-mentioned formula (I), but it is obvious to one skilled in the art that such a structure is in isomeric relation and thus falls within the scope of the present invention.

The pyridyl-methanamine derivative represented by the above formula (I) or its salt can be produced by the following production processes [1] to [10] and in accordance with a usual method for producing a salt.

PRODUCTION PROCESS [1]

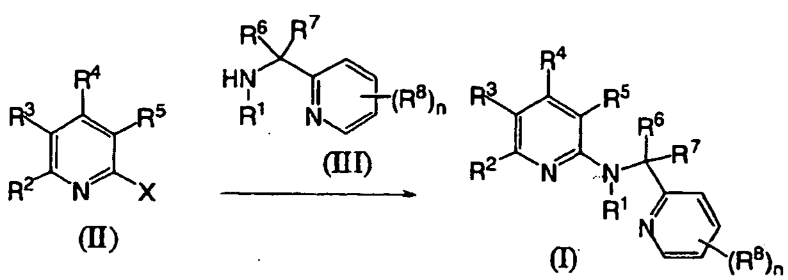

$R^1$, $R^2$, $R^3$, $R^4$, $R^5$, $R^6$, $R^7$, $R^8$ and n are as defined above; and X is halogen, and the halogen may be an atom of fluorine , chlorine, bromine or iodine.

The reaction for the production process [1] may be carried out in the presence of a solvent.

The solvent may be any solvent so long as it is inert to the reaction. For example, it may be an alcohol such as methanol, ethanol, propanol or butanol; an aromatic hydrocarbon such as benzene, toluene or xylene; an aliphatic hydrocarbon such as pentane, hexane, heptane, petroleum ether, ligroin or petroleum benzine; an ether such as diethyl ether, dipropyl ether, dibutyl ether, tetrahydrofuran or dioxane; an ester such as methyl acetate or ethyl acetate; a nitrile such as acetonitrile or propionitrile; an acid amide such as dimethylformamide or dimethylacetamide; a sulfoxide such as dimethylsulfoxide; a sulfone such as sulfolane; a phosphoric acid amide such as hexamethylphosphoramide; a halogenated hydrocarbon such as chloroform, dichloromethane, carbon tetrachloride or 1,2-dichloroethane; or a mixed solvent thereof.

In the reaction for the production process [1], in order to carry out the reaction efficiently, the reaction may be carried out in the presence of a base, as the case requires. Such a base may, for example, be an organic base such as triethylamine or pyridine; an alkali metal hydroxide such as sodium hydroxide or potassium hydroxide; an alkali metal carbonate such as lithium carbonate, sodium carbonate or potassium carbonate; an alkali metal hydrogencarbonate such as lithium hydrogencarbonate, sodium hydrogencarbonate or potassium hydrogencarbonate; an alkali metal hydride such as lithium hydride, sodium hydride or potassium hydride; or an alkali metal alkoxide such as sodium methoxide, sodium ethoxide or potassium tertiary butoxide.

In the reaction for the production process [1], the compound of the formula (III) can be used in a proportion of from 0.8 to 5 equivalents, preferably from 1 to 2.5 equivalents, to 1 mol of the compound of the formula (II).

The reaction for the production process [1] is carried out usually at a reaction temperature of from 0 to 150°C, preferably from 0 to 100°C. The reaction time is usually from 0.5 to 100 hours.

Various conditions for reaction in the production process [1] may suitably mutually be combined. Further, among these conditions for the reaction, there are reaction conditions of usual ranges and reaction conditions of preferred

ranges, and they may also suitably mutually be selected and combined.

PRODUCTION PROCESS [2]

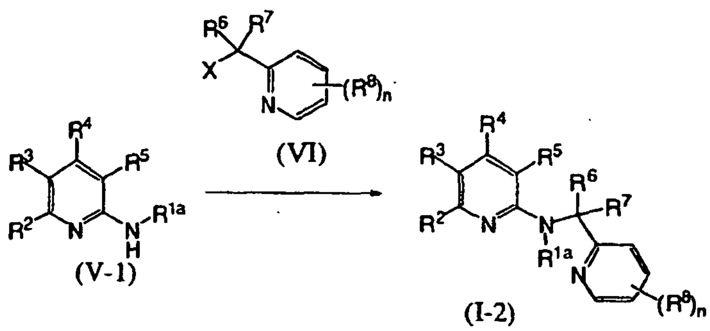

$R^{1a}$ is alkyl which may be substituted by $R^b$, alkenyl which may be substituted by $R^b$, alkynyl which may be substituted by $R^b$, aryl, a heterocyclic group which may be substituted by alkyl or haloalkyl, N=CHR$^c$, OR$^c$, S(O)$_p$R$^c$, COSR$^c$, COOR$^c$ or COR$^c$, and $R^b$, $R^c$, p, $R^2$, $R^3$, $R^4$, $R^5$, $R^6$, $R^7$, $R^8$, n and X are as defined above.

The reaction for the production process [2] can be carried out in the presence of a base and a solvent.

The base may, for example, be an alkali metal hydride such as sodium hydride or potassium hydride; an alkali metal hydroxide such as sodium hydroxide or potassium hydroxide; an alkali metal such as sodium or potassium; an alkali metal alkoxide such as sodium methoxide, sodium ethoxide or potassium tertiary butoxide; an alkali metal carbonate such as sodium carbonate or potassium carbonate; an alkali metal hydrogencarbonate such as sodium hydrogencarbonate or potassium hydrogencarbonate; or an organic base such as triethylamine or pyridine. The base may be used in an amount of from 1 to 3 equivalents, preferably from 1 to 1.5 equivalents, to the compound of the formula (V-1).

The solvent may be any solvent so long as it is inert to the reaction, and it may, for example, be an alcohol such as methanol, ethanol, propanol or butanol; an aromatic hydrocarbon such as benzene, toluene or xylene; an aliphatic hydrocarbon such as pentane, hexane, heptane, petroleum ether, ligroin or petroleum benzine; an ether such as diethyl ether, dipropyl ether, dibutyl ether, tetrahydrofuran or dioxane; an ester such as methyl acetate or ethyl acetate; a nitrile such as acetonitrile or propionitrile; an acid amide such as dimethylformamide or dimethylacetamide; a sulfoxide such as dimethylsulfoxide; a sulfone such as sulfolane; a phosphoric acid amide such as hexamethylphosphoramide; a halogenated hydrocarbon such as chloroform, dichloromethane, carbon tetrachloride or 1,2-dichloroethane; or a mixed solvent thereof.

In the reaction for the production process [2], the compound of the formula (VI) can be used in a proportion of from 0.8 to 2 equivalents to 1 mol of the compound of the formula (V-1).

The reaction for the production process [2] is carried out usually at a temperature of from 0 to 100°C, preferably from 0 to 50°C. The reaction time is usually from 0.5 to 24 hours, preferably from 0.5 to 5 hours.

Various conditions for the reaction in the production process [2] may suitably mutually be combined. Further, among these various conditions for the reaction, there are reaction conditions of usual ranges and reaction conditions of preferred ranges, and they may also suitably mutually be selected and combined.

PRODUCTION PROCESS [3]

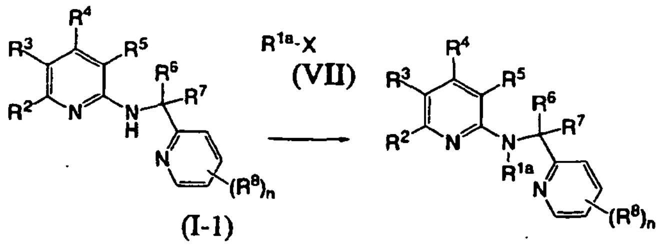


(I-1)

(I-2)

$R^{1a}$, $R^2$, $R^3$, $R^4$, $R^5$, $R^6$, $R^7$, $R^8$, n and X are as defined above.

The reaction for the production process [3] can be carried out in the presence of a base and a solvent.

The base may, for example, be an alkali metal hydride such as sodium hydride or potassium hydride; an alkali metal hydroxide such as sodium hydroxide or potassium hydroxide; an alkali metal such as sodium or potassium; an alkali metal alkoxide such as sodium methoxide, sodium ethoxide or potassium tertiary butoxide; an alkali metal carbonate such as sodium carbonate or potassium carbonate; an alkali metal hydrogencarbonate such as sodium hydrogencarbonate or potassium hydrogencarbonate; or an organic base such as triethylamine or pyridine. The base may be used in an amount of from 1 to 3 equivalents, preferably from 1 to 1.5 equivalents, to the compound of the formula (I-1).

The solvent may be any solvent so long as it is inert to the reaction, and it may, for example, be an alcohol such as methanol, ethanol, propanol or butanol; an aromatic hydrocarbon such as benzene, toluene or xylene; an aliphatic hydrocarbon such as pentane, hexane, heptane, petroleum ether, ligroin or petroleum benzine; an ether such as diethyl ether, dipropyl ether, dibutyl ether, tetrahydrofuran or dioxane; an ester such as methyl acetate or ethyl acetate; a nitrile such as acetonitrile or propionitrile; an acid amide such as dimethylformamide or dimethylacetamide; a sulfoxide such as dimethylsulfoxide; a sulfone such as sulfolane; a phosphoric acid amide such as hexamethylphosphoramide; a halogenated hydrocarbon such as chloroform, dichloromethane, carbon tetrachloride or 1,2-dichloroethane; or a mixed solvent thereof.

In the reaction for the production process [3], the compound of the formula (VII) may be used in a proportion of from 0.8 to 2 equivalents to 1 mol of the compound of the formula (I-1).

The reaction for the production process [3] is carried out at a reaction temperature of usually from 0 to 100°C, preferably from 0 to 50°C. The reaction time is usually from 0.5 to 24 hours, preferably from 0.5 to 5 hours.

Various conditions for the reaction in the production process [3] may suitably mutually be combined. Further, among these conditions for the reaction, there are reaction conditions of usual ranges and reaction conditions of preferred ranges, but they may also suitably mutually be selected and combined.

PRODUCTION PROCESS [4]

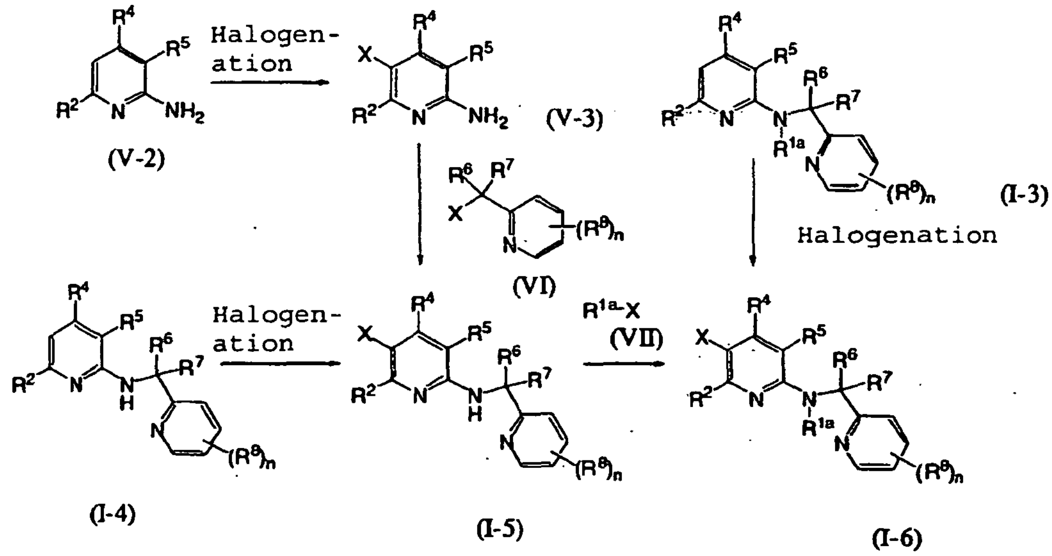


(V-2) (V-3) (I-3) (VI) (I-4) (I-5) (I-6)

$R^{1a}$, $R^2$, $R^4$, $R^5$, $R^6$, $R^7$, $R^8$, n and x are as defined above.

The halogenation reaction in the production process [4] may be carried out in the presence of a solvent by using a halogenating agent.

The halogenating agent may, for example, be chlorine, bromine, iodine, N-chlorosuccinimide, N-bromosuccinimide or N-iodosuccinimide. The halogenating agent may be used in an amount of from 1 to 2 equivalents, preferably from 1 to 1.5 equivalent, to 1 mol of each of the compounds of the formulae (V-2), (1-3) and (1-4).

The solvent may be any solvent so long as it is inert to the reaction, and it may, for example, be an acid amide such as dimethylformamide or dimethylacetamide; a nitrile such as acetonitrile, propionitrile or acrylonitrile; a halogenated hydrocarbon such as chloroform, dichloromethane, carbon tetrachloride or 1,2-dichloroethane; an aromatic hydrocarbon such as benzene, toluene or xylene; an ester such as methyl acetate or ethyl acetate; an organic acid such as acetic acid; or a mixed solvent thereof.

The halogenation reaction is carried out usually at a reaction temperature of from 0 to 150°C, preferably from 20 to 100°C. The reaction time is usually from 0.5 to 24 hours, preferably from 0.5 to 12 hours.

The reaction of the compound of the formula (V-3) with the compound of the formula (VI) in the production process [4] can be carried out in the same manner as the method in the above production process [2].

The reaction of the compound of the formula (I-5) with the compound of the formula (VII) in the production process [4], can be carried out in the same manner as in the method in the above production process [3].

PRODUCTION PROCESS [5]

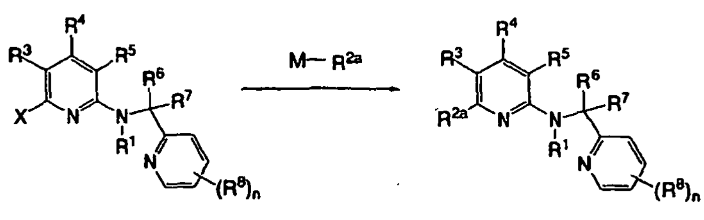


(I-7) (I-8)

$R^1$, $R^3$, $R^4$, $R^5$, $R^5$, $R^7$, $R^8$, n and X are as defined above; $R^{2a}$ is alkyl which may be substituted by $R^8$, cycloalkyl, alkenyl, alkynyl, aryl or a heterocyclic group; and M is a leaving group to generate $R^{2a}$, such as copper, boron, zinc, magnesium, lithium, tin or silicon.

The reaction for the production process [5] may be carried out by using a compound represented by the formula

M-$R^{2a}$, in the presence of a base.

The compound represented by the formula M-$R^{2a}$ may, for example, be an organic copper compound, an organic boron compound, an organic zinc compound, an organic magnesium compound, an organic lithium compound, an organic tin compound or an organic silicon compound. Such a compound may be used in an amount of from 1 to 3 equivalents, preferably from 1 to 1.5 equivalents, to 1 mol of the compound of the formula (1-7).

The base may, for example, be an alkali metal hydride such as sodium hydride or potassium hydride; an alkali metal hydroxide such as sodium hydroxide or potassium hydroxide; an alkali metal alkoxide such as sodium methoxide, sodium ethoxide or potassium tertiary butoxide; an alkali metal carbonate such as sodium carbonate, potassium carbonate; an alkali metal hydrogencarbonate such as sodium hydrogencarbonate or potassium hydrogencarbonate; or an organic base such as triethylamine or pyridine.

For the reaction for the production process [5], in order to carry out the reaction efficiently, it is possible to employ a catalyst such as a palladium compound or a nickel compound, as the case requires.

The reaction for the production process [5] may be carried out in the presence of a solvent, as the case requires. The solvent may be any solvent so long as it is inert to the reaction, and it may, for example, be an alcohol such as methanol, ethanol, propanol or butanol; a ketone such as acetone, methyl ethyl ketone, dimethyl ketone, diethyl ketone or methyl isobutyl ketone; an aromatic hydrocarbon such as benzene, toluene or xylene; an aliphatic hydrocarbon such as pentane, hexane, heptane, petroleum ether, ligroin or petroleum benzine; an ether such as diethyl ether, dipropyl ether, dibutyl ether, tetrahydrofuran or dioxane; an ester such as methyl acetate or ethyl acetate; a nitrile such as acetonitrile or propionitrile; an acid amide such as dimethylformamide or dimethylacetamide; a sulfoxide such as dimethylsulfoxide; a sulfone such as sulfolane; a phosphoric acid amide such as hexamethylphosphoramide; a halogenated hydrocarbon such as chloroform, dichloromethane, carbon tetrachloride or 1,2-dichloroethane; water; or a mixed solvent thereof.

The reaction for the production process [5] is carried out at a reaction temperature of usually from 0 to 200˚C, preferably from 20 to 120˚C. The reaction time is usually from 0.5 to 24 hours.

Various conditions for the reaction in the production process [5] may suitably mutually be combined. Further, among such various conditions for the reaction, there are reaction conditions of usual ranges and reaction conditions of preferred ranges, but they may also suitably mutually be selected and combined.

PRODUCTION PROCESS [6]

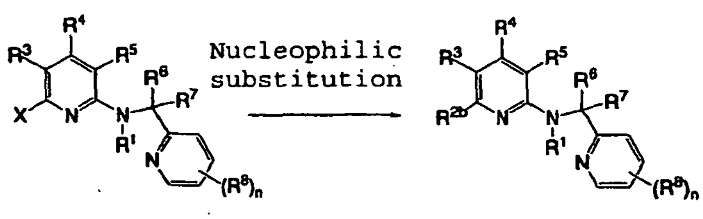


(I-7) (I-9)

$R^1$, $R^3$, $R^4$, $R^5$, $R^6$, $R^7$, $R^8$, n and X are as defined above, and $R^{2b}$ is $NR^aR^c$, $OR^a$ or $SR^a$.

The nucleophilic substitution reaction for the production process [6] may be carried out in the presence of a solvent by using a nucleophilic reagent.

The nucleophilic reagent may, for example, be an alkali metal alkoxide such as sodium methoxide or sodium ethoxide; an alkali metal mercaptide such as sodium methylmercaptan; or a primary or secondary amine such as methylamine, dimethylamine or piperidine. Such a nucleophilic reagent may be used in an amount of from 1 to 5 equivalents, preferably from 1 to 3 equivalents, to 1 mol of the compound of the formula (1-7).

The solvent may be any solvent so long as it is inert to the reaction, and it may, for example, be an alcohol such as methanol, ethanol, propanol or butanol; a ketone such as acetone, methyl ethyl ketone, dimethylketone, diethylketone or methyl isobutyl ketone; an aromatic hydrocarbon such as benzene, toluene or xylene; an aliphatic hydrocarbon such as pentane, hexane, heptane, petroleum ether, ligroin or petroleum benzine; an ether such as diethyl ether, dipropyl ether, dibutyl ether, tetrahydrofuran or dioxane; an ester such as methyl acetate or ethyl acetate; a nitrile such as acetonitrile or propionitrile; an acid amide such as dimethylformamide or dimethylacetamide; a sulfoxide such as dimethylsulfoxide; a sulfone such as sulfolane; a phosphoric acid amide such as hexamethylphosphoramide; a

halogenated hydrocarbon such as chloroform, dichloromethane, carbon tetrachloride or 1,2-dichloroethane; water; or a mixed solvent thereof.

The nucleophilic substitution reaction for the production process [6] is carried out at a reaction temperature of usually from 0 to 200˚C, preferably from 0 to 100˚C. The reaction time is usually from 0.5 to 24 hours.

Various conditions for the reaction in the production process [6] may suitably mutually be combined. Further, among such various conditions for the reaction, there are the reaction conditions of usually ranges and reaction conditions of preferred ranges, but they may also suitably mutually be selected and combined.

PRODUCTION PROCESS [7]

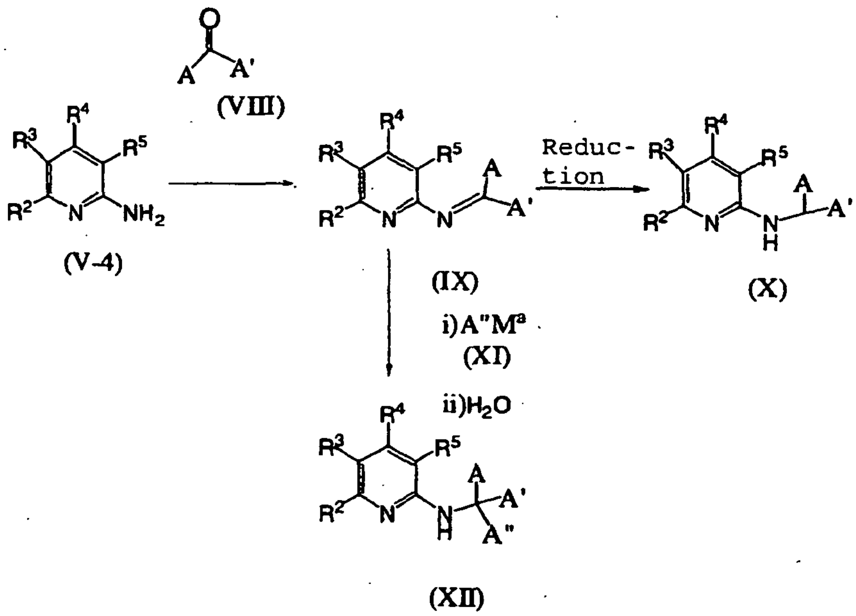


Each of A and A' which are independent of each other, is hydrogen, cyano, alkyl, haloalkyl, cycloalkyl, aryl or a heterocyclic group which may be substituted by $R^8$; A" is alkyl, alkenyl, haloalkyl, cycloalkyl or cyano; $M^a$ is a magnesium halide, a metal or a leaving group to generate $CN^-$; and $R^2$, $R^3$, $R^4$, $R^5$ and $R^8$ are as defined above.

The compound of the formula (IX) can be produced by subjecting the compound of the formula (V-4) and the compound of the formula (VIII) to a condensation reaction in a solvent.

The solvent may be any solvent so long as it is inert to the reaction, and it may, for example, be an alcohol such as methanol, ethanol, propanol or butanol; an aromatic hydrocarbon such as benzene, toluene or xylene; an aliphatic hydrocarbon such as pentane, hexane, heptane, petroleum ether, ligroin or petroleum benzine; an ether such as diethyl ether, dipropyl ether, dibutyl ether, tetrahydrofuran or dioxane; an ester such as ethyl acetate or methyl acetate; an acid amide such as dimethylformamide or dimethylacetamide; a sulfoxide such as dimethylsulfoxide; a sulfone such as sulfolane; a phosphoric acid amide such as hexamethylphosphoramide; a halogenated hydrocarbon such as chloroform, dichloromethane, carbon tetrachloride or 1,2-dichloroethane; or a mixed solvent thereof.

In order to carry out the above condensation reaction efficiently, an acid catalyst may be used as the case requires. The acid catalyst may, for example, be an inorganic acid such as hydrochloric acid or sulfuric acid; or an organic acid such as acetic acid, camphor sulfonic acid, p-toluenesulfonic acid or pyridinium p-toluene sulfonate.

In the condensation reaction, the compound of the formula (VIII) may be used in a proportion of from 1 to 2 equivalents, preferably from 1.2 to 1.5 equivalents, to 1 mol of the compound of the formula (V-4).

The condensation reaction is carried out at a reaction temperature of usually from 0 to 150˚C, preferably from 50 to 100˚C. The reaction time is usually from 5 to 100 hours.

Various conditions for the condensation reaction may suitably mutually be combined. Further, among such various conditions for the reaction, there are reaction conditions of usual ranges and reaction conditions of preferred ranges, but they may also suitably mutually be selected and combined.

The compound of the formula (X) can be produced by reacting a compound of the formula (IX) with a reducing agent in a solvent.

The reducing agent may, for example, be a metal hydride such as lithium aluminum hydride, sodium borohydride, sodium cyanoborohydride; or a hydrosilane such as triethylsilane or trichlorosilane. Further, it is also possible to select a method of employing ammonium formate as a reducing agent in catalytic reduction or Leuckart-Wallach reaction.

The solvent may be any solvent so long as it is inert to the reaction, and it may, for example, be an alcohol such as methanol, ethanol, propanol or butanol; an aromatic hydrocarbon such as benzene, toluene or xylene; an aliphatic hydrocarbon such as pentane, hexane, heptane, petroleum ether, ligroin or petroleum benzine; an ether such as diethyl ether, dipropyl ether, dibutyl ether, tetrahydrofuran or dioxane; an acid amide such as dimethylformamide or dimethylacetamide; a sulfoxide such as dimethylsulfoxide; a sulfone such as sulfolane; a phosphoric acid amide such as hexamethylphosphoramide; an ester such as ethyl acetate or methyl acetate; a halogenated hydrocarbon such as chloroform, dichloromethane, carbon tetrachloride or 1,2-dichloroethane; or a mixed solvent thereof.

The above reduction reaction is carried out usually at a reaction temperature of from 0 to 100°C, preferably from 0 to 40°C. The reaction time is usually from 1 to 40 hours.

Various conditions for the reduction reaction may suitably mutually be combined. Further, among such various conditions for the reaction, there are reaction conditions of usual ranges and reaction conditions of preferred ranges, and they may also suitably mutually be selected and combined.

The compound of the formula (XII) can be produced by reacting the compound of the formula (IX) with the compound of the formula (XI) in a solvent, followed by hydrolysis by a usual method.

The compound of the formula (XI) may, for example, be, when A" is alkyl, alkenyl, haloalkyl or cycloalkyl, a Grignard reagent, such as an alkylmagnesium halide such as methylmagnesium bromide or isopropylmagnesium chloride, an alkenyl magnesium halide such as allyl magnesium bromide, a haloalkylmagnesium halide such as trifluoromethylmagnesium bromide, or a cycloalkylmagnesium halide such as cyclopropylmagnesium bromide; an alkyllthium such as methyllithium or butyllithium; an alkyl zinc or dialkyl zinc such as methylzinc, ethylzinc or diethylzinc. Further, when A" is cyano, a cyanide compound such as hydrogen cyanide, trimethylsilyl cyanide or tributyltin cyanide may, for example, be mentioned. The compound of the formula (XI) may be used in a proportion of usually from 1 to 4 equivalents, preferably from 1.2 to 1.5 equivalents, to 1 mol of the compound of the formula (IX).

The solvent may be any solvent so long as it is inert to the reaction, and it may, for example, be an alcohol such as methanol, ethanol, propanol or butanol; an aromatic hydrocarbon such as benzene, toluene or xylene; an aliphatic hydrocarbon such as pentane, hexane, heptane, petroleum ether, ligroin or petroleum benzine; an ether such as diethyl ether, dipropyl ether, dibutyl ether, tetrahydrofuran or dioxane; a phosphoric acid amide such as hexamethylphosphoramide; a halogenated hydrocarbon such as chloroform, dichloromethane, carbon tetrachloride or 1,2-dichloroethane; and a mixed solvent thereof.

This reaction is carried out usually at a reaction temperature of from 0 to 100°C, preferably from 0 to 40°C. The reaction time is usually from 1 to 50 hours.

PRODUCTION PROCESS [8]

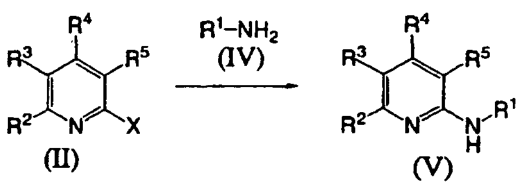


$R^1$, $R^2$, $R^3$, $R^4$, $R^5$ and X are as defined above.

The reaction for the production process [8] can be carried out in the presence of a solvent.

The solvent may, for example, be an alcohol such as methanol, ethanol, propanol or butanol; an aromatic hydrocarbon such as benzene, toluene or xylene; an aliphatic hydrocarbon such as pentane, hexane, heptane, petroleum ether, ligroin or petroleum benzine; an ether such as diethyl ether, dipropyl ether, dibutyl ether, tetrahydrofuran or dioxane; an ester such as methyl acetate or ethyl acetate; a nitrile such as acetonitrile or propionitrile; an acid amide such as dimethylformamide or dimethylacetamide; a sulfoxide such as dimethylsulfoxide; a sulfone such as sulfolane; a phosphoric acid amide such as hexamethylphosphoramide; a halogenated hydrocarbon such as chloroform, dichloromethane, carbon tetrachloride or 1,2-dichloroethane; or a mixed solvent thereof.

In the reaction for the production process [8], in order to carry out the reaction efficiently, the reaction may be carried out in the presence of a base, as the case requires. Such a base may, for example, be an organic base such as triethylamine or pyridine, an alkali metal hydroxide such as sodium hydroxide or potassium hydroxide; an alkali metal

carbonate such as lithium carbonate, sodium carbonate or potassium carbonate; an alkali metal hydrogencarbonate such as lithium hydrogencarbonate, sodium hydrogencarbonate, or potassium hydrogencarbonate; an alkali metal hydride such as lithium hydride, sodium hydride or potassium hydride; or an alkali metal alkoxide such as sodium methoxide, sodium ethoxide or potassium tertiary butoxide.

In the reaction for the production process[8], the compound of the formula (IV) may be used in a proportion of from 0.8 to 5 equivalents, preferably from 1 to 2.5 equivalents to 1 mol of the compound of the above formula (II).

The reaction for the production process [8] is carried out usually at a reaction temperature of from 0 to 150°C, preferably from 0 to 100°C. The reaction time is usually from 0.5 to 100 hours.

Various conditions for the reaction in the production process [8] may suitably mutually be combined. Further, among such conditions for the reaction, there are reaction conditions of usual ranges and reaction conditions of preferred ranges, and they may also suitably mutually be selected and combined.

PRODUCTION PROCESS [9]

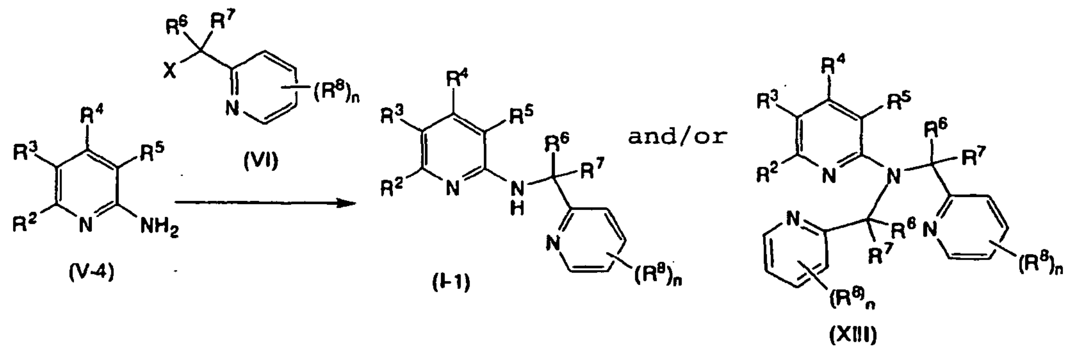

$R^2$, $R^3$, $R^4$, $R^5$, $R^6$, $R^7$, $R^8$, n and X are as defined above.

The reaction for the production process [9] can be carried out in the presence of a base and a solvent.

The base may, for example, be an alkali metal hydride such as sodium hydride or potassium hydride; an alkali metal hydroxide such as sodium hydroxide or potassium hydroxide; an alkali metal such as sodium or potassium; an alkali metal alkoxide such as sodium methoxide, sodium ethoxide or potassium tertiary butoxide; an alkali metal carbonate such as sodium carbonate or potassium carbonate; an alkali metal hydrogencarbonate such as sodium hydrogencarbonate or potassium hydrogencarbonate; or an organic base such as triethylamine or pyridine. The base may be used in an amount of from 1 to 3 equivalents to 1 mol of the compound of the formula (V-4). In order to obtain the compound of the formula (I-1), the base is preferably used in an amount of from 1 to 1.5 equivalents, and in order to obtain the compound of the formula (XIII), the base is preferably used in an amount of from 2 to 2.5 equivalents.

The solvent may be any solvent so long as it is inert to the reaction, and it may, for example, be an alcohol such as methanol, ethanol, propanol or butanol; an aromatic hydrocarbon such as benzene, toluene or xylene; an aliphatic hydrocarbon such as pentane, hexane, heptane, petroleum ether, ligroin or petroleum benzine, an ether such as diethyl ether, dipropyl ether, dibutyl ether, tetrahydrofuran or dioxane; an ester such as methyl acetate or ethyl acetate; a nitrile such as acetonitrile or propionitrile; an acid amide such as dimethylformamide or dimethylacetamide; a sulfoxide such as dimethylsulfoxide; a sulfone such as sulfolane; a phosphoric acid amide such as hexamethylphosphoramide; a halogenated hydrocarbon such as chloroform, dichloromethane, carbon tetrachloride or 1,2-dichloroethane; or a mixed solvent thereof.

In the reaction for the production process [9], the compound of the formula (VI) may be used in a proportion of from 0.8 to 2.5 equivalents to 1 mol of the compound of the above formula (V-4). In order to obtain the compound of the formula (I-1), the compound of the formula (VI) is preferably used in an amount of from 0.8 to 1.5 equivalents, and in order to obtain the compound of the formula (XIII), the compound of the formula (VI) is preferably used in an amount of from 2 to 2.5 equivalents.

The reaction for the production process [9] is carried out usually at a reaction temperature of from 0 to 100°C, preferably from 0 to 50°C. The reaction time is usually from 0.5 to 24 hours.

Various conditions for the reaction in the production process [9] may suitably mutually be combined. Further, among such various conditions for the reaction, there are reaction conditions of usual ranges and reaction conditions of preferred ranges, and they may also suitably mutually be selected and combined.

PRODUCTION PROCESS [10]

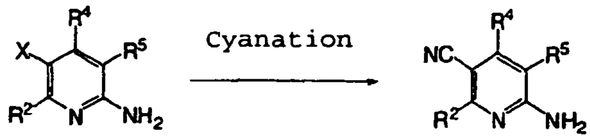


(V-3) (V-4a)

$R^2$, $R^4$, $R^5$ and X are as defined above.
The reaction for the production process [10] can be carried out by using a cyanating agent in the presence of a solvent.
The cyanating agent may, for example, be copper cyanide, zinc cyanide, sodium cyanide, trimethylsilyl cyanide or tributyltin cyanide, but copper cyanide is preferred.
The solvent may, for example, be an alcohol such as methanol, ethanol, propanol or butanol; an aromatic hydrocarbon such as benzene, toluene or xylene; an aliphatic hydrocarbon such as pentane, hexane, heptane, petroleum ether, ligroin or petroleum benzine, an ether such as diethyl ether, dipropyl ether, dibutyl ether, tetrahydrofuran or dioxane; an ester such as methyl acetate or ethyl acetate; a nitrile such as acetonitrile or propionitrile; an acid amide such as dimethylformamide or dimethylacetamide; a sulfoxide such as dimethylsulfoxide; a sulfone such as sulfolane; a phosphoric acid amide such as hexamethylphosphoramide; a halogenated hydrocarbon such as chloroform, dichloromethane, carbon tetrachloride or 1,2-dichloroethane; or a mixed solvent thereof.
In the reaction for the production process [10], the cyanating agent may be used in a proportion of from 0.8 to 5 equivalents, preferably from 1 to 2.5 equivalents, to 1 mol of the compound of the above formula (V-3).
The reaction for the production process [10] is carried out usually at a reaction temperature of from 80 to 200°C, preferably from 100 to 150°C. The reaction time is usually from 1 to 24 hours.
Various conditions for the reaction in the production process [10] may suitably mutually be combined. Further, among such various conditions for the reaction, there are reaction conditions of usual ranges and reaction conditions of preferred ranges, and they may also suitably mutually be selected and combined.
Preferred embodiments of pesticides containing the compounds of the present invention (which are hereinafter in this specification meant for all compounds represented by the formula (1) unless otherwise specified) will be described below. The pesticides containing the compounds of the present invention are particularly useful, for example, as agents for controlling various pests which become problematic in the agricultural and horticultural fields, i.e. agricultural and horticultural pesticides, or as agents for controlling pests which are parasitic on animals, i.e. pesticides against parasites on animals.
The agricultural and horticultural pesticides containing the compounds of the present invention are useful as an insecticide, a miticide, a nematicide or a soil pesticide, and they are effective for controlling plant parasitic mites such as two-spotted spider mite (Tetranychus urticae), carmine spider mite (Tetranychus cinnabarinus), kanzawa spider mite (Tetranychus kanzawai), citrus red mite (Panonychus citri), European red mite (Panonychus ulmi), broad mite (Polyphagotarsonemus latus), pink citrus rust mite (Aculops pelekassi) and bulb mite (Rhizoglyphus echinopus); aphids such as green peach aphid (Myzus persicae) and cotton aphid (Aphis gossypii); agricultural insect pests such as diamondback moth (Plutella xylostella), cabbage armyworm (Mamestra brassicae), common cutworm (Spodoptera litura), codling moth (Laspeyresia pomonella), bollworm (Heliothis zea), tobacco budworm (Heliothis virescens), gypsy moth (Lymantria dispar), rice leafroller (Cnaphalocrocis medinalis), Adoxophyes sp., colorado potato beetle (Leptinotarsa decemlineata), cucurbit leaf beetle (Aulacophora femoralis), boll weevil (Anthonomus grandis), planthoppers, leafhoppers, scales, bugs, whiteflies, thrips, grasshoppers, anthomyiid flies, scarabs, black cutworm (Agrotis ipsilon), cutworm (Agrotis segetum) and ants; plant parasitic nematodes such as root-knot nematodes, cyst nematodes, root-lesion nematodes, rice white-tip nematode (Aphelenchoides besseyi), strawberry bud nematode (Nothotylenchus acris), pine wood nematode (Bursaphelenchus lignicolus); gastropods such as slugs and snails; soil pests such as isopods such as pillbugs (Armadilidium vulgare) and pillbugs (Porcellio scaber); hygienic insect pests such as tropical rat mite (Ornithonyssus bacoti), cockroachs, housefly (Musca domestica) and house mosquito (Culex pipiens); stored grain insect pests such as angoumois grai moth (Sitotroga cerealella), adzuki bean weevil (Callosobruchus chinensis), red flour beetle (Tribolium castaneum) and mealworms; household goods insect pests such as casemaking clothes moth (Tinea pellionella), black carpet beetle (Anthrenus scrophularidae) and subterranean termites; domestic mites such as mold mite (Tyrophagus putrescentiae), Dermatophagoides farinae, Chelacaropsis moorei, and so on. Among them, the agricultural and horticultural pesticides

containing the compounds of the present invention are particularly effective for controlling plant parasitic mites, agricultural insect pests, plant parasitic nematodes or the like. Further, they are effective against insect pests having acquired resistance to organophosphorus, carbamate and/or synthetic pyrethroid insecticides. Moreover, the compounds of the present invention have excellent systemic properties, and by the application of the agricultural and horticultural pesticides containing the compounds of the present invention to soil treatment, not only noxious insects, noxious mites, noxious nematodes, noxious gastropods and noxious isopods in soil but also foliage pests can be controlled.

Another preferred embodiments of the pesticides containing compounds of the present invention may be agricultural and horticultural pesticides which collectively control the above-mentioned plant parasitic mites, agricultural insect pests, plant parasitic nematodes, gastropods and soil pests.

The agricultural and horticultural pesticide containing the compound of the present invention, is usually formulated by mixing the compound with various agricultural adjuvants and used in the form of a formulation such as a dust, granules, water-dispersible granules, a wettable powder, a water-based suspension concentrate, an oil-based suspension concentrate, water soluble granules, an emulsifiable concentrate, a soluble concentrate, a paste, an aerosol or an ultra low-volume formulation. However, so long as it is suitable for the purpose of the present invention, it may be formulated into any type of formulation which is commonly used in this field. Such agricultural adjuvants include solid carriers such as diatomaceous earth, slaked lime, calcium carbonate, talc, white carbon, kaoline, bentonite, a mixture of kaolinite and sericite, clay, sodium carbonate, sodium bicarbonate, mirabilite, zeolite and starch; solvents such as water, toluene, xylene, solvent naphtha, dioxane, acetone, isophorone, methyl isobutyl ketone, chlorobenzene, cyclohexane, dimethyl sulfoxide, N,N-dimethylformamide, dimethylacetamide, N-methyl-2-pyrrolidone, and alcohol; anionic surfactants and spreaders such as a salt of fatty acid, a benzoate, an alkylsulfosuccinate, a dialkylsulfosuccinate, a polycarboxylate, a salt of alkylsulfuric acid ester, an alkyl sulfate, an alkylaryl sulfate, an alkyl diglycol ether sulfate, a salt of alcohol sulfuric acid ester, an alkyl sulfonate, an alkylaryl sulfonate, an aryl sulfonate, a lignin sulfonate, an alkyldiphenyl ether disulfonate, a polystyrene sulfonate, a salt of alkylphosphoric acid ester, an alkylaryl phosphate, a styrylaryl phosphate, a salt of polyoxyethylene alkyl ether sulfuric acid ester, a polyoxyethylene alkylaryl ether sulfate, a salt of polyoxyethylene alkylaryl ether sulfuric acid ester, a polyoxyethylene alkyl ether phosphate, a salt of polyoxyethylene alkylaryl phosphoric acid ester, and a salt of a condensate of naphthalene sulfonate with formalin; nonionic surfactants and spreaders such as a sorbitan fatty acid ester, a glycerin fatty acid ester, a fatty acid polyglyceride, a fatty acid alcohol polyglycol ether, acetylene glycol, acetylene alcohol, an oxyalkylene block polymer, a polyoxyethylene alkyl ether, a polyoxyethylene alkylaryl ether, a polyoxyethylene styrylaryl ether, a polyoxyethylene glycol alkyl ether, a polyethylene glycol, a polyoxyethylene fatty acid ester, a polyoxyethylene sorbitan fatty acid ester, a polyoxyethylene glycerin fatty acid ester, a polyoxyethylene hydrogenated castor oil, and a polyoxypropylene fatty acid ester; vegetable and mineral oils such as olive oil, kapok oil, castor oil, palm oil, camellia oil, coconut oil, sesame oil, corn oil, rice bran oil, peanut oil, cottonseed oil, soybean oil, rapeseed oil, linseed oil, tung oil, and liquid paraffins; and so on. Each of the components as such adjuvants may be one or more suitably selected for use, so long as the purpose of the present invention can thereby be accomplished. Further, various adjuvants which are commonly used, such as a filler, a thickener, an anti-settling agent, an anti-freezing agent, a dispersion stabilizer, a phytotoxicity reducing agent, an anti-mold agent, and so on, may also be employed.

The weight ratio of the compound of the present invention to the various agricultural adjuvants is usually from 0.001:99.999 to 95:5, preferably from 0.005:99.995 to 90:10.

In the actual application of such a formulation, it may be used as it is, or may be diluted to a predetermined concentration with a diluent such as water, and various spreaders e.g. surfactants, vegetable oils or mineral oils may be added thereto, as the case requires.

The application of the agricultural and horticultural pesticide containing the compound of the present invention can not generally be defined, as it varies depending upon the weather conditions, the type of the formulation, the application season, the application site or the types or degree of outbreak of the pest insects. However, it is usually applied in a concentration of the active ingredient being from 0.05 to 800,000 ppm, preferably from 0.5 to 500,000 ppm, and the dose per unit area is such that the compound of the present invention is from 0.05 to 50,000 g, preferably from 1 to 30,000 g, per hectare. Further, agricultural and horticultural pesticides as another preferred embodiment of pesticides containing the compounds of the present invention may be applied in accordance with the above-described application of pesticides. The present invention includes such an agricultural or horticultural use of an affective amount of the pyridylmethamine of the present invention for controlling pests, particularly for controlling plant parasitic mites, agricultural insect pests or plant parasitic nematodes.

Various formulations of agricultural and horticultural pesticides containing the compounds of the present invention or their diluted compositions may be applied by conventional methods for application which are commonly employed, such as spraying (e.g. spraying, jetting, misting, atomizing, powder or grain scattering or dispersing in water), soil application (e.g. mixing or drenching), surface application (e.g. coating, powdering or covering) or impregnation to obtain poisonous feed. Further, it is possible to feed domestic animals with a food containing the above active ingredient and to control the outbreak or growth of pests, particularly insect pests, with their excrements. Furthermore, the active

ingredient may also be applied by a so-called ultra low-volume application method. In this method, the composition may be composed of 100% of the active ingredient.

Further, the agricultural and horticultural pesticides containing compounds of the present invention may be mixed with or may be used in combination with other agricultural chemicals, fertilizers or phytotoxicity-reducing agents, whereby synergistic effects or activities may sometimes be obtained. Such other agricultural chemicals include, for example, a herbicide, an insecticide, a miticide, a nematicide, a soil pesticide, a fungicide, an antivirus agent, an attractant, an antibiotic, a plant hormone, a plant growth regulating agent, and so on. Especially, with a mixed pesticide having a compound of the present invention mixed with or used in combination with one or more active compounds of other agricultural chemicals, the application range, the application time, the pesticidal activities, etc. may be improved to preferred directions. The compound of the present invention and the active compounds of other agricultural chemicals may separately be formulated so that they may be mixed for use at the time of application, or they may be formulated together. The present invention includes such a mixed pesticidal composition.

The mixing ratio of the compound of the present invention to the active compounds of other agricultural chemicals can not generally be defined, since it varies depending upon the weather conditions, the types of formulations, the application time, the application site, the types or degree of outbreak of insect pests, etc., but it is usually within a range of from 1:300 to 300:1, preferably from 1:100 to 100:1, by weight. Further, the dose for the application is such that the total amount of the active compounds is from 0.1 to 50,000 g, preferably from 1 to 30,000 g, per hectare. The present invention includes an agricultural or horticultural use of an effective amount of the pyridyl-methamine of the present invention for controlling pests by an application of such a mixed pesticide composition.

The active compounds of insect pest control agents such as insecticides, miticides, nematicides or soil pesticides in the above-mentioned other agricultural chemicals, include, for example, (by common names, some of them are still in an application stage) organic phosphate compounds such as profenofos, dichlorvos, fenamiphos, fenitrothion, EPN, diazinon, chlorpyrifos-methyl, acephate, prothiofos, fosthiazate, phoshocarb, cadusafos, dislufoton, chlorpyrifos, demeton-S-methyl, dimethoate, methamidophos, imicyafos, isoxathion, isofenphos, ethion, etrimfos, quinalphos, dimethylvinphos, sulprofos, thiometon, vamidothion, pyraclofos, pyridaphenthion, pirimiphos-methyl, propaphos, phosalone, formothion, malathion, tetrachlovinphos, chlorfenvinphos, cyanophos, trichlorfon, methidathion, phenthoate, ESP, azinphos-methyl, fenthion, heptenophos, methoxychlor, paration, monocrotophos, parathion-methyl, terbufos, phospamidon, phosmet and phorate; carbamate compounds such as carbaryl, propoxur, aldicarb, carbofuran, thiodicarb, methomyl, oxamyl, ethiofencarb, pirimicarb, fenobucarb, carbosulfan, benfuracarb, bendiocarb, furathiocarb, isoprocarb, metolcarb, xylylcarb, XMC and fenothiocarb; nereistoxin derivatives such as cartap, thiocyclam, bensultap and thiosultap-sodium; organic chlorine compounds such as dicofol, tetradifon, endosulufan, dienochlor and dieldrin; organic metal compounds such as fenbutatin Oxide and cyhexatin; pyrethroid compounds such as fenvalerate, permethrin, cypermethrin, deltamethrin, cyhalothrin, tefluthrin, ethofenprox, fenpropathrin, bifenthrin, cyfluthrin, flucythrinate, fluvalinate, cycloprothrin, lambda-cyhalothrin, pyrethrins, esfenvalerate, tetramethrin, resmethrin, protrifenbute, zeta-cypermethrin, acrinathrin, alpha-cypermethrin, allethrin, gamma-cyhalothrin, theta-cypermethrin, taufluvalinate, tralomethrin, profluthrin, betacypermethrin, beta-cyfluthrin and metofluthrin; benzoylurea compounds such as diflubenzuron, chlorfluazuron, teflubenzuron, flufenoxuron, lufenuron, novaluron, triflumuron, hexaflumuron, noviflumuron, bistrifluron and fluazuron; juvenile hormone-like compounds such as methoprene, pyriproxyfen, fenoxycarb and diofenolan; pyridazinone compounds such as pyridaben; pyrazole compounds such as fenpyroximate, fipronil, tebufenpyrad, ethiprole, tolfenpyrad, acetoprole, pyrafluprole and pyriprole; neonicotinoids such as imidacloprid, nitenpyram, acetamiprid, thiacloprid, thiamethoxam, clothianidin, dinotefuran and nithiazine; hydrazine compounds such as tebufenozide, methoxyfenozide, chromafenozide and halofenozide; other compounds such as flonicamid, buprofezin, hexythiazox, amitraz, chlordimeform, silafluofen, triazamate, pymetrozine, pyrimidifen, chlorfenapyr, indoxacarb, acequinocyl, etoxazole, cyromazine, 1,3-dichloropropene, diafenthiuron, benclothiaz, flufenrim, pyridalyl, spirodiclofen, bifenazate, spiromesifen, spirotetramat, propargite, clofentezine, fluacrypyrim, metaflumizone, flubendiamide, chlorantraniliprole, cyflumetofen, cyenopyrafen, pyrifluquinazon, fenazaquin, pyridaben, amidoflumet, chlorobenzoate, sulfluramid, hydramethylnon, metaldehyde and ryanodine. Further, microbial agricultural chemicals such as Bacillus thuringienses aizawai, Bacillus thuringienses kurstaki, Bacillus thuringienses israelensis, Bacillus thuringienses japonensis, Bacillus thuringienses tenebrionis, insecticidal crystal protein produced by Bacillus thuringienses, insect viruses, etomopathogenic fungi, and nematophagous fungi; antibiotics or semisynthetic antibiotics such as avermectin, emamectin-benzoate, milbemectin, spinosad, ivermectin, lepimectin, spinetoram, abamectin and emamectin; natural products such as azadirachtin and rotenone; and repellents such as deet may, for example, be mentioned.

The fungicidal active compounds in the above-mentioned other agricultural chemicals include, for example, (by common names, some of them are still in an application stage, or test codes of Japan Plant Protection Association) anilinopyrimidine compounds such as mepanipyrim, pyrimethanil and cyprodinil; pyridinamine compounds such as fluazinam; azole compounds such as triadimefon, bitertanol, triflumizole, etaconazole, propiconazole, penconazole, flusilazole, myclobutanil, cyproconazole, tebuconazole, hexaconazole, furconazole-cis, prochloraz, metconazole, epoxiconazole, tetraconazole, oxpoconazole fumarate, sipconazole, prothioconazole, triadimenol, flutriafol, difenoconazole, fluquinconazole, fenbuconazole, bromuconazole, diniconazole, tricyclazole, probenazole, simeconazole, pefurazoate, ipconazole and imibenconazole; quinoxaline compounds such as quinomethionate; dithiocarbamate compounds such as maneb, zineb, mancozeb, polycarbamate, metiram, propineb and thiram; organic chlorine compounds such as fthalide, chlorothalonil and quintozene; imidazole compounds such as benomyl, thiophanate-methyl, carbendazim, thiabendazole, fuberiazole and cyazofamid; cyanoacetamide compounds such as cymoxanil; phenylamide compounds such as metalaxyl, metalaxyl-M, mefenoxam, oxadixyl, ofurace, benalaxyl, benalaxyl-M (another name: kiralaxyl, chiralaxyl), furalaxyl and cyprofuram; sulfenic acid compounds such as dichlofluanid; copper compounds such as cupric hydroxide and oxine copper; isoxazole compounds such as hymexazol; organophosphorus compounds such as fosetyl-Al, tolcofos-methyl, S-benzyl, O,O-diisopropylphosphorothioate, O-ethyl, S,S-diphenylphosphorodithioate, aluminum ethylhydrogen phosphonate, edifenphos and iprobenfos; N-halogenothioalkyl compounds such as captan, captafol and folpet; dicarboximide compounds such as procymidone, iprodione and vinclozolin; benzanilide compounds such as flutolanil, mepronil, zoxamid and tiadinil; anilide compounds such as carboxin, oxycarboxin, thifluzamide, penthiopyrad and boscalid; piperazine compounds such as triforine; pyridine compounds such as pyrifenox; carbinol compounds such as fenarimol and flutriafol; piperidine compounds such as fenpropidine; morpholine compounds such as fenpropimorph and tridemorph; organotin compounds such as fentin hydroxide and fentin acetate; urea compounds such as pencycuron; cinnamic acid compounds such as dimethomorph and flumorph; phenylcarbamate compounds such as diethofencarb; cyanopyrrole compounds such as fludioxonil and fenpiclonil, strobilurin compounds such as azoxystrobin, kresoxim-methyl, metominofen, trifloxystrobin, picoxystrobin, oryzastrobin, dimoxystrobin, pyraclostrobin, fluoxastrobin and fluacrypyrin; oxazolidinone compounds such as famoxadone; thiazolecarboxamide compounds such as ethaboxam; silylamide compounds such as silthiopham; aminoacid amidecarbamate compounds such as iprovalicarb and benthiavalicarb-isopropyl; imidazolidine compounds such as fenamidone; hydroxanilide compounds such as fenhexamid; benzenesulfonamide compounds such as flusulfamide; oxime ether compounds such as cyflufenamid; phenoxyamide compounds such as fenoxanil; antibiotics such as validamycin, kasugamycin and polyoxins; guanidine compounds such as iminoctadine; and other compounds such as isoprothiolane, pyroquilon, diclomezine, quinoxyfen, propamocarb hydrochloride, spiroxamine, chloropicrin, dazomet, metam-sodium, nicobifen, metrafenone, UBF-307, diclocymet, proquinazid, amisulbrom (another name: amibromdole), KIF-7767 (KUF-1204, pyribencarb methyl, mepyricarb), Syngenta 446510 (mandipropamid, dipromandamid), fluopicolide, carpropamid, BCF051, BCM061 and BCM062.

Further, agricultural chemicals which may be used in admixture with or in combination with the compounds of the present invention, may, for example, be the active ingredient compounds in the herbicides as disclosed in Farm Chemicals Handbook (2002 edition), particularly those of soil treatment type.

The pesticides against parasites on animals are effective for controlling e.g. external parasites which are parasitic on the body surface of host animals (such as the back, the axilla, the lower abdomen or inside of the thigh) or internal parasites which are parasitic in the body of host animals (such as the stomach, the intestinal tract, the lung, the heart, the liver, the blood vessels, the subcutis or lymphatic tissues), but they are particularly effective for controlling the external parasites.

The external parasites may, for example, be animal parasitic acarus or fleas. Their species are so many that it is difficult to list all of them, and therefore, their typical examples will be given.

The animal parasitic acarus may, for example, be ticks such as Boophilus microplus, Rhipicephalus sanguineus, Haemaphysalis longicornis, Haemaphysalis flava, Haemaphysalis campanulata, Haemaphysalis concinna, Haemaphysalis japonica, Haemaphysalis kitaokai, Haemaphysalis ias, Ixodes ovatus, Ixodes nipponensis, Ixodes persulcatus, Amblyomma testudinarium, Haemaphysalis megaspinosa, Dermacentor reticulates, and Dermacentor taiwanesis; common red mite (Dermanyssus gallinae); northern fowl mites such as Ornithonyssus sylviarum, and Ornithonyssus bursa; trombidioids such as Eutrombicula wichmanni, Leptotrombidium akamushi, Leptotrombidium pallidum, Leptotrombidium fuji, Leptotrombidium tosa, Neotrombicula autumnalis, Eutrombicula alfreddugesi, and Helenicula miyagawai; cheyletidae such as Cheyletiella yasguri, Cheyletiella parasitivorax, and Cheyletiella blakei; sarcoptic mange mites such as Psoroptes cuniculi, Chorioptes bovis, Otodectes cynotis, Sarcoptes scabiei, and Notoedres cati; and Demodicidae such as Demodex canis. The pesticides against parasites on animals, containing the compounds of the present invention, are particularly effective for the control of ticks among them.

The fleas may, for example, be externally parasitic wingless insects belonging to Siphonaptera, more specifically, fleas belonging to Pulicidae, Ceratephyllus, etc. Fleas belonging to Pulicidae may for example, be Ctenocephalides canis, Ctenocephalides felis, Pulex irritans, Echidnophaga gallinacea, Xenopsylla cheopis, Leptopsylla segnis, Nosopsyllus fasciatus, and Monopsyllus anisus. The pesticides against parasites on animals, containing the compounds of the present invention, are particularly effective for the control of fleas belonging to Pulicidae, particularly Ctenocephalides canis and Ctenocephalides felis, among them.

Other external parasites may, for example, be sucking lice (Anoplura) such as shortnosed cattle louse (Haematopinus eurysternus), horse sucking louse (Haematopinus asini), sheep louse, longnosed cattle louse (Linognathus vituli), and head louse (Pediculus capitis); biting lice such as dog biting louse (Trichodectes canis); and blood-sucking dipterous insects such as horsefly (Tabanus trigonus), biting midges (Culicoides schultzei), and blackfly (Simulium

ornatum). Further, the internal parasites may, for example, be nematodes such as lung worms, whipworms (Trichuris), tuberous worms, gastric parasites, ascaris, and filarioidea; cestoda such as Spirometra erinacei, Diphyllobothrium latum, Dipylidium caninum, Taenia multiceps, Echinococcus granulosus, Echinococcus multilocularis; trematoda such as Schistosoma japonicum, Fasciola hepatica; and protozoa such as coccidia, malaria parasites (Plasmodium malariae), intestinal sarcocyst, toxoplasma, and cryptosporidium.

The host animals may, for example, be pet animals, domestic animals, and poultry, such as dogs, cats, mice, rats, hamsters, guinea pigs, squirrels, rabbits, ferrets, birds (such as pigeons, parrots, hill mynas, Java sparrows, honey parrots, lovebirds and canaries), cows, horses, pigs, sheep, ducks and chickens. The pesticides against parasites on animals, containing the compounds of the present invention, are particularly effective for the control of pests parasitic on pet animals or domestic animals, especially for the control of external parasites, among them. Among pet animals or domestic animals, they are effective particularly for dogs and cats, cows and horses.

When the compound of the present invention is used as a pesticide against parasites on animals, it may be used as it is or may be used together with suitable adjuvants, as formulated into various formulations such as a dust, granules, tablets, a powder, capsules, a soluble concentrate, an emulsifiable concentrate, a water-based suspension concentrate and an oil-based suspension concentrate. In addition to such formulations, it may be formulated into any type of formulation which is commonly used in this field, so long as it is suitable for the purpose of the present invention. The adjuvants to be used for formulations may, for example, be anionic surfactants or nonionic surfactants exemplified above as adjuvants for formulation of agricultural and horticultural pesticides; a cationic surfactant such as cetyl trimethylammonium bromide; a solvent such as water, acetone, acetonitrile, monomethylacetamide, dimethylacetamide, dimethylformamide, 2-pyrrolidone, N-methyl-2-pyrrolidone, kerosene, triacetin, methanol, ethanol, isopropanol, benzyl alcohol, ethylene glycol, propylene glycol, polyethylene glycol, liquid polyoxyethylene glycol, butyl diglycol, ethylene glycol monomethyl ether, ethylene glycol monoethyl ether, diethylene glycol monoethyl ether, diethylene glycol n-butyl ether, dipropylene glycol monomethyl ether, or dipropylene glycol n-butyl ether; an antioxidant such as butylhydroxyanisole, butylhydroxytoluene, ascorbic acid, sodium hydrogenmetasulfite, propyl gallate or sodium thiosulfate; a coating film-forming agent such as polyvinylpyrrolidone, polyvinyl alcohol, or a copolymer of vinyl acetate and vinyl pyrrolidone; the vegetable oils and mineral oils as exemplified above as adjuvants for formulation of agricultural and horticultural pesticides; a carrier such as lactose, sucrose, glucose, starch, wheat flour, corn powder, soybean cake and meal, defatted rice bran, calcium carbonate or other commercially available feed materials; and so on. One or more of the respective components of these adjuvants may be suitably selected for use, so long as such will not depart from the purpose of the present invention. Further, other than the above-mentioned adjuvants, some among those known in this field may suitably be selected for use, and still further, some among the above-mentioned various adjuvants to be used in the agricultural and horticultural field may suitably be selected for use.

The blend ratio of the compound of the present invention to various adjuvants is usually from 0.1:99.9 to 90:10. In the actual use of such a formulation, it may be used as it is, or may be diluted to a predetermined concentration with a diluent such as water, and various spreaders (e.g. surfactants, vegetable oils or mineral oils) may be added thereto, as the case requires.

Administration of the compound of the present invention to a host animal is carried out orally or parenterally. As an oral administration method, a method of administering a tablet, a liquid agent, a capsule, a wafer, a biscuit, a minced meat or other feed, containing the compound of the present invention, may be mentioned. As a parenteral administration method, there may, for example, be mentioned a method wherein the compound of the present invention is formulated into a suitable formulation and then taken into the body by e.g. intravenous administration, intramuscular administration, intradermal administration, hypodermic administration, etc.; a method wherein it is administered on the body surface by spot-on treatment, pour-on treatment or spray treatment; or a method of embedding a resin fragment or the like containing the compound of the present invention under the skin of the host animal.

The dose of the compound of the present invention to a host animal varies depending upon the administration method, the purpose of administration, the deceased symptom, etc., but it is usually administered in a proportion of from 0.01 mg to 100 g, preferably from 0.1 mg to 10 g, per 1 kg of the body weight of the host animal.

A method for controlling a pest by the above-mentioned administration method or by the above-mentioned dose, particularly a method for controlling external parasites or internal parasites is hereby disclosed.

Further, by controlling pests parasitic on animals as described above, it is possible to prevent or cure various diseases of the host animal thereby caused in some cases. Thus, the present invention also includes a preventive or therapeutic agent for an animal disease caused by parasites, containing the compound of the present invention as an active ingredient.

When the compound of the present invention is used as a pesticide against parasites on animals, various vitamins, minerals, amino acids, nutrients, enzymes, antipyretics, sedatives, antiphlogistics, fungicides, colorants, aromatic substances, preservatives, etc., may be used in admixture with or in combination with the adjuvants. Further, as the case requires, other animal drugs or agricultural chemicals, such as vermicides, anti-coccidium agents, insecticides, miticides, pulicides, nematocides, bactericides or antibacterial agents, may be mixed or combined for use, whereby

improved effects may sometimes be obtained. The present invention includes such a mixed pesticidal composition having the above-mentioned various components mixed or combined for use.

Preferred embodiments of the compound represented by the above formula (I) are as follows. However, it should be understood that the present invention is by no means thereby restricted.

(1) A pyridyl-methanamine derivative represented by the formula (I) or its salt:

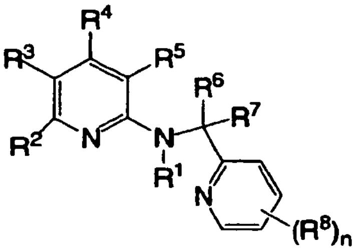

( I )

wherein $R^1$ is hydrogen, alkyl which may be substituted by $R^b$, alkenyl which may be substituted by $R^b$, alkynyl which may be substituted by $R^b$, aryl, cyano, $N{=}CHR^c$, $OR^c$, $S(O)_pR^c$, $COSR^c$, $COOR^c$, $COR^c$, or a heterocyclic group which may be substituted by alkyl or haloalkyl; each of $R^2$ and $R^3$ which are independent of each other, is hydrogen, halogen, cyano, nitro, alkyl which may be substituted by $R^8$, cycloalkyl, alkenyl, alkynyl, aryl, a heterocyclic group, $NR^aR^c$, $OR^a$ , $SR^a$, $COR^a$ , $COOR^a$, $CONR^aR^c$, $CH{=}NOR^a$, $SO_2R^a$ or $SOR^a$; $R^4$ istrifluoromethyl or chlorodifluoromethyl; $R^5$ is hydrogen, halogen, cyano, nitro, alkyl which may be substituted by $R^8$, alkenyl which may be substituted by $R^8$, alkynyl which may be substituted by $R^8$, $OR^a$, $SR^a$, $NR^aR^c$, $COOR^a$ or $COR^a$; each of $R^6$ and $R^7$ which are independent of each other, is hydrogen, cyano, alkyl, haloalkyl or cycloalkyl, or $R^6$ and $R^7$ may together form $C_{3-6}$ cycloalkyl which may be substituted by halogen; $R^8$ is alkyl, cycloalkyl, alkoxyalkyl, alkoxyalkoxyalkyl, hydroxyalkyl, halogen, haloalkyl, cyano, nitro, aryl which may be substituted by halogen, a heterocyclic group which may be substituted by halogen, heterocyclic oxy which may be substituted by halogen, $CONR^aR^c$, $COR^c$, $COOR^c$, $NR^aR^c$ or $OR^a$; $R^a$ is hydrogen, alkyl, cycloalkyl, haloalkyl or heterocyclic alkyl; $R^b$ is halogen, aryl which may be substituted by $R^8$, a heterocyclic group which may be substituted by $R^8$, heterocyclic oxy which may be substituted by $R^8$, heterocyclic thio which may be substituted by $R^8$, cyano, $NR^aR^c$, $NHCOOR^a$, $COR^c$, $COOR^c$, $CONR^aR^c$, alkoxyalkoxy, $OR^a$ or $S(O)_pR^a$; $R^c$ is hydrogen, alkyl, haloalkyl, cycloalkyl, alkoxyalkyl, hydroxyalkyl, aryl.which may be substituted by halogen, or a heterocyclic group which may be substituted by haloalkyl; n is an integer of from 0 to 4, p is an integer of from 0 to 2, in the $NR^aR^c$ moiety in each of the above substituents, $R^a$ and $R^c$ may together form a 5- or 6-membered heterocyclic ring together with the nitrogen atom to which they are bonded, provided that N-(2-pyridylmethyl)-3,5,6-trichloro-4-(trifluoromethyl)-2-pyridylamine is excluded.

(2) The pyridyl-methanamine derivative or its salt according to (1), wherein $R^1$ is hydrogen, alkyl which may be substituted by $R^b$, alkenyl which may be substituted by $R^b$ alkynyl which may be substituted by $R^b$, aryl, a heterocyclic group which may be substituted by haloalkyl, $N{=}CHR^c$, $OR^c$, $COSR^c$ or $COR^c$; each of $R^2$ and $R^3$ which are independent of each other, is hydrogen, halogen, cyano, nitro, alkyl which may be substituted by $R^8$, cycloalkyl, alkenyl, alkynyl, aryl, a heterocyclic group, $NR^aR^c$, $OR^a$, $SR^a$, $COR^a$, $COOR^a$, $CONR^aR^c$, $SO_2R^a$ or $SOR^a$; $R^5$ is hydrogen, halogen, cyano, nitro, alkyl which may be substituted by $R^8$, $COOR^a$ or $COR^a$; each of $R^6$ and $R^7$ which are independent of each other, is hydrogen, cyano, alkyl, haloalkyl or cycloalkyl; $R^8$ is alkyl, cycloalkyl, alkoxyalkyl, alkoxyalkoxyalkyl, hydroxyalkyl, halogen, haloalkyl, cyano, nitro, $NR^aR^c$ or $OR^a$; $R^a$ is hydrogen, alkyl, cycloalkyl or haloalkyl; $R^b$ is halogen, aryl which may be substituted by $R^8$, a heterocyclic group which may be substituted by $R^8$, cyano, $NR^aR^c$, $NHCOOR^a$, $OR^a$ or $SR^a$; and $R^c$ is hydrogen, alkyl, cycloalkyl, aryl or a heterocyclic group which may be substituted by haloalkyl.

(3) The pyridyl-methanamine derivative or its salt according to (2), wherein each of $R^2$ and $R^3$ which are independent of each other, is hydrogen, halogen, cyano, nitro, alkyl, haloalkyl, alkenyl, alkynyl, aryl, a heterocyclic group, $NR^aR^c$, $OR^a$, $SR^a$, $COR^a$ or $SOR^a$; $R^5$ is hydrogen, halogen or $COR^a$; $R^8$ is alkyl, halogen, haloalkyl, alkoxyalkoxyalkyl, hydroxyalkyl, cyano, $NR^aR^c$ or $OR^a$; $R^a$ is hydrogen, alkyl or haloalkyl; $R^b$ is halogen, aryl, a heterocyclic group which may be substituted by $R^8$, cyano, $NR^aR^c$, $OR^a$ or $SR^a$; and $R^c$ is hydrogen, alkyl, aryl or a heterocyclic group.

(4) The pyridyl-methanamine derivative or its salt according to (2), wherein $R^1$ is hydrogen, alkyl which may be substituted by $R^b$, alkenyl which may be substituted by $R^b$, alkynyl, aryl, a heterocyclic group which may be substituted by haloalkyl, $OR^c$ or $COR^c$; $R^2$ is hydrogen, halogen, cyano, alkyl, haloalkyl, alkynyl, aryl, $NR^aR^c$, $OR^a$ or $SR^a$; $R^3$ is hydrogen, halogen, cyano, nitro, haloalkyl, aryl, $NR^aR^c$, $SR^a$ or $COR^a$; $R^4$ is trifluoromethyl; $R^5$ is hydrogen, halogen or $COR^a$; each of $R^6$ and $R^7$ which are independent of each other, is hydrogen, alkyl or cycloalkyl; $R^8$ is alkyl, halogen, haloalkyl, alkoxyalkoxyalkyl, hydroxyalkyl, cyano or $OR^a$; $R^a$ is hydrogen or alkyl; $R^b$ is halogen, aryl,

a heterocyclic group which may be substituted by $R^8$, $OR^a$ or $NR^aR^c$; $R^c$ is hydrogen, alkyl, aryl or a heterocyclic group.
(5) The pyridyl-methanamine derivative or its salts according to (2), wherein $R^1$ is hydrogen, alkyl which may be substituted by $R^{b'}$, alkenyl which may be substituted by $R^{b''}$, alkynyl which may be substituted by $R^b$, aryl, $OR^c$ or $COR^c$; $R^{b'}$ is halogen, aryl which may be substituted by $R^8$, cyano, $OR^a$ or $SR^a$; $R^{b''}$ is halogen, aryl which may be substituted by $R^8$, a heterocyclic group which may be substituted by $R^8$, cyano, $OR^a$ or $SR^a$.

The pyridyl-methanamine derivative or its salt in the above (1) is a novel compound and can be produced by at least one process among processes for producing pyridyl-methanamine derivatives shown by the above production processes [1], [2], [3], [4], [5], [6], [7], [8], [9] and [10]. For example, it can be produced by at least one process among the above production processes [1], [2] and [3].

EXAMPLES

Now, the present invention will be described in further detail with reference to Examples, but it should be understood that the present invention is by no means thereby restricted. Firstly, Preparation Examples of the compound of the present invention will be described.

PREPARATION EXAMPLE 1

PREPARATION OF N-(2-PYRIDYLMETHYL)-6-CHLORO-4-(TRIFLUOROMETHYL)-2-PYRIDYLAMINE (Compound No. 128)

To a solution of 10 g of 2,6-dichloro-4-(trifluoromethyl)pyridine in 50 ml of ethanol, 10 g of 2-picolylamine was added and stirred at room temperature for 24 hours, and then reacted at 60˚C for 16 hours and at 80˚C for 24 hours. After completion of the reaction, ethanol was distilled off under reduced pressure, and to the residue, ethyl acetate was added. The ethyl acetate solution was washed with a saturated sodium chloride aqueous solution and then dried over anhydrous magnesium sulfate, and ethyl acetate was distilled off under reduced pressure. The obtained solid residue was recrystallized from hexane to obtain 9.55 g of the desired product.

PREPARATION EXAMPLE 2

PREPARATION OF N-(2-PYRIDYLMETHYL)-5,6-DICHLORO-4-(TRIFLUOROMETHYL)-2-PYRIDYLAMINE (Compound No. 118)

To a solution of 5.0 g of N-(2-pyridylmethyl)-6-chloro-4-(trifluoromethyl)-2-pyridylamine in 50 ml of dimethylformamide, 2.4 g of N-chlorosuccinimide was added at room temperature, followed by gradual heating to 50˚C. 3 Hours later, 0.2 g of N-chlorosuccinimide was added and reacted for 1 hour, and further, 0.2 g was added and reacted for 45 minutes. The reaction solution was left to cool and then water was added thereto, followed by extraction twice with ethyl acetate. The extract solution was washed with a saturated sodium chloride aqueous solution and then dried over anhydrous magnesium sulfate, and the solvent was distilled off under reduced pressure. The residue was purified by silica gel chromatography (eluent: n-hexane/ethyl acetate=1/1) to obtain 2.8 g of the desired product.

PREPARATION EXAMPLE 3

PREPARATION OF N-ALLYL-N-(2-PYRIDYLMETHYL)-5,6-DICHLORO-4-(TRIFLUOROMETHYL)-2-PYRIDYLAMINE (Compound No. 55)

To a solution of 300 mg of N-(2-pyridylmethyl)-5,6-dichloro-4-(trifluoromethyl)-2-pyridylamine in 4 ml of dimethylformamide, 40 mg of sodium hydride was added, followed by stirring at room temperature. 10 Minutes later, 110 mg of allyl bromide was dropwise added, followed by stirring at room temperature for 2.5 hours. After completion of the reaction, water was added to the reaction solution, followed by extraction twice with ethyl acetate. The extract solution was washed with a saturated sodium chloride aqueous solution and then dried over anhydrous magnesium sulfate, and the solvent was distilled off under reduced pressure. The residue was purified by silica gel chromatography (eluent: N-hexane/ethyl acetate=3/1) to obtain 150 mg of the desired product.

PREPARATION EXAMPLE 4

PREPARATION OF N,N-BIS(2-PYRIDYLMETHYL)-5-NITRO-4-(TRIFLUOROMETHYL)-2-PYRIDYLAMINE (Compound No. 9)

(1) Under cooling with ice, 1.0 g of 2-amino-4-(trifluoromethyl)pyridine was gradually added to 5 ml of 97% sulfuric acid, followed by stirring at room temperature until it was dissolved. The reaction solution was cooled to -10˚C, and a cooled mixed solution of 0.8 ml of 69% nitric acid and 0.6 ml of 97% sulfuric acid was dropwise added, followed by a reaction at -10˚C for 30 minutes. The reaction solution was poured into 40 g of ice, and 28% aqueous ammonia was added for neutralization. After stirring, precipitated crystals were collected by filtration and dried. The obtained colorless powder was added to 5.5 ml of 97% sulfuric acid under cooling with ice, followed by stirring for 30 minutes. Further, it was stirred at room temperature for 1 hour and then reacted at 50˚C for 1 hour. After cooling, the reaction solution was poured to 40 g of ice, and 28% aqueous ammonia was added for neutralization, followed by stirring under cooling with ice. Precipitated crystals were collected by filtration, washed with cool water and then dried to obtain 0.41 g of 2-amino-5-nitro-4-(trifluoromethyl)pyridine having a melting point of 138-140˚C.

(2) To a solution of 260 mg of 2-amino-5-nitro-4-(trifluoromethyl)pyridine and 350 mg of (2-bromomethyl)pyridine hydrobromide in 6 ml of dimethylsulfoxide, 0.38 ml of a 10 M sodium hydroxide aqueous solution was dropwise added and reacted at room temperature for 16 hours. Water was added to the reaction solution, followed by extraction twice with ethyl acetate. The extract solution was washed with a saturated sodium chloride aqueous solution and dried over anhydrous magnesium sulfate, and the solvent was distilled off under reduced pressure. The residue was purified by silica gel chromatography (eluent: n-hexane/ethyl acetate=1/2) to obtain 130 mg of the desired product.

PREPARATION EXAMPLE 5

PREPARATION OF N,N-BIS(2-PYRIDYLMETHYL)-6-BROMO-4-(TRIFLUOROMETHYL)-2-PYRIDYLAMINE (Compound No. 90)

(1) To 5.0 g of 2,6-dichloro-4-(trifluoromethyl)pyridine, 25 ml of a hydrogen bromide-saturated acetic acid solution (about 30%) was added and reacted under reflux. 3 Hours later, 10 ml of a hydrogen bromide-saturated acetic acid solution (about 30%) was additionally added, followed by refluxing for 2 hours. Further, 10 ml of a hydrogen bromide-saturated acetic acid solution (about 30%) was added and refluxed for 2 hours, and then, 10 ml of a hydrogen bromide-saturated acetic acid (about 30%) was added and refluxed for 1 hour. The reaction solution was cooled to room temperature, and then added to 200 ml of a 10% sodium hydroxide aqueous solution under cooling with ice. To the obtained solution, sodium hydroxide (solid) was added to make the solution basic. The solution was extracted twice with diethyl ether, and the extract solution was dried over anhydrous magnesium sulfate and the solvent was distilled off under reduced pressure to obtain 6.9 g of oily 2,6-dibromo-4-(trifluoromethyl)pyridine.

(2) To a solution of 1.0 g of 2,6-dibromo-4-(trifluoromethyl)pyridine in 10 ml of dimethylformamide, 0.72 g of 2,2'-dipicolylamine and 0.33 g of sodium hydrogencarbonate were added and reacted at 90˚C for 19 hours. The reaction solution was left to cool and then water was added thereto, followed by extraction twice with ethyl acetate. The extract solution was washed with a saturated sodium chloride aqueous solution and the dried over anhydrous magnesium sulfate, and the solvent was distilled off under reduced pressure. The residue was purified by silica gel chromatography (eluent: n-hexane/ethyl acetate=1/3) to obtain 1.1 g of the desired product.

PREPARATION EXAMPLE 6

PREPARATION OF N,N-BIS(2-PYRIDYLMETHYL)-6-BROMO-5-CHLORO-4-(TRIFLUOROMETHYL)-2-PYRIDYLAMINE (Compound No. 92)

To a solution of 500 mg of N,N-bis(2-pyridylmethyl)-6-bromo-4-(trifluoromethyl)-2-pyridylamine in 5 ml of dimethylformamide, 170 mg of N-chlorosuccinimide was added and reacted at 60˚C for 7 hours. The reaction solution was left to cool and then water was added thereto, followed by extraction twice with ethyl acetate. The extract solution was washed with a saturated sodium chloride aqueous solution and then dried over anhydrous magnesium sulfate, and the solvent was distilled off under reduced pressure. The residue was purified by silica gel chromatography (eluent: n-hexane/ethyl acetate=1/3) to obtain 500 mg of the desired product.

PREPARATION EXAMPLE 7

PREPARATION OF N,N-BIS(2-PYRIDYLMETHYL)-5-CHLORO-6-ETHYNYL-4-(TRIFLUOROMETHYL)-2-PYRIDYLAMINE (Compound No. 94)

370 mg of N,N-bis(2-pyridylmethyl)-6-bromo-5-chloro-4-(trifluoromethyl)-2-pyridylamine, 95 mg of trimethylsilyl acetylene, 20 mg of transdichlorobistriphenylphosphine palladium, 10 mg of copper iodide and 4 ml of triethylamine were refluxed for 4 hours. Triethylamine was distilled off under reduced pressure, and then, water was added, followed by filtration through celite. The filtrate was extracted twice with diethyl ether, and the extraction solvent was distilled off under reduced pressure. To the residue, 2 ml of methanol and 4 ml of a 1N sodium hydroxide aqueous solution were added, followed by stirring at room temperature for 2 hours. Then, 10% hydrochloric acid was added to make the solution acidic. Methanol was distilled off under reduced pressure, and sodium carbonate was added to make the solution basic, followed by extraction twice with diethyl ether. The extract solution was dried over anhydrous magnesium sulfate, and the solvent was distilled off under reduced pressure. The residue was purified by silica gel chromatography (eluent: n-hexane/ethyl acetate=1/3) to obtain 250 mg of the desired product.

PREPARATION EXAMPLE 8

PREPARATION OF N-(1-(3-PYRIDYL)ETHYL)-N-(2-PYRIDYLMETHYL)-5,6-DICHLORO-4-TRIFLUOROMETHYL-2-PYRIDYLAMINE (Compound No. 97)

(1) To a mixed solution comprising 4.56 g of 2-amino-6-chloro-4-trifluoromethylpyridine and 30 ml of dimethylformamide, 3.4 g of N-chlorosuccinimide was added. Then, mixed solution was heated to 80˚C and reacted for 2 hours. After completion of the reaction, the mixed solution was cooled with ice, and a saturated sodium hydrogencarbonate aqueous solution was added. It was extracted twice with ethyl acetate, and then, the organic layer was washed with a saturated sodium chloride aqueous solution and dried over anhydrous sodium sulfate. The solvent in the organic layer was distilled off under reduced pressure, and the residue was purified by silica gel chromatography (eluent: n-hexane/ethyl acetate=3/1) to obtain 3.8 g of 6-amino-2,3-dichloro-4-trifluoromethylpyridine having a melting point of 118-121˚C.

(2) To a mixed solution comprising 800 mg of 6-amino-2,3-dichloro-4-trifluoromethylpyridine and 20 ml of ethanol, 557 mg of 3-pyridine aldehyde, 3 g of molecular sieve and 0.5 ml of acetic acid were added and reacted for 3 days under reflux. After completion of the reaction, the solvent was distilled off under reduced pressure. The residue was dissolved in 50 ml of diethyl ether, and under cooling with ice, 4.6 ml of a methyl magnesium bromide solution (3M, diethyl ether solution) was dropwise added. After completion of the dropwise addition, the mixture was stirred at room temperature overnight. The mixed solution was cooled with ice, and 20 ml of water was added. The solution was extracted twice with ethyl acetate. Then, the organic layer was washed with a saturated sodium chloride aqueous solution and dried over anhydrous sodium sulfate. The solvent in the organic layer was distilled off under reduced pressure, and the residue was purified by silica gel chromatography (eluent: n-hexane/ethyl acetate=1/1), and the obtained crude crystals were recrystallized from ethyl acetate/hexane to obtain 308 mg of N-(1-(3-pyridyl)ethyl)-5,6-dichloro-4-trifluoromethyl-2-pyridylamine having a melting point of 159-161˚C.

(3) To a suspension comprising 19 mg of sodium hydride and 10-ml of dimethylformamide, a mixed solution comprising 133 mg of N-(1-(3-pyridyl)ethyl)-5,6-dichloro-4-trifluoromethyl-2-pyridylamine and 1 ml of dimethylformamide was gradually dropwise added under cooling with ice, followed by stirring at 0˚C for 30 minutes. Then, a mixed solution comprising 136 mg of 2-bromomethylpyridine and 2 ml of dimethylformamide was gradually dropwise added. After completion of the dropwise addition, the mixed solution was reacted at room temperature for 2 hours. After completion of the reaction, the mixed solution was cooled with ice, and 10 ml of water was added thereto, followed by extraction three times with ethyl acetate. The organic layer was washed with a saturated sodium chloride aqueous solution and dried over anhydrous sodium sulfate. The solvent in the organic layer was distilled off under reduced pressure, and the residue was purified by silica gel chromatography (eluent: n-hexane/ethyl acetate=1/1) to obtain 111 mg of the desired product as a colorless oil.

PREPARATION EXAMPLE 9

PREPARATION OF N,N-BIS(2-PYRIDYLMETHYL)-5-CHLORO-4,6-BIS(TRIFLUOROMETHYL)-2-PYRIDYLAMINE (Compound No. 103)

(1) To a solution of 1.5 g of 2-amino-4,6-bis(trifluoromethyl)pyridine in 5 ml of dimethylformamide, 870 mg of N-chlorosuccinimide was added. Then, the mixed solution was heated to 40˚C and reacted for 1 hour. The reaction solution was left to cool and then water was added thereto and stirred, followed by extraction twice with ethyl acetate. The, the organic layer was washed with a saturated sodium chloride aqueous solution and dried over anhydrous sodium sulfate. The solvent in the organic layer was distilled off under reduced pressure, and the residue was purified by silica gel chromatography (eluent: n-hexane/ethyl acetate=7/3) to obtain 1.1 g of 2-amino-5-chloro-4,6-bis(trifluoromethyl)pyridine having a melting point of 97˚C.
(2) To a solution of 150 mg of 2-amino-5-chloro-4,6-bis(trifluoromethyl)pyridine and 260 mg of 2-bromomethylpyridine hydrogen bromide in 4 ml of dimethylsulfoxide, 0.2 ml of a 10M sodium hydroxide aqueous solution was dropwise added and reacted at room temperature for 2.5 hours. Water was added to the reaction solution, followed by extraction twice with ethyl acetate. The extract solution was washed with a saturated sodium chloride aqueous solution and then dried over anhydrous magnesium sulfate, and the solvent was distilled off under reduced pressure. The residue was purified by silica gel chromatography (eluent: n-hexane/ethyl acetate=1/3) to obtain 110 mg of the desired product.

PREPARATION EXAMPLE 10

PREPARATION OF N-(1-(2-PYRIDYL)-2-METHYL)PROPYL-5,6-DICHLORO-4-TRIFLUOROMETHYL-2-PYRIDYLAMINE (Compound No. 75)

To a mixed solution comprising 313 mg of 6-amino-2,3-dichloro-4-(trifluoromethyl)pyridine, 160 mg of 2-pyridinealdehyde and 10 ml of methanol, a catalytic amount of acetic acid was added and reacted under reflux. 6 Hours later, the solvent was distilled off under reduced pressure, and to the oily residue obtained, 30 ml of diethyl ether was added. To this solution, 2.7 ml of a diethyl ether solution of isopropyl magnesium chloride (2M) was added under cooling with ice. After completion of the dropwise addition, the mixture was heated to room temperature and stirred overnight. To the reaction solution, water was added, followed by extraction twice with ethyl acetate. The extract solution was washed with a saturated sodium chloride aqueous solution and then dried over anhydrous magnesium sulfate, and the solvent was distilled off under reduced pressure. The residue was purified by silica gel chromatography (eluent: n-hexane/ethyl acetate=1/4) to obtain 182 mg of the desired product having a melting point of 165-168˚C.

PREPARATION EXAMPLE 11

PREPARATION OF N-(2-PYRIDYLMETHYL)-5-CYANO-4,6-BIS(TRIFLUOROMETHYL)-2-PYRIDYLAMINE (Compound No. 286)

(1) To a solution of 1.4 g of 6-amino-3-bromo-2,4-bis(trifluoromethyl)pyridine in 3 ml of hexamethylphosphoric acid triamide, 1.0 g of copper cyanide was added and reacted at 140˚C for 4 hours under irradiation with microwave. The reaction solution was left to cool and water and ethyl acetate were added thereto, and an insoluble solid was filtered off by celite. The organic layer was separated, and the water layer was further extracted twice with ethyl acetate. The organic layers were put together and washed with a saturated sodium chloride aqueous solution and dried over anhydrous magnesium sulfate, and the solvent was distilled off under reduced pressure. The residue was purified by silica gel chromatography (eluent: n-hexane/ethyl acetate=1/2) to obtain 690 mg of 6-amino-3-cyano-2,4-bis(trifluoromethyl)pyridine having a melting point of 158-160˚C.
(2) To a mixed solution comprising 350 mg of 6-amino-3-cyano-2,4-bis(trifluoromethyl)pyridine and 4 ml of ethanol, 150 mg of 2-pyridinealdehyde, 0.4 g of molecular sieve and 0.1 ml of acetic acid were added, and reacted for 40 hours under reflux. After completion of the reaction, the reaction product was subjected to filtration, and the solvent was distilled off under reduced pressure. The residue was dissolved in 4 ml of ethanol, and 52 mg of sodium borohydride was added under cooling with ice, followed by stirring at room temperature for 3 hours. After completion of the reaction, 20 ml of water was added, followed by extraction twice with ethyl acetate. Then, the organic layer

was washed with a saturated sodium chloride aqueous solution and dried over anhydrous sodium sulfate. The solvent in the organic layer was distilled off under reduced pressure, and the residue was purified by silica gel flush chromatography to obtain 82 mg of the desired product having a melting point of 122-123˚C.

PREPARATION EXAMPLE 12

PREPARATION OF N,N-BIS(2-PYRIDYLMETHYL)-6-CHLORO-5-CYANO-4-(TRIFLUOROMETHYL)-2-PYRIDYLAMINE HYDROCHLORIDE

(Compound No. 330)

To a solution of 350 mg of N,N-bis(2-pyridylmethyl)-6-chloro-5-cyano-4-(trifluoromethyl)-2-pyridylamine (Compound No. 24) in 7 ml of ethanol, 1.5 ml of concentrated hydrochloric acid was dropwise added. After stirring at room temperature for 1 hour, the solvent was distilled off under reduced pressure. The obtained white solid was subjected to repulp cleaning with ethanol to obtain 410 mg of the desired product having a melting point of 144-146˚C.

Now, typical examples of the compound represented by the above formula (I) will be given in Table 1. These compounds can be prepared by the above-described Preparation Examples 1 to 12 or by the above-mentioned various processes for the production of the compound of the present invention. In Table 1, No. represents the Compound No., Me methyl, Et ethyl, Pr(i) isopropyl, Bu(t) tertiary butyl, CO carbonyl, COO carboxyl, Ph phenyl, pyridyl pyridyl, pyridyloxy pyridyloxy, piperidino piperidino, morpholinyl morpholinyl, respectively. The temperature shown as the physical properties is the melting point, "oil" represents an oily substance, "amorphous" non-crystalline, "gummy oil" a sticky oil substance, respectively. Further, nD represents a refractive index. With respect to ones having no melting point or refractive index shown, $^1$H-NMR was shown in Table 2.

TABLE 1

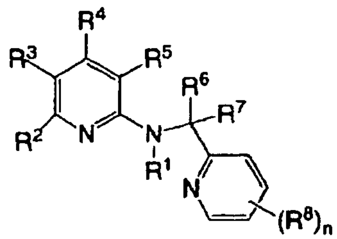

| No. | $R^1$ | $R^2$ | $R^3$ | $R^4$ | $R^5$ | $R^6$ | $R^7$ | $(R^8)_n$ | Physical properties |
|---|---|---|---|---|---|---|---|---|---|
| 1 | H | Cl | Cl | $CF_3$ | Cl | H | H | n=0 | 99-100˚C |
| 2 | H | H | Cl | $CF_3$ | Cl | H | H | n=0 | 76.5˚C |
| 3 | H | H | Cl | $CF_3$ | H | H | H | n=0 | 74-75˚C |
| 4 | 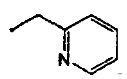 | Cl | Cl | $CF_3$ | Cl | H | H | n=0 | oil |
| 5 | H | H | Br | $CF_3$ | Br | H | H | n=0 | 110-111˚C |
| 6 | 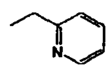 | H | Cl | $CF_3$ | Cl | H | H | n=0 | 62-68˚C |
| 7 | 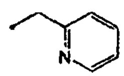 | H | H | $CF_3$ | H | H | H | n=0 | oil |
| 8 | H | Cl | Cl | $CF_3$ | Cl | H | H | 6-CN | 108-112˚C |
| 9 | 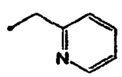 | H | $NO_2$ | $CF_3$ | H | H | H | n=0 | oil |
| 10 | Me | Cl | Cl | $CF_3$ | Cl | H | H | n=0 | $nD_{29.2}$=1. 5618 |
| 11 | H | H | $NO_2$ | $CF_3$ | H | H | H | n=0 | 137-138˚C |
| 12 | H | H | $NH_2$ | $CF_3$ | H | H | H | n=0 | 85-86˚C |
| 13 | H | H | $CF_3$ $CF_3$ | $CF_3$ | H | H | H | n=0 | 92-93˚C |
| 14 | H | H | H | $CF_3$ | $NO_2$ | H | H | n=0 | 121-123˚C |

(continued)

| No. | $R^1$ | $R^2$ | $R^3$ | $R^4$ | $R^5$ | $R^6$ | $R^7$ | $(R^8)_n$ | Physical properties |
|---|---|---|---|---|---|---|---|---|---|
| 15 | H | H | Ph | $CF_3$ | H | H | H | n=0 | amorphous |
| 16 | H | Cl | Cl | $CF_3$ | Cl | Me | H | n=0 | 52°C |
| 17 | 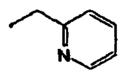 | H | $CF_3$ | $CF_3$ | H | H | H | n=0 | oil |
| 18 | 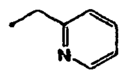 | H | Cl | $CF_3$ | H | H | H | n=0 | oil |
| 19 | 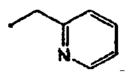 | Cl | Cl | $CF_3$ | H | H | H | n=0 | 76-77°C |
| 20 | H | Cl | CN | $CF_3$ | H | H | H | n=0 | 156-157°C |
| 21 | H | Cl | H | $CF_3$ | CN | H | H | n=0 | 81-82°C |
| 22 | H | H | CN | $CF_3$ | H | H | H | n=0 | 93-95°C |
| 23 | $CH_2Ph$ | Cl | Cl | $CF_3$ | Cl | H | H | n=0 | oil |
| 24 | 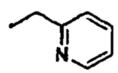 | Cl | CN | $CF_3$ | H | H | H | n=0 | 80-81°C |
| 25 | H | Cl | CN | $CF_3$ | CH (OH) Me | H | H | n=0 | 131-132°C |
| 26 | H | Cl | H | $CF_3$ | COMe | H | H | n=0 | 87-88°C |
| 27 | H | Cl | COMe | $CF_3$ | H | H | H | n=0 | oil |
| 28 | H | H | $NO_2$ | $CF_3$ | $NO_2$ | H | H | n=0 | 176-179°C |
| 29 | 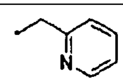 | Cl | COMe | $CF_3$ | H | H | H | n=0 | oil |
| 30 | H | Cl | H | $CF_3$ | $NO_2$ | H | H | n=0 | 117-118°C |
| 31 | H | Cl | $NO_2$ | $CF_3$ | H | H | H | n=0 | 154-155°C |
| 32 | 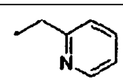 | Cl | H | $CF_3$ | COMe | H | H | n=0 | oil |
| 33 | H | Cl | Cl | $CF_3$ | $NO_2$ | H | H | n=0 | 150-152°C |
| 34 | H | Cl | $NO_2$ | $CF_3$ | Cl | H | H | n=0 | 137-138°C |

(continued)

| No. | $R^1$ | $R^2$ | $R^3$ | $R^4$ | $R^5$ | $R^6$ | $R^7$ | $(R^8)_n$ | Physical properties |
|---|---|---|---|---|---|---|---|---|---|
| 35 | 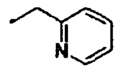 | Cl | $NO_2$ | $CF_3$ | H | H | H | n=0 | oil |
| 36 | 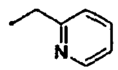 | Cl | H | $CF_3$ | $NO_2$ | H | H | n=0 | oil |
| 37 | H | Cl | COOMe | $CF_3$ | COOMe | H | H | n=0 | 100-101˚C |
| 38 | 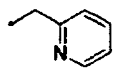 | Cl | H | $CF_3$ | Cl | H | H | n=0 | oil |
| 39 | 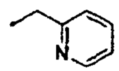 | Cl | $NO_2$ | $CF_3$ | Cl | H | H | n=0 | oil |
| 40 | H | Cl | Cl | $CF_2Cl$ | Cl | H | H | n=0 | 105-106˚C |
| 41 | H | Cl | Cl | $CF_2Cl$ | H | H | H | n=0 | 119-120˚C |
| 42 | 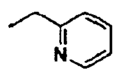 | Cl | H | $CF_2Cl$ | H | H | H | n=0 | oil |
| 43 | 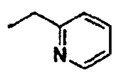 | Cl | Cl | $CF_2Cl$ | H | H | H | n=0 | oil |
| 44 | H | Cl | $CONMe_2$ | $CF_3$ | H | H | H | n=0 | 146-147˚C |
| 45 | Me | Cl | Cl | $CF_3$ | H | H | H | n=0 | 82-86˚C |
| 46 | 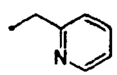 | CN | H | $CF_3$ | H | H | H | n=0 | oil |
| 47 | 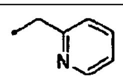 | Cl | $CONH_2$ | $CF_3$ | H | H | H | n=0 | 158-159˚C |
| 48 | 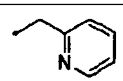 | CN | Cl | $CF_3$ | H | H | H | n=0 | oil |
| 49 | 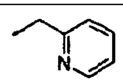 | Cl | $CONMe_2$ | $CF_3$ | H | H | H | n=0 | amorphous |
| 50 | 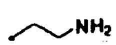 | Cl | Cl | $CF_3$ | H | H | H | n=0 | oil |

(continued)

| No. | $R^1$ | $R^2$ | $R^3$ | $R^4$ | $R^5$ | $R^6$ | $R^7$ | $(R^8)_n$ | Physical properties |
|---|---|---|---|---|---|---|---|---|---|
| 51 | H | CN | H | $CF_3$ | H | H | H | n=0 | 94-96˚C |
| 52 | 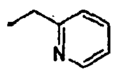 | Cl | CH(OH)Pr(i) | $CF_3$ | H | H | H | n=0 | oil |
| 53 | 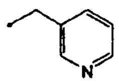 | Cl | Cl | $CF_3$ | H | H | H | n=0 | oil |
| 54 | 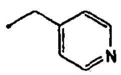 | Cl | Cl | $CF_3$ | H | H | H | n=0 | oil |
| 55 | 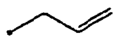 | Cl | Cl | $CF_3$ | H | H | H | n=0 | oil |
| 56 | H | CN | Cl | $CF_3$ | H | H | H | n=0 | 116-118˚C |
| 57 | 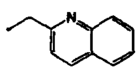 | Cl | Cl | $CF_3$ | H | H | H | n=0 | oil |
| 58 | 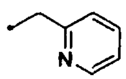 | F | H | $CF_3$ | H | H | H | n=0 | oil |
| 59 | 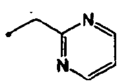 | Cl | Cl | $CF_3$ | Cl | H | H | n=0 | amorphous |
| 60 | 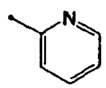 | Cl | Cl | $CF_3$ | H | H | H | n=0 | 159-160˚C |
| 61 | 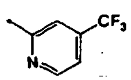 | Cl | Cl | $CF_3$ | Cl | H | H | n=0 | 138-140˚C |
| 62 | 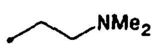 | Cl | Cl | $CF_3$ | H | H | H | n=0 | oil |
| 63 | 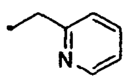 | F | Cl | $CF_3$ | H | H | H | n=0 | oil |
| 64 | 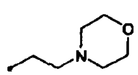 | Cl | Cl | $CF_3$ | H | H | H | n=0 | oil |
| 65 | Bu (t) | Cl | H | $CF_3$ | H | H | H | n=0 | oil |

(continued)

| No. | $R^1$ | $R^2$ | $R^3$ | $R^4$ | $R^5$ | $R^6$ | $R^7$ | $(R^8)_n$ | Physical properties |
|---|---|---|---|---|---|---|---|---|---|
| 66 | Bu(t) | Cl | Cl | $CF_3$ | H | H | H | n=0 | 82°C |
| 67 | 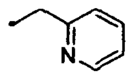 | Cl | Cl | $CF_3$ | Cl | Me | H | n=0 | oil |
| 68 | 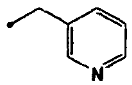 | Cl | CN | $CF_3$ | H | H | H | n=0 | 100-101°C |
| 69 | H | Cl | Cl | $CF_3$ | Cl | H | H | 3-Me | 165-167°C |
| 70 | 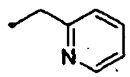 | Cl | Cl | $CF_3$ | H | Me | H | n=0 | oil |
| 71 | 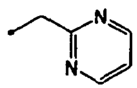 | Cl | Cl | $CF_3$ | H | H | H | n=0 | amorphous |
| 72 | 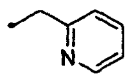 | F | CN | $CF_3$ | H | H | H | n=0 | 58-59°C |
| 73 | 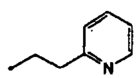 | Cl | Cl | $CF_3$ | H | H | H | n=0 | 56-57°C |
| 74 | 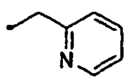 | OMe | CN | $CF_3$ | H | H | H | n=0 | 71-73°C |
| 75 | H | Cl | Cl | $CF_3$ | H | Pr (i) | H | n=0 | 165-168°C |
| 76 | 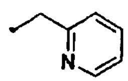 | Cl | Cl | $CF_3$ | H | Pr (i) | H | n=0 | oil |
| 77 | 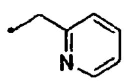 | Cl | Br | $CF_3$ | H | H | H | n=0 | 85-87°C |
| 78 | 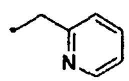 | Cl | I | $CF_3$ | H | H | H | n=0 | 102-103°C |

(continued)

| No. | $R^1$ | $R^2$ | $R^3$ | $R^4$ | $R^5$ | $R^6$ | $R^7$ | $(R^8)_n$ | Physical properties |
|---|---|---|---|---|---|---|---|---|---|
| 79 | 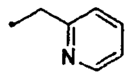 | Cl | Cl | $CF_3$ | H | Et | H | n=0 | oil |
| 80 | 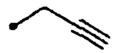 | Cl | Cl | $CF_3$ | H | H | H | n=0 | oil |
| 81 | 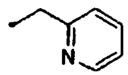 | $NMe_2$ | Cl | $CF_3$ | H | H | H | n=0 | oil |
| 82 | OMe | Cl | Cl | $CF_3$ | H | H | H | n=0 | oil |
| 83 | 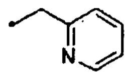 | H | CN | $CF_3$ | H | H | H | n=0 | oil |
| 84 | H | H | CN | $CF_3$ | Cl | H | H | n=0 | 112-114˚C |
| 85 | H | Cl | Cl | $CF_3$ | Cl | H | H | 3-OMe | 163-165˚C |
| 86 | 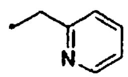 | Cl | Cl | $CF_3$ | H | 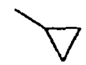 | H | H | gummy oil |
| 87 | H | $CF_3$ | Cl | $CF_3$ | Cl | H | H | n=0 | 92-93˚C |
| 88 | H | H | $CF_3$ | $CF_3$ | Cl | H | H | n=0 | 100-102˚C |
| 89 | COME | Cl | Cl | $CF_3$ | H | H | H | n=0 | 81-82˚C |
| 90 | 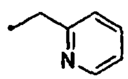 | Br | H | $CF_3$ | H | H | H | n=0 | oil |
| 91 | 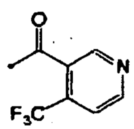 | Cl | Cl | $CF_3$ | H | H | H | n=0 | oil |
| 92 | 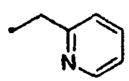 | Br | Cl | $CF_3$ | H | H | H | n=0 | 77-78˚C |
| 93 | 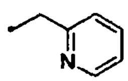 | Br | Br | $CF_3$ | H | H | H | n=0 | 71-73˚C |

(continued)

| No. | $R^1$ | $R^2$ | $R^3$ | $R^4$ | $R^5$ | $R^6$ | $R^7$ | $(R^8)_n$ | Physical properties |
|---|---|---|---|---|---|---|---|---|---|
| 94 | 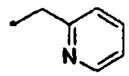 | -C≡CH | Cl | $CF_3$ | H | H | H | n=0 | oil |
| 95 | 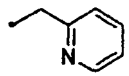 | Me | $NO_2$ | $CF_3$ | H | H | H | n=0 | oil |
| 96 | 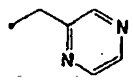 | Cl | Cl | $CF_3$ | H | H | H | n=0 | oil |
| 97 | 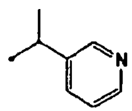 | Cl | Cl | $CF_3$ | H | H | H | n=0 | oil |
| 98 | H | Me | Cl | $CF_3$ | Cl | H | H | n=0 | 85-87°C |
| 99 | 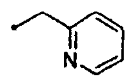 | SMe | Cl | $CF_3$ | H | H | H | n=0 | 103-105°C |
| 100 | 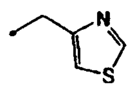 | Cl | Cl | $CF_3$ | H | H | H | n=0 | 68-70°C |
| 101 | H | Me | Cl | $CF_3$ | H | H | H | n=0 | 94-96°C |
| 102 | 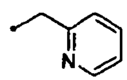 | Me | Cl | $CF_3$ | H | H | H | n=0 | oil |
| 103 | 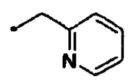 | $CF_3$ | Cl | $CF_3$ | H | H | H | n=0 | 58-59°C |
| 104 | 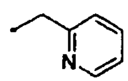 | F | F | $CF_3$ | F | H | H | n=0 | oil |
| 105 | 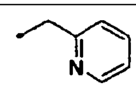 | Ph | CN | $CF_3$ | H | H | H | n=0 | 158-160°C |
| 106 | H | Cl | CN | $CF_3$ | F | H | H | n=0 | 72-73°C |

(continued)

| No. | $R^1$ | $R^2$ | $R^3$ | $R^4$ | $R^5$ | $R^6$ | $R^7$ | $(R^8)_n$ | Physical properties |
|---|---|---|---|---|---|---|---|---|---|
| 107 | 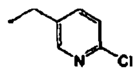 | Cl | Cl | $CF_3$ | H | H | H | n=0 | gummy oil |
| 108 | 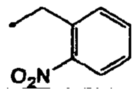 | Cl | Cl | $CF_3$ | H | H | H | n=0 | gummy oil |
| 109 | 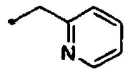 | Cl | CN | $CF_3$ | F | H | H | n=0 | 87-88°C |
| 110 | 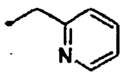 | $CF_3$ | H | $CF_3$ | H | H | H | n=0 | oil |
| 111 | 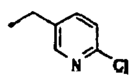 | Cl | Cl | $CF_3$ | H | Me | H | n=0 | oil |
| 112 | H | Cl | Cl | $CF_3$ | H | H | H | 3-Me | 123-126°C |
| 113 | H | Cl | Cl | $CF_3$ | H | H | H | 3-$CF_3$ | 149-151°C |
| 114 | 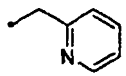 | OMe | $NO_2$ | $CF_3$ | H | H | H | n=0 | oil |
| 115 | 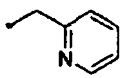 | F | $NO_2$ | $CF_3$ | H | H | H | n=0 | oil |
| 116 | 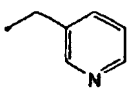 | $CF_3$ | Cl | $CF_3$ | H | H | H | n=0 | oil |
| 117 | H | Cl | H | $CF_3$ | Cl | H | H | n=0 | 110.6°C |
| 118 | H | Cl | Cl | $CF_3$ | H | H | H | n=0 | 112.7°C |
| 119 | H | H | H | $CF_3$ | H | H | H | n=0 | $nD_{31.2}$ = 1.5245 |
| 120 | H | $CF_3$ | H | $CF_3$ | H | H | H | n=0 | 113.2°C |
| 121 | H | F | F | $CF_3$ | F | H | H | n=0 | 87.7°C |
| 122 | H | $CF_3$ | Cl | $CF_3$ | Cl | H | H | n=0 | 160.7 |

(continued)

| No. | $R^1$ | $R^2$ | $R^3$ | $R^4$ | $R^5$ | $R^6$ | $R^7$ | $(R^8)_n$ | Physical properties |
|---|---|---|---|---|---|---|---|---|---|
| 123 | H | OEt | Cl | $CF_3$ | Cl | H | H | n=0 | 112.3°C |
| 124 | H | SMe | Cl | $CF_3$ | Cl | H | H | n=0 | $nD_{30.2}$=1.5426 |
| 125 | H | SMe | SMe | $CF_3$ | Cl | H | H | n=0 | 116.5°C |
| 126 | H | $SO_2Me$ | Cl | $CF_3$ | Cl | H | H | n=0 | 175.1°C |
| 127 | H | Cl | Cl | $CF_3$ | Cl | H | H | 5-$CF_3$ | 141.4°C |
| 128 | H | Cl | H | $CF_3$ | H | H | H | n=0 | 78.6°C |
| 129 | H | Cl | H | $CF_3$ | COOMe | H | H | n=0 | 99.5°C |
| 130 | H | Cl | COOMe | $CF_3$ | H | H | H | n=0 | 101.6°C |
| 131 | H | H | H | $CF_3$ | COOMe | H | H | n=0 | $nD_{29.0}$=1.5229 |
| 132 | H | H | COOMe | $CF_3$ | H | H | H | n=0 | 90.1°C |
| 133 | 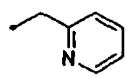 | $CF_3$ | Br | $CF_3$ | H | H | H | n=0 | 57-58°C |
| 134 | 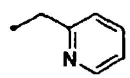 | $CF_3$ | SMe | $CF_3$ | H | H | H | n=0 | oil |
| 135 | 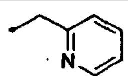 | $CF_3$ | SOMe | $CF_3$ | H | H | H | n=0 | oil |
| 136 | 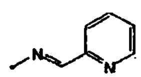 | H | $CF_3$ | $CF_3$ | H | H | H | n=0 | oil |
| 137 | $CH_2Ph$ | Cl | Cl | $CF_3$ | H | H | H | n=0 | oil |
| 138 | COSEt | Cl | Cl | $CF_3$ | H | H | H | n=0 | amorphous |
| 139 | 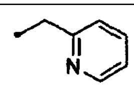 | Cl | Cl | $CF_3$ | H | H | H | 6-$CH_2OCH_2OCH_3$ | oil |
| 140 | 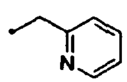 | Cl | Cl | $CF_3$ | H | H | H | 6-$CH_2OH$ | oil |

(continued)

| No. | $R^1$ | $R^2$ | $R^3$ | $R^4$ | $R^5$ | $R^6$ | $R^7$ | $(R^8)_n$ | Physical properties |
|---|---|---|---|---|---|---|---|---|---|
| 141 | 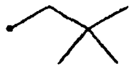 | Cl | Cl | $CF_3$ | H | H | H | n=0 | oil |
| 142 | 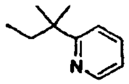 | Cl | Cl | $CF_3$ | H | H | H | n=0 | oil |
| 143 | 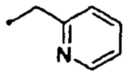 | Cl | Cl | $CF_3$ | H | CN | H | n=0 | |
| 144 | 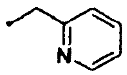 | Cl | Cl | $CF_3$ | H | Me | Me | n=0 | |
| 145 | 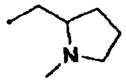 | Cl | Cl | $CF_3$ | H | H | H | n=0 | oil |
| 146 | 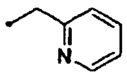 | Cl | Cl | $CF_3$ | H | H | H | 3-OMe | oil |
| 147 | 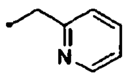 | Cl | Cl | $CF_3$ | H | H | H | 3-Me | oil |
| 148 | 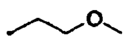 | Cl | Cl | $CF_3$ | H | H | H | n=0 | oil |
| 149 | 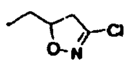 | Cl | Cl | $CF_3$ | H | H | H | n=0 | oil |
| 150 | 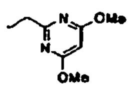 | Cl | Cl | $CF_3$ | H | H | H | n=0 | |
| 151 | 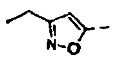 | Cl | Cl | $CF_3$ | H | H | H | n=0 | oil |
| 152 | 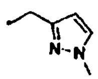 | Cl | Cl | $CF_3$ | H | H | H | n=0 | oil |
| 153 | 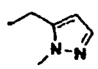 | Cl | Cl | $CF_3$ | H | H | H | n=0 | |

(continued)

| No. | $R^1$ | $R^2$ | $R^3$ | $R^4$ | $R^5$ | $R^6$ | $R^7$ | $(R^8)_n$ | Physical properties |
|---|---|---|---|---|---|---|---|---|---|
| 154 | 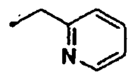 | $CF_3$ | Cl | $CF_3$ | H | H | H | 6-Me | oil |
| 155 | 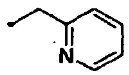 | Cl | Cl | $CF_3$ | H | H | H | 6-Me | oil |
| 156 | 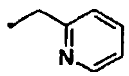 | $CF_3$ | Cl | $CF_3$ | H | H | H | 4-OMe | |
| 157 | 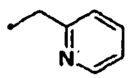 | Cl | Cl | $CF_3$ | H | H | H | 4-OMe | oil |
| 158 | 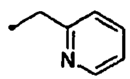 | $CF_3$ | Cl | $CF_3$ | H | H | H | 4-$NMe_2$ | |
| 159 | 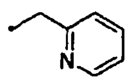 | Cl | Cl | $CF_3$ | H | H | H | 4-$NMe_2$ | |
| 160 | 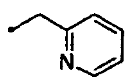 | $CF_3$ | Cl | $CF_3$ | H | H | H | 4-Me | |
| 161 | 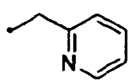 | Cl | Cl | $CF_2$ | H | H | H | 4-Me | oil |
| 162 | 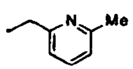 | $CF_3$ | Cl | $CF_3$ | H | H | H | 6-Me | oil |
| 163 | 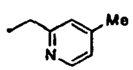 | $CF_3$ | Cl | $CF_3$ | H | H | H | 4-Me | |
| 164 | 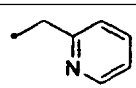 | $CF_3$ | Cl | $CF_3$ | H | Me | H | n=0 | oil |
| 165 | 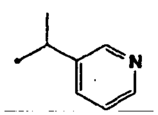 | $CF_3$ | Cl | $CF_3$ | H | H | H | n=0 | oil |
| 166 | Et t | $CF_3$ | Cl | $CF_3$ | H | H | H | n=0 | oil |

(continued)

| No. | $R^1$ | $R^2$ | $R^3$ | $R^4$ | $R^5$ | $R^6$ | $R^7$ | $(R^8)_n$ | Physical properties |
|---|---|---|---|---|---|---|---|---|---|
| 167 | 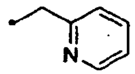 | Cl | $CF_2H$ | $CF_3$ | H | H | H | n=0 | oil |
| 168 | 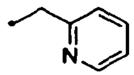 | $CF_2H$ | Cl | $CF_3$ | H | H | H | n=0 | oil |
| 169 | 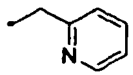 | COMe | Cl | $CF_3$ | H | H | H | n=0 | |
| 170 | 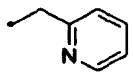 | $CF_2Me$ | Cl | $CF_3$ | H | H | H | n=0 | oil |
| 171 | 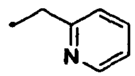 | $CF_3$ | Me | $CF_3$ | H | H | H | n=0 | oil |
| 172 | $CH_2CH_2NHCOOBu$ (t) | Cl | Cl | $CF_3$ | H | H | H | n=0 | 127-131°C |
| 173 | $CH_2CH_2OMe$ | $CF_3$ | Cl | $CF_3$ $CF_3$ | H | H | H | n=0 | oil |
| 174 | H | Cl | Cl I | $CF_3$ | Me | H | H | n=0 | 125-127°C |
| 175 | H | Me | Cl | $CF_3$ | Me | H | H | n=0 | 60-61°C |
| 176 | H | Cl | Cl | $CF_3$ | $NH_2$ | H | H | n=0 | 119-121°C |
| 177 | H | H | CN | $CF_3$ | Me | H | H | n=0 | 100-101°C |
| 178 | H | Cl | CN | $CF_3$ | Me | H | H | n=0 | 139-141°C |
| 179 | H | Cl | Me | $CF_3$ | CN | H | H | n=0 | 93-95°C |
| 180 | H | $CF_3$ | Cl | $CF_3$ | H | H | H | n=0 | 145-146°C |
| 181 | H | $CF_3$ | H | $CF_3$ | Cl | H | H | n=0 | 90-95°C |
| 182 | H | $CF_3$ | Cl | $CF_3$ | Me | H | H | n=0 | 106-107°C |
| 183 | H | $CF_3$ | Cl | $CF_3$ | Me | Me | H | n=0 | 76-78°C |
| 184 | H | $CF_3$ | Cl | $CF_3$ | Et | H | H | n=0 | 91-92°C |
| 185 | H | $CF_3$ | Cl | $CF_3$ | SMe | H | H | n=0 | 98-99°C |
| 186 | H | $CF_3$ | Cl | $CF_3$ | OMe | H | H | n=0 | 84-86°C |

(continued)

| No. | $R^1$ | $R^2$ | $R^3$ | $R^4$ | $R^5$ | $R^6$ | $R^7$ | $(R^8)_n$ | Physical properties |
|---|---|---|---|---|---|---|---|---|---|
| 187 | H | Cl | H | $CF_3$ | $CH_2CH=CH_2$ | H | H | n=0 | 59°C |
| 188 | H | Cl | $CH_2CH=CH_2$ | $CF_3$ | H | H | H | n=0 | oil |
| 189 | H | Cl | H | $CF_3$ | $CH_2C$ (Me) $=CH_2$ | H | H | n=0 | 68-70°C |
| 190 | H | Cl | $CH_2C$ (Me) $=CH_2$ | $CF_3$ | H | H | H | n=0 | oil |
| 191 | H | $CF_3$ | C1 | $CF_3$ | Br | H | H | n=0 | 88-89°C |
| 192 | 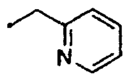 | Br | $CHF_2$ | $CF_3$ | H | H | H | n=0 | oil |
| 193 | 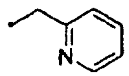 | Cl | $COCF_3$ | $CF_3$ | H | H | H | n=0 | oil |
| 194 | 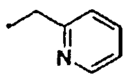 | Cl | $CH(NH_2)CF_3$ | $CF_3$ | H | H | H | n=0 | oil |
| 195 | 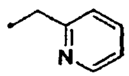 | Cl | COOMe | $CF_3$ | H | H | H | n=0 | oil |
| 196 | 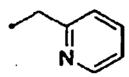 | Cl | $CH_2CH=CH_2$ | $CF_3$ | H | H | H | n=0 | oil |
| 197 | 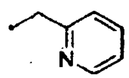 | Cl | $CH_2C(Me)=CH_2$ | $CF_3$ | H | H | H | n=0 | oil |
| 198 | 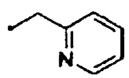 | Cl | CH=N-OMe | $CF_3$ | H | H | H | n=0 | oil |
| 199 | $CH_2CF_3$ | Cl | CN | $CF_3$ | H | H | H | n=0 | 74-78°C |
| 200 | $CH(Mc)CH_2OMe$ | Cl | Cl | $CF_3$ | H | H | H | n=0 | oil |
| 201 | $CH_2CH$ (Me) $OCH_2OMe$ | Cl | Cl | $CF_3$ | H | H | H | n=0 | oil |
| 202 | $CH_2CH_2OH$ | Cl | Cl | $CF_3$ | H | H | H | n=0 | 95-97°C |
| 203 | $CH_2CH(Me)$ OH | Cl | Cl | $CF_3$ | H | H | H | n=0 | 104°C |
| 204 | $CH_2SMe$ | Cl | Cl | $CF_3$ | H | H | H | n=0 | oil |

(continued)

| No. | $R^1$ | $R^2$ | $R^3$ | $R^4$ | $R^5$ | $R^6$ | $R^7$ | $(R^8)_n$ | Physical properties |
|---|---|---|---|---|---|---|---|---|---|
| 205 | $CH_2CH_2SEt$ | Cl | Cl | $CF_3$ | H | H | H | n=0 | oil |
| 206 | $CH_2CH_2SOEt$ | Cl | Cl | $CF_3$ | H | H | H | n=0 | oil |
| 207 | $CH_2CH_2SO_2Et$ | Cl | Cl | $CF_3$ | H | H | H | n=0 | 117°C |
| 208 | 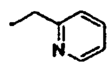 | $CF_3$ | Cl | $CF_3$ | Me | H | H | n=0 | oil |
| 209 | H | Cl | Cl | $CF_3$ | Br | H | H | n=0 | 102-103°C |
| 210 | $CH_2CH\ (OMc)_2$ | Cl | Cl | $CF_3$ | H | H | H | n=0 | oil |
| 211 | 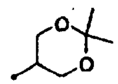 | Cl | Cl | $CF_3$ | H | H | H | n=0 | oil |
| 212 | $CH(CH_2OH)_2$ | Cl | Cl | $CF_3$ | H | H | H | n=0 | 133-135°C |
| 213 | $CH\ (CH_2OCH_2OMe)_2$ | Cl | Cl | $CF_3$ | H | H | H | n=0 | oil |
| 214 | $CH_2CH_2CH_2OMe$ | Cl | Cl | $CF_3$ | H | H | H | n=0 | oil |
| 215 | $CH_2CH_2CH\ (OMe)_2$ | Cl | Cl | $CF_3$ | H | H | H | n=0 | 47°C |
| 216 | $CH_2CH(OH)CH_2OH$ | Cl | Cl | $CF_3$ | H | H | H | n=0 | 131-134°C |
| 217 | $CH_2CH(SEt)OMe$ | Cl | Cl | $CF_3$ | H | H | H | n=0 | oil |
| 218 | $CH_2CH(SEt)_2$ | Cl | Cl | $CF_3$ | H | H | H | n=0 | oil |
| 219 | $CH_2CH(SEt)_2$ | $CF_3$ | Cl | $CF_3$ | H | H | H | n=0 | oil |
| 220 | $CH_2CH(SEt)_2$ | Cl | Cl | $CF_3$ | H | H | H | n=0 | oil |
| 221 | $CH_2CH=CCl_2$ | Cl | Cl | $CF_3$ | H | H | H | n=0 | amorphous |
| 222 | $CH_2COOEt$ | Cl | Cl | $CF_3$ | H | H | H | n=0 | 69°C |
| 223 | $CH_2C00Et$ | $CF_3$ | Cl | $CF_3$ | H | H | H | n=0 | 67-69°C |
| 224 | $CH_2COOEt$ | Cl | CN | $CF_3$ | H | H | H | n=0 | 97°C |
| 225 | CH(Me)COOMe | Cl | Cl | $CF_3$ | H | H | H | n=0 | oil |
| 226 | $CH(COOMe)CHMe_2$ | Cl | Cl | $CF_3$ | H | H | H | n=0 | oil |
| 227 | $-C(COOMe)=CH_2$ | Cl | Cl | $CF_3$ | H | H | H | n=0 | oil |

(continued)

| No. | $R^1$ | $R^2$ | $R^3$ | $R^4$ | $R^5$ | $R^6$ | $R^7$ | $(R^8)_n$ | Physical properties |
|---|---|---|---|---|---|---|---|---|---|
| 228 | $CH_2COOCH_2CH_2OMe$ | Cl | Cl | $CF_3$ | H | H | H | n=0 | oil |
| 229 | $CH_2COOCH_2CH_2OH$ | Cl | Cl | $CF_3$ | H | H | H | n=0 | oil |
| 230 | $CH_2COOBu(t)$ | Cl | Cl | $CF_3$ | H | H | H | n=0 | 57-60°C |
| 231 | $CH_2COOH$ | Cl | Cl | $CF_3$ | H | H | H | n=0 | 164°C |
| 232 | $CH_2CONH_2$ | Cl | Cl | $CF_3$ | H | H | H | n=0 | 165-166°C |
| 233 | $CH_2CONHCH_2CH_2OMe$ | Cl | Cl | $CF_3$ | H | H | H | n=0 | oil |
| 234 | $CH_2CONEt_2$ | Cl | Cl | $CF_3$ | H | H | H | n=0 | 79-81°C |
| 235 | 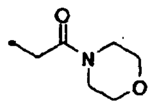 | Cl | Cl | $CF_3$ | H | H | H | n=0 | oil |
| 236 | $CH_2CHO$ | Cl | Cl | $CF_3$ | H | H | H | n=0 | oil |
| 237 | $CH_2COMe$ | Cl | Cl | $CF_3$ | H | H | H | n=0 | 80-81°C |
| 238 | $CH_2CN$ | Cl | CN | $CF_3$ | H | H | H | n=0 | 85-86°C |
| 239 | $CH_2CH_2COOH$ | Cl | Cl | $CF_3$ | H | H | H | n=0 | 104°C |
| 240 | $CH_2CH_2COOMe$ | Cl | Cl | $CF_3$ | H | H | H | n=0 | oil |
| 241 | CN | Cl | Cl | $CF_3$ | H | H | H | n=0 | 166-167°C |
| 242 | 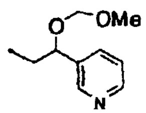 | Cl | Cl | $CF_3$ | H | H | H | n=0 | oil |
| 243 | 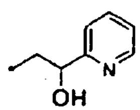 | Cl | Cl | $CF_3$ | H | H | H | n=0 | 139°C |
| 244 | 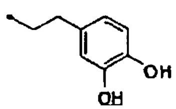 | Cl | H | $CF_3$ | H | H | H | n=0 | 130-133°C |

(continued)

| No. | $R^1$ | $R^2$ | $R^3$ | $R^4$ | $R^5$ | $R^6$ | $R^7$ | $(R^8)_n$ | Physical properties |
|---|---|---|---|---|---|---|---|---|---|
| 245 | 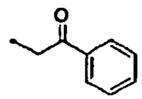 | Cl | H | $CF_3$ | H | H | H | n=0 | 112-116°C |
| 246 | 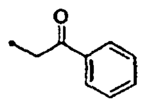 | Cl | Cl | $CF_3$ | H | H | H | n=0 | 125°C |
| 247 | 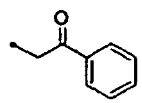 | Cl | CN | $CF_3$ | H | H | H | n=0 | 131°C |
| 248 | 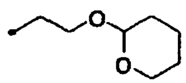 | Cl | Cl | $CF_3$ | H | H | H | n=0 | oil |
| 249 | 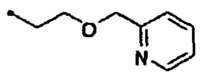 | Cl | Cl | $CF_3$ | H | H | H | n=0 | oil |
| 250 | 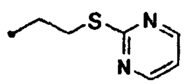 | Cl | Cl | $CF_3$ | H | H | H | n=0 | 91-93 |
| 251 | 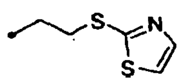 | Cl | Cl | $CF_3$ | H | H | H | n=0 | 97°C |
| 252 | 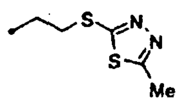 | Cl | Cl | $CF_3$ | H | H | H | n=0 | 102°C |
| 253 | 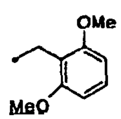 | Cl | Cl | | H | H | H | n=0 | 113-116°C |
| 254 | 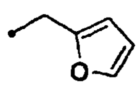 | Cl | Cl | | H | H | H | n=0 | oil |
| 255 | 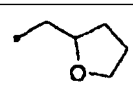 | Cl | Cl | $CF_3$ | H | H | H | n=0 | oil |

(continued)

| No. | $R^1$ | $R^2$ | $R^3$ | $R^4$ | $R^5$ | $R^6$ | $R^7$ | $(R^8)_n$ | Physical properties |
|---|---|---|---|---|---|---|---|---|---|
| 256 | 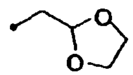 | Cl | Cl | $CF_3$ | H | H | H | n=0 | oil |
| 257 | 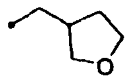 | Cl | Cl | $CF_3$ | H | H | H | n=0 | oil |
| 258 | 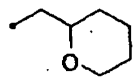 | Cl | Cl | $CF_3$ | H | H | H | n=0 | oil |
| 259 | 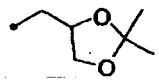 | Cl | Cl | $CF_3$ | H | H | H | n=0 | 108°C |
| 260 | 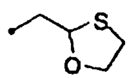 | Cl | Cl | $CF_3$ | H | H | H | n=0 | oil |
| 261 | 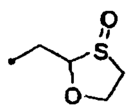 | Cl | Cl | $CF_3$ | H | H | H | n=0 | oil |
| 262 | 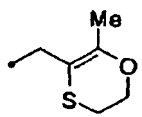 | Cl | Cl | $CF_3$ | H | H | H | n=0 | oil |
| 263 | 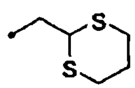 | Cl | Cl | $CF_3$ | H | H | H | n=0 | oil |
| 264 | 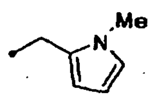 | Cl | H | $CF_3$ | H | H | H | n=0 | oil |
| 265 | 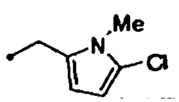 | Cl | H | $CF_3$ | H | H | H | n=0 | oil |
| 266 | 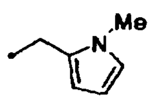 | Cl | CN | $CF_3$ | H | H | H | n=0 | 127-128°C |

(continued)

| No. | $R^1$ | $R^2$ | $R^3$ | $R^4$ | $R^5$ | $R^6$ | $R^7$ | $(R^8)_n$ | Physical properties |
|---|---|---|---|---|---|---|---|---|---|
| 267 | 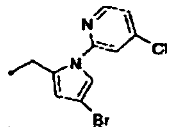 | Cl | Cl | $CF_3$ | H | H | H | n=0 | oil |
| 268 | 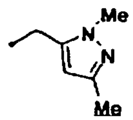 | Cl | Cl | $CF_3$ | H | H | H | n=0 | oil |
| 269 | 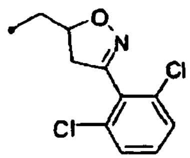 | Cl | Cl | $CF_3$ | H | H | H | n=0 | amorphous |
| 270 | 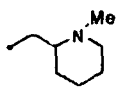 | Cl | Cl | $CF_3$ | H | H | H | n=0 | oil |
| 271 | $COCHCl_2$ | Cl | Cl | $CF_3$ | H | H | H | n=0 | oil |
| 272 | 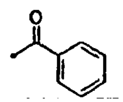 | Cl | Cl | $CF_3$ | H | H | H | n=0 | oil |
| 273 | 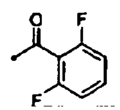 | Cl | Cl | $CF_3$ | H | H | H | n=0 | oil |
| 274 | 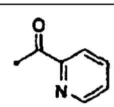 | Cl | CN | $CF_3$ | H | H | H | n=0 | 48-49°C |
| 275 | $SO_2Et$ | Cl | Cl | $CF_3$ | H | H | H | n=0 | 71-75°C |
| 276 | $SO_2Ph$ | Cl | Cl | $CF_3$ | H | H | H | n=0 | 106-108°C |
| 277 | 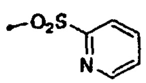 | Cl | Cl | $CF_3$ | H | H | H | n=0 | 124-126°C |

(continued)

| No. | $R^1$ | $R^2$ | $R^3$ | $R^4$ | $R^5$ | $R^6$ | $R^7$ | $(R^8)_n$ | Physical properties |
|---|---|---|---|---|---|---|---|---|---|
| 278 | H | $CF_3$ | Br | $CF_3$ | H | H | H | n=0 | 158-159°C |
| 279 | 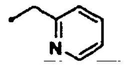 | Cl | Cl | $CF_3$ | H | Me | H | n=0 | oil |
| 280 | 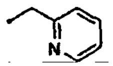 | Cl | CN | $CF_3$ | H | Me | H | n=0 | oil |
| 281 | H | $CF_3$ | H | $CF_3$ | Cl | Me | H | n=0 | 63-64°C |
| 282 | H | $CF_3$ | Cl | $CF_3$ | H | Me | H | n=0 | 125-127°C |
| 283 | Me | $CF_3$ | Cl | $CF_3$ | H | Me | H | n=0 | oil |
| 284 | H | Cl | CN | $CF_3$ | H | Me | Me | n=0 | 98-102°C |
| 285 | 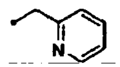 | Cl | CN | $CF_3$ | H | Me | Me | n=0 | 99-101°C |
| 286 | H | $CF_3$ | CN | $CF_3$ | H | H | H | n=0 | 122-123°C |
| 287 | 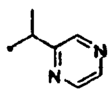 | $CF_3$ | Cl | $CF_3$ | H | H | H | n=0 | oil |
| 288 | 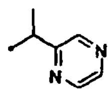 | Cl | Cl | $CF_3$ | H | H | H | n=0 | oil |
| 289 | 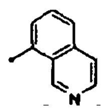 | Cl | CN | $CF_3$ | H | H | H | n=0 | amorphous |
| 290 | 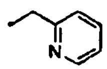 | Cl | Cl | $CF_3$ | H | H | H | 5-Me | 88-89°C |
| 291 | 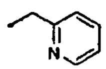 | Cl | Cl | $CF_3$ | H | H | H | 5-F | oil |
| 292 | 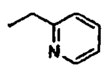 | $CF_3$ | Cl | $CF_3$ | H | H | H | 5-F | amorphous |
| 293 | 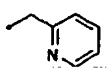 | Cl | CN | $CF_3$ | H | H | H | 6-Me | oil |

(continued)

| No. | $R^1$ | $R^2$ | $R^3$ | $R^4$ | $R^5$ | $R^6$ | $R^7$ | $(R^8)_n$ | Physical properties |
|---|---|---|---|---|---|---|---|---|---|
| 294 | 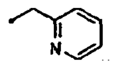 | OMc | Cl | $CF_3$ | H | H | H | 6-0Me | oil |
| 295 | 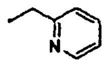 | Cl | Cl | $CF_3$ | H | H | H | 6-0Me | oil |
| 296 | 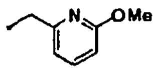 | Cl | Cl | $CF_3$ | H | H | H | 6-0Me | oil |
| 297 | 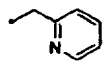 | Cl | Cl | $CF_3$ | H | H | H | 6-Br | oil |
| 298 | 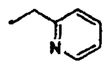 | Cl | Cl | $CF_3$ | H | H | H | 6-F | oil |
| 299 | 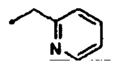 | $CF_3$ | Cl | $CF_3$ | H | H | H | 6-F | oil |
| 300 | 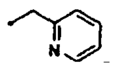 | Cl | Cl | $CF_3$ | H | H | H | 6-(2-pyridyl) | 97-99°C |
| 301 | 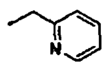 | Cl | Cl | $CF_3$ | H | H | H | 6-Ph | oil |
| 302 | 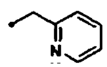 | Cl | Cl | $CF_3$ | H | H | H | 6- (2-pyridyloxy) | oil |
| 303 | 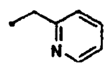 | Cl | Cl | $CF_3$ | H | H | H | 6-$NH_2$ | 82-85°C |
| 304 | 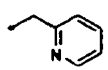 | Cl | Cl | $CF_3$ | H | H | H | 6-COOMe | oil |
| 305 | 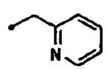 | Cl | Cl | $CF_3$ | H | H | H | 6-COOH | oil |
| 306 | 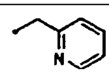 | Cl | Cl | $CF_3$ | H | H | H | 6-CN | oil |
| 307 | 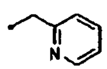 | Cl | Cl | $CF_3$ | H | H | H | 3, 5-Me. 4-OMe | 73-75°C |

(continued)

| No. | $R^1$ | $R^2$ | $R^3$ | $R^4$ | $R^5$ | $R^6$ | $R^7$ | $(R^8)_n$ | Physical properties |
|---|---|---|---|---|---|---|---|---|---|
| 308 | 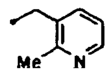 | Cl | Cl | $CF_3$ | H | H | H | n=0 | oil |
| 309 | 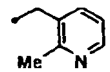 | Cl | CN | $CF_3$ | H | H | H | n=0 | 126°C |
| 310 | 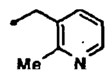 | CF3 | Cl | $CF_3$ | H | H | H | n=0 | oil |
| 311 | 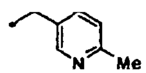 | Cl | Cl | $CF_3$ | H | H | H | n=0 | oil |
| 312 | 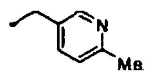 | $CF_3$ | Cl | $CF_3$ | H | H | H | n=0 | oil |
| 313 | 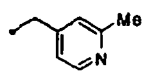 | Cl | Cl | $CF_3$ | H | H | H | n=0 | oil |
| 314 | 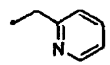 | $CF_3$ | CN | $CF_3$ | H | H | H | n=0 | 70-71°C |
| 315 | $CH_2CH(OMe)_2$ | $CF_3$ | Br | $CF_3$ | H | H | H | n=0 | |
| 316 | 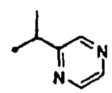 | Cl | CN | $CF_3$ | H | H | H | n=0 | 89-90°C |
| 317 | 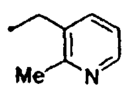 | $CF_3$ | Br | $CF_3$ | H | H | H | n=0 | 104-105°C |
| 318 | 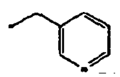 | $CF_3$ | Br | $CF_3$ | H | H | H | n=0 | oil |
| 319 | 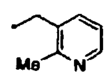 | $CF_3$ | CN | $CF_3$ | H | H | H | n=0 | 84-85°C |
| 320 | 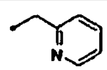 | $CF_3$ | CN | $CF_3$ | H | Me | H | n=0 | oil |

(continued)

| No. | $R^1$ | $R^2$ | $R^3$ | $R^4$ | $R^5$ | $R^6$ | $R^7$ | $(R^8)_n$ | Physical properties |
|---|---|---|---|---|---|---|---|---|---|
| 321 | 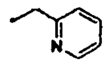 | $NMC_2$ | CN | $CF_3$ | H | H | H | n=0 | oil |
| 322 | H | Cl | CN | $CF_3$ | H | H | H | 6-piperidino | 90-92°C |
| 323 | H | Cl | CN | $CF_3$ | H | H | H | 6-norphnlinyl | 150-152°C |
| 324 | 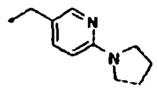 | Cl | CN | $CF_3$ | H | H | H | n=0 | oil |
| 325 | 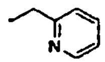 | Cl | CN | $CF_3$ | H | H | H | 6-piperidino | 91°C |
| 326 | 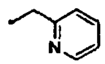 | Cl | CN | $CF_3$ | H | H | H | 6-morpholinyl | 94°C |
| 327 | 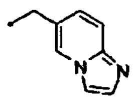 | Cl | CN | $CF_3$ | H | H | H | n=0 | 123-124°C |
| 328 | H | Cl | CN | $CF_3$ | H | Me | H | 6-Me | 118-119°C |
| 329 | 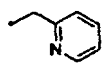 | Cl | CN | $CF_3$ | H | Me | H | 6-Me | oil |
| 330 | Hydrochloride of Compound NO. 24 | | | | | | | | 144-146°C |
| 331 | Hydrochloride of Compound No. 103 | | | | | | | | 132-134°C |
| 332 | Hydrochloride of Compound No. 314 | | | | | | | | 142-145°C |
| 333 | COOMe | Cl | Cl | $CF_3$ | H | H | H | n=0 | |
| 334 | COOEt | $CF_3$ | Br | $CF_3$ | H | Me | H | n=0 | |
| 335 | 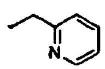 | Cl | CN | $CF_3$ | H | $-(CH_2)_3-$ | | n=0 | |
| 336 | 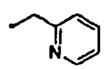 | Cl | H | $CF_3$ | H | H | H | n=0 | |
| 337 | 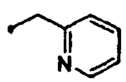 | CN | CN | $CF_3$ | H | H | H | n=0 | |

(continued)

| No. | $R^1$ | $R^2$ | $R^3$ | $R^4$ | $R^5$ | $R^6$ | $R^7$ | $(R^8)_n$ | Physical properties |
|---|---|---|---|---|---|---|---|---|---|
| 338 | 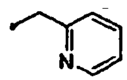 | $CF_3$ | CN | $CF_3$ | H | Et | H | n=0 | |
| 339 | 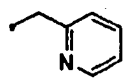 | $CF_3$ | $CHF_2$ | $CF_3$ | H | H | H | n=0 | |
| 340 | 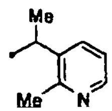 | $CF_3$ | Cl | $CF_3$ | H | H | H | n=0 | |

TABLE 2

| No. | $^1$H-NMR (δ ppm (Solvent : $CDCl_3$ /400MHz) |
|---|---|
| 4 | 4.83(4H, s), 7. 18 (2H. dd, J = 5.2.6. 8Hz), 7. 47 (2H, d. J = 8.0Hz) 7. 66 (1H, td, J = 8. 0, 2.0Hz , 8.54(2H, d, J = 5. 2Hz) |
| 7 | 5. 01 (4H, s), 6.73(1H, s), 6.79(1H, s), 6.78(1H d, J = 5.6Hz), 7. 18 (2H, dd, J =8.0, 5.2Hz), 7. 22 (2H. d. J = 8.0Hz), 7. 62 (2H. ddd, J = 8.0. 7.2. 2.0Hz), 8.30(1H, d, J =5.6Hz), 8. 57 (2H, d, J = 5. 2Hz) |
| 9 | (Solvent : DMSO-$d_6$ 5.12(4H, brs), 7,24(1H, s), 7. 29 (4H, brs), 7.7 5(2H, brs), 8. 51 (2H, brs), 8.95 (1H. s) |
| 15 | 4. 74 (2H, d, J = 5. 2Hz). 6.06(1H, s), 6. 82 (1H, s). 7. 19-7. 24 (1H. m ) . 7. 30-7. 45 (6H, m), 7.69 (1H, td, J =7.6. 1.7Hz), 8.11(1H. s), 8 60 (1H, d. J = 5. 2Hz) |
| 17 | 5. 06 (4H, s), 6.96 (1H. s) . 7.17-7.24 (4H, m). 7.64(2H. td. J =7.6. 1.7Hz), 8.56 (1H, s), 8. 57-8. 58 (2H, m) |
| 18 | 4.98(4H. s), 6.83 (1H, s), 7.18-7.20 (2H, m), 7.21 (2H, d, J =8. 0Hz ), 7.63(2H, td, J =7.7, 1.5Hz), 8.23 (1H, s), 8.7(2H, d, J =4.8 Hz) |
| 23 | 4.66 (2H, s), 4.70 (2H, s), 7.18 (1H, dd, J = 8. 0. 5. 2Hz), 7. 24-7.31 (5H, m), 7.40 (1H, d, J = 7.6Hz), 7.67 (1H, td, J =8.0. 2.0Hz), 8.55 (1H, d, J=8.0Hz) |
| 27 | 2.58 (3H, s), 4.69 (2H, d, J =4.8Hz), 6.61(1H, brs), 6. 67(1H, s), 7.23-7.26 (1H, m), 7.32(1H, d. J =8.0Hz), 7.70(1H td, J =7.7,1 . 9Hz), 8. 56 (1H, d. J =4.8Hz) |
| 29 | 2.56(3H, s) 4.99(4H, s), 6.71 (1H, s), 7. 20-7. 24 (4H, m), 7.65 (2H, td. J = 7. 7, 1. 7Hz), 8.56 (2H, d, J = 4.4Hz) |
| 32 | 2.58 (3H, s), 4. 71 (4H, s), 7.05 (1H, s), 7.17 (2H, dd, J = 7.2. 4. 8H z), 7.26 (2H, d, J = 5.2Hz), 7.63 (2H, td, J = 7.7, 1.9Hz), 8.53(2H d, J = 5.2Hz) |
| 35 | 5.02 (4H, brs), 6.83 (1H, s), 7.22-7.34 (4H, m), 7.68 (2H, td, J = 7. 7. 1.9Hz), 8.57 (2H, d, J = 4.4Hz) |
| 36 | 4.84 (4H, s), 6.95 (1H, s), 7.20(2H, ddd, J = 7.2, 4.8, 0.9Hz), 7. 3 0 (2H, d, J=7.6Hz), 7.67 (2H, td, J=7.7. 1.6Hz), 8.53 (2H, dt, J = 4. 8, 1.0Hz) |
| 38 | 4.90 (4H, s), 7.08 (1H, s), 7.17 (2H, dd, J = 7.2. 5.2Hz), 7.47(2H, d, J = 7. 2Hz), 7.66(2H, td. J = 7.7, 1.7Hz), 8.54 (2H, d, J = 4. 8H z) |
| 39 | 5.02 (4H, s). 7.21 (2H. ddd. J = 7. 4. 5.0. 1.0Hz). 7.41 (2H. d. J = 8. 0Hz), 7. 69 (2H. td, J = 7. 5. 1. 7Hz). 8. 55 (2H. dt, J = 5. 2, 1. 0Hz) |
| 42 | 4.99(4H. s). 6.60 (1H. s). 6.78 (1H, s), 7.20 (2H, dd, J = 7.2, 4. 8H z), 7. 26-7. 28 (2H, m). 7.64 (2H, td, J = 7.7, 1.9Hz). 8.56 (2H, dd. J = 5.0. 1.0Hz) |
| 43 | 4.96 (4H, s), 6.75 (1H. s). 7.20 (2H, dd, J = 7.2. 4.8Hz), 7.26 (2H, d. J = 7.6Hz). 7.65 (2H. td. J = 7.7, 1.9Hz). 8.56 (2H. dt. J = 4. 8 . 1.0Hz) |
| 46 | 5.01 (4H, s). 7.02 (1H. s). 7.12 (1H, s). 7.21 (2H, dd, J = 7.8, 5.0H z). 7.26 (2H, d, J = 8.0Hz). 7.65 (2H, td, J = 7.6, 1.9Hz), 8. 56 (2H dt, J = 4.8, 1.0Hz) |
| 48 | 4.98 (4H, s). 7.17 (1H, s), 7.20-7.25 (4H. m), 7.66 (2H, td, J = 7.8, 1. 7Hz). 8.56 (2H, d, J = 4.8Hz) |
| 49 | 2.89 (3H, s), 3.12 (3H, s), 4.92-5.06 (4H. m), 6.73 (1H. s). 7.19-7.2 5 (4H, m). 7.5 (2H. td. J = 7.7, 1.9Hz), 8.56 (2H, dt, J = 5.2, 1.1 Hz) |
| 50 | 1.57 (2H, brs). 2.96 (2H, t, J = 6.4Hz), 3.69 (2H, t, 1 = 6.4Hz). 4. 79 (2H. s). 6.70 (1H, s). 8.16 (1H, brt. J = 5.0Hz), 8.20 (1H, d, J = 8.0Hz). 8.61 (1H, td. J = 7.6, 1.5Hz), 8.51 (1H, d, J = 4.4Hz) |
| 52 | 0.67 (3H, d, J = 6.4Hz), 1.18 (3H, d, J = 6.4Hz), 1.26 (1H, t. J = 7 2Hz), 2. 53 (1H, d, J = 8.8Hz). 4.98 (4H, d, J = 3.6Hz), 6. 73 (1H, s) 7.20 (2H, dd, J = 7.2, 4.8Hz), 7.25-7.26 (2H, m). 7.64 (2H, td, J = 7.6, 1.7Hz), 8.56 (2H, d. J = 4. 8Hz) |
| 53 | 4.80 (2H, s). 4.94 (2H, s), 6.72 (1H, s). 7.18 (1H, d, J = 8.0Hz), 7. 22 (2H. dd, J = 7.4, 5.0Hz), 7.26-7.29 (1H, m), 7.63-7.68 (2H, m), 8 . 54-8. 58 (3H, m) |
| 54 | 4.83 (2H, s), 4.93 (2H, s), 6.69 (1H, s). 7.17 (2H, d, J = 6.0Hz), 7. 21-7.24 (2H, m), 7.66 (1H, td, J = 7.7, 1.9Hz), 8.56 (1H, d, J = 5.2 Hz), 8.56 (1H, d. J = 6. 4Hz) |

(continued)

| No. | $^1$H-NMR (δ ppm (Solvent : $CDCl_3$ /400MHz) |
|---|---|
| 55 | 4.22 (2H, d, J = 4. 8Hz), 4.84 (2H, s), 5.20 (1H, dd, J = 28. 6. 1.4Hz ), 5.21 (1H, s), 5. 79-5. 86 (1H, m), 6.69 (1H, s), 7. 19-7.26 (2H, m), 7.65 (1H, t, J = 6.8Hz), 8.56 (1H, d. J = 4.0Hz) |
| 57 | 5.01 (2H, s), 5.15 (2H, s), 6.93 (1H, s), 7.17-7.20 (1H, m), 7.22-7.2 9 (1H, m), 7.39 (1H, d, J 8. 0Hz), 7.51 (1H, dd, J = 7.6, 6.4Hz), 7 60-7. 70 (2H, m), 7.79 (1H, d. J = 7.6Hz), 8.02 (1H, d. J = 8.8Hz), 8 10 (1H, d, J = 8.0Hz), 8.56 (1H, d, J = 4. 4Hz) |
| 58 | 4.97 (4H, s), 6.35 (1H, d, J = 2.0Hz), 6.57 (1H, s). 7.19 (2H, dd, J = 8.0, 4.8Hz), 7.24 (2H, d, J = 7.6Hz), 7.64 (2H, td, J = 7.7, 1.7H z), 8.56 (2H, dd. J = 4.8, 0.8Hz) |
| 59 | 4.96 (2H, s), 5.04 (2H, s), 7.14-7.25 (2H, m), 7.61 (1H, d, J = 7.6Hz ). 7. 69 (1H, td, J = 7.2, 1.2Hz), 8.56 (1H, dd, J = 4.8, 1.0Hz). 8. 67 (2H. d, J =4.8Hz) |
| 62 | 2.67 (6H, s), 2.53 (2H, t, J = 6.8Hz), 3.75 (2H, t, J = 6.8Hz), 4. 83 (2H, s), 6.70 (1H, s), 7.19-7.23 (2H, m), 7.64 (1H, td, J = 7.6, 1. 7 Hz), 8.57 (1H, d, J = 4. 8Hz) |
| 63 | 4.96 (4H, s), 6. 72 (1H, s), 7.20 (2H, dd, J = 7.8, 5.0Hz), 7.24 (2H, d. J = 7.6Hz), 7.64 (2H, td, J = 7.8, 2.0Hz), 8.56 (2H, d, J = 4.8H z) |
| 64 | 2.51 (4H, brs), 2. 59 (2H, t, J = 6.4Hz), 3.67 (4H, brs). 3.79 (2H, t, J = 6.4Hz), 4.82 (2H, s), 6.70 (1H, s), 7.19-7.24 (2H, m), 7.64 (1H, td, J = 7.6, 1.7Hz), 8.57 (1H, d, J = 4. 8Hz) |
| 65 | 1.58 (9H, s), 4.79 (2H, s), 6.49 (1H, s), 6.77 (1H, s), 7.19 (1H, dd, J = 7.6, 5.2Hz), 7. 24 (1H, d. J = 7.6Hz), 7.66 (1H, td, J = 7.6, 2.0 Hz). 8. 59 (1H, dd. J = 4.8, 1.2Hz) |
| 67 | 1.78 (3H, d, J = 6.8Hz), 4.62 (2H, s), 5.29 (1H, q, J = 6.8Hz), 7. 10 (1H, dd, J = 6. 8. 4.8Hz), 7.21 (1H, dd, J = 8.4, 2. 8Hz), 7.28 (1H, d. J = 2. 8Hz), 7.51-7.56 (2H, m), 7. 65 (1H, td, J = 8.0, 2.0Hz), 8. 47 (1H, dd. J = 4.0, 1.2Hz), 8.55 (1H, dd. J = 4.0, 2.0Hz) |
| 70 | 1.68 (3H, d. J = 6.8Hz), 4.11 (1H, d, J = 6.8Hz), 4.14 (1H, d. J = 6 .8Hz), 6.12-6.13 (1H, m), 6.61 (1H, s), 7.02 (1H, d, J = 8.0Hz), 7.1 2-7.17 (2H, m), 7.34 (1H, d, J = 7.6Hz), 7.53-7.57 (1H, m), 7.59-7.6 3 (1H, m), 8. 51-8.55 (2H, m) |
| 71 | 5.03 (2H, s), 5.04 (2H, s), 6.84 (1H, s), 7. 18-7. 22 (2H, m), 7.36 (1H, d, J =4.8Hz), 7. 66 (1H, td, J = 8.0, 2.0Hz), 8. 56 (1H, dd, J = 4.8 , 1.0Hz), 8.69 (2H, d, J = 4.8Hz) |
| 76 | 0.87 (3H, d, J = 6.8Hz), 0.99 (3H, d, J = 6, 8Hz), 2. 85-2. 91 (1H, m), 4.72 (1H, d, J = 17.6Hz), 5. 25 (1H, d, J = 17.6Hz), 5.75-5.85 (1H, m), 6.16 (1H, d. J = 7.6Hz), 6. 61 (1H, s). 7.00 (1H, dd, J = 6.8, 5. 6Hz), 7.09-7.12 (1H, m), 7.22-7.28 (1H, m), 7.46 (1H, d, J = 7.6Hz), 7.60-7.64 (1H, m), 8.40 (1H, dd, J = 4.8, 0.8Hz), 8.44 (1H, dd, J = 2.0, 1.2Hz) |
| 79 | 0.91 (3H, t, J = 7.2Hz), 2-04-2.11) (1H, m), 2.25-2.34 (1H, m), 4. 82 ( 1H, d, J = 17.6Hz), 4.92 (1H, d, J = 17.6Hz), 5. 89-5.95 (1H, m), 6.6 5 (1H, s), 6.80 (1H, d, J = 7.6Hz), 7.08-7.16 (2H, m), 7.41 (1H, d, J = 8.0Hz), 7.44-7.48 (1H, m), 7.61 (1H, td, J = 8.0, 2.0Hz), 8. 50 (2 H, ddd, J = 2.8, 2.0, 0.8Hz) |
| 80 | 2.25 (1H, t, J = 2.6Hz), 4. 43 (2H. d. J = 2.4Hz), 4.91 (2H, s), 6.84 (1H, s), 7.21 (1H, dd, J = 6.8, 5.2Hz), 7.29 (1H, d, J = 8.0Hz), 7. 66 (1H, td, J = 7. 8. 2.0Hz), 8.56-8.58 (1H, m) |
| 81 | 2.85 (6H, s), 4.95 (4H, s), 6. 36 (1H, s), 7.16-7.19 (2H, m), 7.21 (2H, d. J =8.0Hz), 7.62 (2H, td, J = 7.7, 1.6Hz), 8.55-8.57 (2H, m) |
| 82 | 3.71 (3H, s), 5.09 (2H, s), 7.21 (2H, dd, J = 7.6, 5.4Hz), 7.24 (1H, s), 7.28-7.32 (1H, m), 7.65 (1H, td, J = 7.6, 1.7Hz), 8.58 (1H, d, J = 3.6Hz) |
| 83 | 5.05 (4H, brs), 6.95 (1H, s), 7.20-7.23 (4H, m), 7.65 (2H, td, J = 7. 6, 1.7Hz), 8.54 (1H, s), 8.57 (2H, d, J = 4.4Hz) |
| 86 | 0.46-0.52 (3H, m), 0.71-0.75 (1H, m), 1.59-1.64 (1H, m), 4.84 (1H, d, J = 17.2Hz), 5.10-5.14 (1H, m), 5.12 (1H, d, J = 17.2Hz), 6.61 (1H, s), 7.13-7.20 (3H, s), 7.45 (1H, d, J = 8.0Hz), 7.56 (1H, dd, J = 8 .0, 1.6Hz), 7.64 (1H, td, J = 7.6, 2.0Hz), 8.54 (2H, dd, J = 4.8, 1 .4Hz) |
| 90 | 4,98 (4H, s), 6.64 (1H, s), 6.92 (1H, s), 7.17-7.20 (2H, m), 7.25-7.2 7 (2H, m). 7.63 (2H, td, J = 8.0, 2.0Hz), 8. 55-8. 57 (2H, m) |
| 91 | 5.32 (2H, brs), 7.20-7.23 (2H, m), 7.34 (1H, brs), 7. 60 (1H, d, J = 4 .8Hz), 7.67 (1H, t, J = 7.4Hz), 8. 54 (1H, d, J = 4,4Hz), 8.80 (2H, d . J = 5.2Hz) |

(continued)

| No. | $^1$H-NMR (δ ppm (Solvent : $CDCl_3$ /400MHz) |
|---|---|
| 94 | 4.98 (4H, s), 6. 87 (1H, s), 7.17-7.20 (2H, m), 7.23-7.25 (2H, m), 7.6 3 (2H, td, J = 7.6, 2.0Hz), 8.56 (2H, dd, J = 4.8, 1.0Hz) |
| 95 | 5.04 (4H, s), 6.71 (1H, s), 7.19-7.23 (4H, m), 7.65 (2H, td, J = 7.8, 1.6Hz), 8. 56 (2H, d. J 4.0Hz) |
| 96 | 4.96 (2H, s), 5.03 (2H, s). 6. 85 (1H, s), 7. 19-7.22 (1H, m), 7.24-7.2 9 (1H, m), 7.63-7.69 (1H, m), 8.49-8.57 (3H, m), 8. 62 (1H, s) |
| 97 | 1.66 (3H, d, J = 6. 8Hz), 4.58 (2H, s), 6.25 (1H, q, J = 6.8Hz), 6.60 (1H, s). 6.99 (1H, d. J = 8.4Hz), 7.14-7.17 (1H, m), 7.23 (1H, dd, J = 8.0, 9.8Hz), 7. 27-7. 59 (1H, m), 7.65 (1H, d, J = 8.0Hz), 8.52 (2H , dd, J = 4.8, 1.2Hz), 8. 63 (1H, brs) |
| 102 | 2.51 (3H, s), 4.98 (4H, s), 6.66 (1H, s), 7.16-7.23 (4H, m), 7.59-7.6 4 (2H, m), 8.56 (2H, dd, J = 4.8, 1.2Hz) |
| 104 | 4.92 (4H, s), 7.16-7.19 (2H, m), 7.29 (2H, d, J = 8.0Hz), 7. 63-7. 67 ( 2H, m), 8. 53-8. 57 (2H, m) |
| 107 | 4.77 (2H, s). 4.91 (2H, s), 6.73 (1H, s), 7.17 (1H, d, J = 7.6Hz), 7. 21-7. 26 (1H, m), 7.28 (1H, d, J = 7.6Hz), 7.60-7.68 (2H, m), 8.32 (1H , d. J = 2.0Hz), 8.56 (1H, ddd, J = 4.8, 2.0, 1.2Hz) |
| 108 | 4.85 (2H, s), 5.27 (2H, s), 6.72 (1H, s), 7. 19-7.23 (1H, m), 7.26-7.2 8 (1H, m), 7.34 (1H, d, J = 8.0Hz), 7.43-7.49 (1H, m), 7.56-7.60 (1H, m), 7.64-7.68 (1H, m), 8.13 (1H, dd, J = 8.0, 0.8Hz), 8.54 (1H, dd, J = 8.0, 0.8Hz) |
| 110 | 5.04 (4H, s), 6.93 (1H, s), 7.09 (1H, s), 7. 18-7.21 (2H, m), 7.30 (2H, d, J =7.6Hz), 7.63 (2H, td, J = 7.7, 1.9Hz), 8.56 (2H, dt, J = 4.0 . 1.0Hz) |
| 111 | 1.68 (3H, d, J = 6.8Hz), 4.73 (1H, d. J = 17.2Hz), 4.79 (1H, d, J = 17.2Hz), 5.81-5.83 (1H, m), 6. 62 (1H, s), 7.16-7.21 (2H, m), 7.30 (1H , d, J = 8.0Hz), 7.42 (1H, dd, J = 8.0, 2.4Hz), 7.61-7.65 (1H, m), 8.15 (1H, d, J = 2.4Hz), 8.54 (1H, d, J = 4.8Hz) |
| 114 | 3.81 (3H, s), 5.01 (4H, s), 6.41 (1H, s), 7.20-7.23 (4H, m), 7.66 (2H, td, J = 7.7, 1.5Hz), 8.57-8.58 (2H, m) |
| 115 | 5.01 (4H, brs), 6.82 (1H, s). 7. 22-7. 25 (4H, m), 7.67 (2H, td, J = 7. 7, 1.9Hz), 8.57 (2H, d, J = 3.6Hz) |
| 116 | 4.84 (2H, s), 4.99 (2H, s), 6.98 (1H, s), 7.19-7.28 (3H, m), 7.65 (2H, td, J = 7.7, 1.9Hz), 8. 54-8. 57 (3H, m) |
| 134 | 2.29 (3H, s), 5.04 (4H, s), 7.06 (1H, s), 7.18-7.22 (2H, m), 7. 30 (2H, d, J =7.2Hz), 7.61-7.67 (2H, m), 8.56 (2H, d, J = 4.0Hz) |
| 135 | 2.97 (3H, s), 5.07 (4H, brs), 7.18 (1H, s), 7.21-7.24 (2H, m), 7. 30-7 .52 (2H, m), 7.66 (2H, td, J = 7.7, 1.7Hz), 8.56 (2H, d, J = 4.4Hz) |
| 136 | 5.55 (2H, s), 7.17-7.21 (2H, m), 7. 29 (1H, d, J = 6.0Hz), 7. 62-7. 72 ( 2H, m), 7.73 (1H, s), 7.98 (1H, d, J = 8.0Hz), 8.50 (1H, dd, J = 2.4, . 1.2Hz), 8.60 (1H, dd, J = 2.0, 1.2H) |
| 137 | 4.85 (2H, s), 4.89 (2H, s), 6.70 (1H, s), 7. 18-7. 37 (7H, m), 7.63 (1H, td, J = 7.6, 2.0Hz), 8.56 (1H, d, J = 2.0Hz) |
| 138 | 1.31 (3H, t, J = 7.6Hz), 2.97 (2H, q, J = 7.6Hz), 5.41 (2H, s), 7.1 6-7.21 (2H, m), 7.64 (1H, td, J = 7.6, 2.0Hz), 8.37 (1H, s), 8.54 (1H , d, J = 4.8Hz) |
| 139 | 3.42 (3H, s), 4.67 (2H, s), 4.76 (2H, s), 4.93 (2H, s), 4.97 (2H, s), 6.84 (1H, s), 7.14 (1H, d, J = 8.0Hz), 7. 20 (1H, dd, J = 8.0, 4.8Hz), 7.25 (1H, d, J = 7.2Hz), 7.34 (1H, d. J = 8.0Hz), 7.61-7.67 (2H, m) , 8.56 (1H, dd, J = 4. 8. 0.8Hz) |
| 140 | 3.66(1H, t. J = 4.8Hz), 4. 73 (2H, s), 4.96(4H, s), 6.80(1H, s), , 7. 13-7. 25 (4H, m), 7.62-7.67(2H, m), 8.56 (1H, d. J = 4.0Hz) |
| 141 | 1.03(9H, s), 3.59(2H, s), 4.87(2H, s), 6.71(1H, s), 7.09(1H, d, J = 7.6Hz), 7. 17 (1H, dd, J = 8. 8, 6.0Hz), 7. 60 (1H, td, J = 8. 0. 2. 0Hz), 8.55(1H, dd, J = 2.0, 0. 8Hz) |
| 142 | 1.49(6H, s), 4.10(2H, s), 4.11(2H, s), 6.44(1H, s), 6. 95 (1H, d, J = 8.0Hz), 7.07-7. 13(2H, m), 7.28(1H, d. J = 8.0Hz), 7.51-7.57(2H , m), 8.48(1H, dd, J = 4.0, 0.8Hz), 8.57(1H, dd, J = 3.2, 0.8Hz) |
| 145 | 1.62-1.64(1H, m), 1.71-1.80(1H, m), 1.93-1.98(1H, m), 2.20-2.26(1H m), 2.42(3H, s), 2. 60-2. 62(1H, m), 3.06-3.10(1H, m), 3.49(dd, 1H J = 7. 2. 14.8Hz). 3. 85-3.86(1H, m), 4. 85 (1H, d, J = 17.2Hz), 4. 95 (1H, d, J = 17.2Hz), 6.78(1H, s), 7. 14-7.20 (2H, m), 7. 62(dt, 1H , J = 5.2, 8.0Hz), 8.56(1H, d. J = 1.2, 4.8Hz) |

(continued)

| No. | $^1$H-NMR (δ ppm (Solvent : $CDCl_3$ /400MHz) |
|---|---|
| 146 | 3.81(3H, s), 4.89(2H, s), 5.08(2H, s), 7.01(1H, s), 7.11-7.19(3H, m), 7.25(1H, d, J = 8.4Hz), 7.62(1H, dt, J = 1.6, 0.8Hz), 8.09(1 H, dd. J = 4.4, 1.6Hz), 8. 53 (1H, d, J = 4.8Hz) |
| 147 | 2.36(3H, s), 4.96(4H, d, J = 5.6Hz), 6.79(1H, s), 7.10(1H, dd, J = 8.0, 4.8Hz), 7. 17 (1H, m), 7.23 (1H, d, J = 8.0Hz), 7. 45 (1H, d. J = 8.0Hz), 7.61(1H, dt, J = 8.0, 2.0Hz), 8.35(1H, d, 3.6Hz), 8. 54 (1H, dd, J = 4.8,0.8Hz) |
| 148 | 3.28(3H, s). 3.60(2H, t, J = 5.2Hz), 3.83(2H, t, J = 5.2Hz), 4.89 (2H, s), 6.78(1H, s), 7. 21-7. 27(2H, m), 7. 67 (1H, t. J = 2.0Hz). 8 55 (1H, d, J = 0. 8Hz) |
| 149 | 3.00 (1H, dd, J=17.6, 8. 0Hz), 3. 29 (1H, dd. 17.6, 10.8Hz), 3.84-3.8 9(1H, m), 4.07-4.13 (1H, m), 4.87(2H, s), 5.09-5.16 (1H, m), 6.75(1 H, s), 7. 21-7. 27 (2H. m), 7.69(1H, dt, J=8.0, 1.2Hz), 8.54(1H, d, 4.8Hz) |
| 151 | 2.37(3H, s), 4.80(2H, s), 4.86(2H, s), 5. 94 (1H, s), 6. 82 (1H, s), 7.19-7.24(2H, m). 7. 65 (1H, dt. J=7.6, 1.6Hz), 7. 55 (1H, d. J=4Hz) |
| 152 | 3. 88 (3H, s), 4.72(2H, s), 5.08(2H, s), 6.67(1H, s), 6.79(1H, s), 6 96(1H, d, J=7.6Hz), 7.16(1H, 1H, dd, J=5.2, 2, 2Hz), 7.32(1H, s), 7.58(1 H, dd, 1=5.6, 8.0), 8.51(1H, d, J=5.2Hz) |
| 154 | 2.52(3H, s), 4.94(2H, s). 5.02(2H, s), 7.04 (1H, d, J = 8. 0Hz), 7. 05(1H, d, J = 8.0Hz), 7.14(1H, s), 7.20(1H, dd, J = 7.2, 4.8Hz), 7.29(1H, d, J = 7.6Hz), 7.51 (1H, t, J = 7.6Hz), 7.64(1H, td, J = 7.6, 1.2Hz), 8.56(1H, d, J = 4.8Hz) |
| 155 | 2.52(3H, s), 4.89(2H, s), 4.98(2H, s), 6,85(1H, s), 7.01 (1H, d, J = 7.2Hz), 7.05(1H, d, J = 7.6Hz), 7.20(1H, t, J = 6.4Hz), 7.25( 1H, d, J = 6.4Hz), 7.51 (1H, t, J = 7.6Hz), 7. 64 (1H, t, J = 8.0Hz) , 8. 56 (1H, d. J = 4.8Hz) |
| 157 | 3.82(3H, s), 4.90(2H, s), 4.96(2H, s), 6.72(1H, dd, J = 5.2.2.4Hz ), 6.79(1H, s), 6.82(1H, d, J = 2.0Hz), 7.20 (1H, dd, J = 7.6, 5. 2Hz), 7. 64 (1H, td, J = 8.0, 2.0Hz), 7.64(1H, td, J = 8.0, 2.0Hz) 8.37(1H, d, J = 5.6Hz), 8.56(1H, d. J = 5.2Hz) |
| 161 | 2.31(3H, s), 4.91(2H, s), 4.96(2H, s), 6.80(1H, s), 7.01(1H, d. J = 5.2Hz), 7.05(1H, s), 7. 20 (1H, ddd, J = 5.2, 4.8, 0.8Hz), 7. 25 ( 1H, d, J = 8.0Hz), 7.64(1H, td, J = 8.0, 2.0Hz), 8.40(1H, d, J = 5. 2Hz), 8.56(1H, ddd, J = 4.8, 1.6, 0.8Hz) |
| 162 | 2.52(6H, s), 4.91(4H, s), 6.87(1H, s), 7.02(2H, d. J = 8.0Hz), 7. 04 (2H, d, J = 8. 0Hz), 7. 51 (2H, t, J = 8. 0Hz) |
| 164 | 1.69(3H, d, J = 7.6Hz), 4.79(1H, d, J = 17.6Hz), 4.99(1H, d, J = 17.2Hz), 6.14(1H, brs), 6.89(1H, s), 7.05(1H, d, J = 7.6Hz), 7.1 4-7.17(2H, m), 7. 35 (1H, d, J = 8.0Hz), 7. 54-7. 63 (2H, m), 8.54(2H, t, J = 2.4Hz) |
| 165 | 1.68(3H, d, J = 7.2Hz), 4.65(2H, s), 6.26(1H, q, J = 6.8Hz), 6. 88 (1H, s). 6.99(1H, d, J = 7.6Hz), 7.17(1H, dd, J = 7.4, 5.0Hz), 7. 21-7.24(1H, m), 7.58(1H, td, J = 7.6, 1.7Hz), 7.66(1H, d, J = 7.4 Hz), 8.50(1H, dd, J =4.6, 1.8Hz), 8.52-8. 54(1H, m), 8.68(1H, d, J = 2.4Hz) |
| 166 | 1.24(3H.t, J = 7.2Hz), 3.71(2H, q, J = 7.2Hz), 4.84(2H, s), 6. 92 (2H, s), 7.20(1H, dd, J = 7.0, 4.8Hz), 7. 25 (1H, d, J = 7.6Hz), 7. 64(1H, td, J = 7.6, 1.6Hz), 8.56(1H, d, J = 4.4Hz) |
| 167 | 5.00(4H, s), 6.83(1H, s), 6.98(1H, t, J = 53.4Hz), 7.19-7.26(4H, m), 7.65(2H, td, J = 7. 6. 1.6Hz), 8.56(2H, d, J = 4. 8Hz) |
| 168 | 5.02(4H, 5), 6.84(1H, t, J = 14.4Hz), 6.99(1H, s), 7.17-7.21 (2H, m), 7.29(2H, d, J = 7.2Hz), 7.61-7.66(2H, m), 8.55-8.56 (2H, m) |
| 170 | 2.36(3H, s), 5.01(4H, s), 6.95 (1H, s), 7.16-7.21(2H, m), 7.28(2H, d, J = 8.0Hz), 7. 60-7. 65 (2H, m), 8. 54-8. 56 (2H, m), 1,86(3H, t, J = 19.2Hz), 4.98(4H, s), 6.99(1H, s), 7.18-7.23(4H, m), 7.63(2H, td, J = 7.7, 1.6Hz), 8.57(2H, d, J = 4.8Hz) |
| 171 | 2.36(3H, s), 5.01(4H, s), 6.95(1H, s), 7.16-7.21(2H, m), 7.28(2H, d, J = 8.0Hz), 7. 60-7.65(2H, m), 8. 54-8. 56 (2H. m) |
| 173 | 3.30(3H, s), 3.63(2H, t, J=5.6Hz), 3. 87 (2H, t, J=4.8Hz), 4.89(2H, s), 7.06(1H, s), 7.17-7.27(2H, m), 7. 63 (1H, dt, 7.2, 2.0Hz), 8.5 3(1H, d, J=4. 8Hz) |
| 188 | 3.48(2H, d, J = 6.4Hz), 4.66(2H, d, J = 4. 8Hz), 4. 99-5. 08(2H, m), 5.86(1H, m), 6.11(1H, br. s), 6.69(1H, s), 7.20-7.24(1H, m), 7.32 (1H, d, J = 8.0Hz). 7. 68 (1H, td, J = 8.0, 2.0Hz), 8.57(1H, d, J = 4.0Hz) |

(continued)

| No. | $^1$H-NMR (δ ppm (Solvent : $CDCl_3$ /400MHz) |
|---|---|
| 190 | 1.83(3H, s), 3.39(2H, s), 4.22(1H, s), 4.67(2H, s), 4.78(1H, s), 6.13(1H, br, s), 6.71(1H, s). 7.21 (1H, m), 7.32 (1H, d, J = 8.0Hz), 7.69(1H, t, J = 7.6Hz), 8.57(1H, d, J =5.2Hz) |
| 192 | 5.00(4H, s), 6.85(1H, s), 6.99(1H, t, J = 13.2Hz), 7.21(2H, dd, J = 7.0, 5. 8Hz), 7. 26 (2H, d, J = 2.4Hz), 7. 65 (2H, td. J = 7.7, 1.9H z), 8.56(2H, d, J = 4.4Hz) |
| 193 | 5.02(4H, s), 6.84(1H, s), 7.23(2H, dd, J = 7.2, 4.8Hz), 7.25-7.30 (2H, m), 7.67(2H, td. J = 7.6, 1.7Hz), 8.57 (2H, d, J = 5.2Hz) |
| 194 | 4.99(4H, s), 5.35(1H, brs), 6.85(1H, s), 7.21 (2H, dd, J = 7.6, 4. 8Hz). 7. 25-7. 30 (2H, m), 7.66(2H, td, J = 7.6, 1.7Hz), 8.56(2H, d, J = 4.0Hz) |
| 195 | 3.90(3H, s), 5.00(4H, s), 6.71(1H, s), 7.16-7.26 (4H. m), 7.62-7.6 9(2H, m), 8.52-8.56 (2H, m) |
| 196 | 3.48(2H, d, J = 6.0Hz), 4.96(2H, s), 4.98(2H, s), 5.00-5.06(2H, m ), 5.86(1H, m), 6.71(1H, s), 7.17-7.20(2H, m), 7.25-7.27(2H, m). 1.63(2H, m), 8.55(2H, d, J = 5.2Hz) |
| 197 | 1.82(3H, s), 3.38(2H, s), 4.22(1H, s), 4.78(1H, s), 4.97(2H, s), 4.98(2H, s), 6.73(1H, s), 7.19(2H, t, J = 6.0Hz), 7.25-7.28(2H, m) , 7.63(2H, td, J = 8.0. 2.0Hz), 8.56(2H, d, J =4. 8Hz) |
| 198 | 3.74(3H, s), 4.87(4H, s), 6.73(1H, s), 7.11-7.18(4H, m), 7.64(dt, 2H, J = 4.4Hz, J = 8.0Hz), 8.08(1H, s), 8. 53 (2H. d. J = 4.4Hz) |
| 200 | 1.25(3H, s), 3.23(3H, s), 3. 39-3. 54 (3H, m), 4.73(2H, s), 6.61(1H, s), 7.16-7.27(2H, m), 7.62(1H, dt, J=8, 2Hz), 8. 57 (1H, d, J=4.8H z) |
| 201 | 1.23(3H, s, J = 6.4Hz), 3.29(3H, s), 3. 64 (1H, dd. J = 7.6, 14. 8Hz ), 3.71-3.74(1H, m), 4. 07-4. 11 (1H, m); 4. 57 (1H, d, J = 6.8Hz), 4. 65(1H, d, J = 6.8Hz), 4.85(1H, d, J = 16.8Hz), 4.97(1H, d, J = 16 8Hz), 6.81(1H, s), 7.18-7.20(2H, m), 7.59(1H, dt, J = 5.2, 8.0Hz ), 8.56(1H, dd, J = 2.0, 5.6Hz) |
| 204 | 2.18(3H, s), 4.89(2H, s), 4.90(2H, s), 6.83(1H, s), 7.20-7.23 (1H. m), 7.26-7.27(1H, m), 7. 66(1H, dt, J = 4, 0Hz, J = 8.0Hz), 8.57(1 H, d, J = 4.0Hz) |
| 205 | 1.30(3H, t, J = 8.4Hz), 2.63(2H, q, J = 8.4Hz), 2.77(2H, t, J = 7 6Hz), 3.82-3. 86(2H, m), 4.81(2H, s), 6.69(1H, s), 7.20-7.23 (2H, m), 7.65(1H, dt, J = 4.8Hz, J = 8.0Hz), 8.57(1H, d, J = 4.8Hz) |
| 206 | 1.37(3H, t, J = 8. 4Hz), 2.77-2.89 (2H, m), 2.94-3.01(1H, m), 3. 19-3.26(1H, m), 4.03-4.15(1H, m), 4.19-4.26 (1H, m). 4. 85 (2H, s), 6.8 5(1H, s), 7.20-7.26(2H, m), 7.67(1H, dt, J = 4.8Hz, J = 8.0Hz), 8 , 55(1H, d, J = 4.8Hz) |
| 208 | 2.52(3H, s), 4.67(4H, s), 7.17(2H, dd, J = 6.8, 4.4Hz), 7.45(2H, d, J = 8.0Hz), 7.65(2H, td, J = 7.7, 1.7Hz), 8.55(2H, d, J = 4. 4Hz ) |
| 210 | 3.42(6H, s), 3.77(2H, d, J = 4.8Hz), 4.57(1H, t, J = 4.8Hz), 4. 86 (2H, s), 6.78(1H, s), 7. 18-7.21 (2H, m), 7. 64(1H, dt, J = 2.0Hz, J = 9.2Hz), 8.56(1H, d, J = 6.0Hz) |
| 211 | 1.42(3H, s), 1.46(3H, s), 4.06-4.13(2H, m), 4.15-4.21 (2H, m), 4.6 0-4.68(1H, m), 5.11(2H, s), 6.66(1H, s). 7.18-7.30(2H, m), 7.65(1 H, dt, J = 4.8Hz, J = 8.0Hz), 8.57(1H, d. J = 4.8Hz) |
| 213 | 3.30(6H, s), 3. 79-3. 87 (4H, m), 4.51(2H, d. J = 6.8Hz), 4. 56 (2H, d J = 6.8Hz), 4.85(2H, s), 5.07(1H, bs), 6.66(1H, s), 7.16-7.19(1 H, m), 7.29(1H, d, J = 8.4Hz), 7.61(1H, dt, J = 4.8Hz, J = 8.4Hz) 8.56(1H, d, J = 4. 8Hz) |
| 214 | 1.84-1.87(1H, m), 3.33(1H, s), 3.40(1H, t, J = 6.4Hz), 3.49(1H, t, J = 6.0Hz), 6.58(1H, s), 7. 18-7. 30 (2H, m), 7.60(1H, dt, 7.2, 2.0Hz) 8.55(1H, d, J = 4.8Hz) |
| 217 | 1.32(3H, t, J = 7.8Hz), 2. 61-2. 71 (2H, m), 3. 35 (3H, s), 3. 73-3. 81( 1H, m), 4. 15-4.20(1H, m), 4. 67-4.70 (1H, m), 4.88(2H, s), 6.78(1H, s), 7.18-7.21 (2H, m), 7. 64 (1H, d t, J = 4. 0Hz. J = 8.0Hz), 8. 57 (1 H, d, J = 4. 0Hz) |
| 218 | 1.20-1.33(6H, m), 2.68-2.79 (4H, m), 4.00(2H, d, J = 7.2Hz), 4. 29 ( 1H, t, J = 6.8Hz). 4.89(2H, s), 6.71(1H, s), 7.18-7.22(2H, m), 7. 64(1H, dt, J = 4.4Hz, J = 8.0Hz), 8. 57(1H, d, J = 4.4Hz) |
| 219 | 1.26(6H, t, J = 7.8Hz), 2. 65-2. 78 (4H, m), 4.05(2H, d, J = 7.6Hz), 4.35(1H, t, J = 7.2Hz), 4.90(2H, s), 6.98 (1H, s), 7. 20-7. 22 (2H, m), 7.65(1H, dt, J = 5.2Hz, J = 8.0Hz), 8.57(1H, d. J = 5.2Hz) |
| 220 | 1.41(6H, t, J = 7. 8Hz), 3. 18-3, 21 (4H, m), 4. 30 (2H, d, J = 6.0Hz), 4.44(1H, t, J = 6. 4Hz), 4. 84 (2H, s), 6.85 (1H, s), 7. 20-7.25 (2H, m), 7.68(1H, dt, J = 4.8Hz, J = 8.0Hz), 8.54(1H, d, J = 4. 8Hz) |

(continued)

| No. | $^{1}$H-NMR (δ ppm (Solvent : $CDCl_3$ /400MHz) |
|---|---|
| 221 | 4.27(2H, d, J = 6.0Hz), 4.80(2H, s), 6.00(1H, t, J = 6.8Hz), 6. 73 (1H, s), 7.23-7.27(2H, m), 7.69(1H, dt, J = 4.0Hz, J = 8.0Hz), 8. 56(1H, d. J = 4.0Hz) |
| 225 | 1. 55 (3H, d, J = 7. 6 H z), 3.70 (3H, s), 4.72(2H, d. J = 6. 8Hz), 5.20 ( 1H, dd. J = 7. 2Hz. J = 14. 8H z), 6. 64 (1H, s), 7. 19-7.23 (1H, m), 7.3 2(1H. d, J= 7.6Hz). 7.65(1H, dt, J = 4.8Hz. J= 8.0Hz), 8.59(1H, d, J = 4. 8Hz) |
| 226 | 0.87(3H, d, J = 6.0Hz), 1. 06 (3H, d, J = 6.0Hz), 2.41-2.50 (1H, m), 3. 61 (3H, s), 4. 71 (1H, d. J = 17. 6Hz), 4. 92 (1H, d, J = 17. 6Hz), 5.1 0 (1H, d, J = 10.4Hz), 6. 67 (1H, s), 7.15-7.19 (2H, m), 7.61(1H, dt, J = 5. 2Hz, J = 8.0Hz), 8.55(1H. d. J = 5. 2Hz) |
| 227 | 3. 80 (3H, s), 5. 06 (2H, s), 5. 77 (1H, s), 6. 21 (1H, s), 6. 71 (1H, s). 7 18-7. 22 (1H, m), 7. 41 (1H, d. J = 8. 0Hz). 7. 66 (1H, dt, J = 4. 8Hz, J = 8. 0Hz), 8. 56 (1H, d, J = 4. 8Hz) |
| 228 | 3.38(3H, s), 3. 60-3. 62 (2H, m), 4. 31-4. 32 (2H, m), 4. 44 (2H, s), 4. 82 (2H, s). 6. 74 (1H, s). 7. 21-7. 24 (1H, m). 7.34(1H, d. J = 8.0Hz), 7. 67 (1H, dt, J = 4. 8Hz, J = 8.0Hz), 8. 58 (1H. d, J = 4. 8Hz) |
| 229 | 3. 07 (1H, bs), 3.83-3.86(2H, m), 4. 29-4. 32 (2H, m), 4. 41 (2H, s), 4.8 3(2H, s). 6.78(1H, s), 7.23-7.28(1H, m). 7.48(1H, d, J = 8.0Hz), 7 . 69 (1H, dt, J = 4. 4Hz. J = 8.0Hz), 8. 56 (1H, d. J = 4. 4Hz) |
| 233 | 3. 24 (3H, s), 3.42-3.45 (2H, m), 3.47-3.51 (2H, m), 4. 25 (2H, s), 4. 86 (2H, s), 6.63(1H. s). 7.21-7.25(1H, m), 7.38(1H, d). 7.74(1H, dt, J 4.8Hz, J= 7. 6Hz). 8. 48 (1H, d, J = 4. 8Hz), 9.67(1H, bs) |
| 235 | 3. 59-3. 65 (4H, m), 3. 71-3.77 (4H, m), 4. 96 (2H, s), 4.85(2H. s), 6. 77 (1H, s), 7.21-7.23(1H, m), 7.36 (1H, d, J= 7.6Hz), 7.68(1H, dt, J = 4.0Hz. J = 8.0Hz), 8. 58 (1H, d, J = 4.0Hz) |
| 236 | 4.50(2H, s), 4. 79 (2H. s), 6. 76 (1H, s), 7. 22-7.25 (1H, m), 7. 31 (1H, d, J = 8. 0 Hz) , 7.71(1H, dt, J = 4.4Hz, J = 8.0Hz), 8.56(1H, d. J = 4.4Hz). 9. 71 (1H, s) |
| 240 | 2.73(2H, dd, J = 6. 8Hz, J = 13. 6Hz), 3. 65 (3H, s), 3. 97 (2H, t, J = 6. 8Hz), 4. 83 (2H, s), 6.72 (1H, s), 7.19-7.27 (2H, m), 7.65 (1H, dt, J = 5. 2Hz. J =8.0Hz). 8. 56 (1H, dd. J =0. 8Hz, J = 4. 8Hz) |
| 242 | 3. 29 (3H, s), 4. 00-4, 15 (2H, m), 4. 60 (1H, d, J = 6. 8Hz), 4. 69 (1H, d. J = 6. 8Hz) , 4. 81 (1H, d. J = 16. 8Hz), 4. 87 (1H, d, J = 16. 8Hz), 5.1 3(1H, dd, J = 4. 8Hz, J = 6. 8Hz), 6.85(1H, s), 7.16 (1H, dd. J = 5.6 Hz, J=6.4Hz), 7. 19-7. 23 (2H, m), 7. 41 (1H, d, J = 8.0Hz), 7. 60 (1H, dt, J = 4. 8Hz, J = 8.0Hz), 7.68 (1H, dt, J = 5.2Hz, J = 8.0Hz), 8. 53 (1H, d, J = 4.8Hz), 8. 61 (1H, d, J = 5. 2Hz) |
| 248 | 1.48-1.71 (6H, m), 3. 47-3. 50 (1H, m), 3. 65-3.69 (111, m), 3. 79-3. 88 (3H , m), 3. 95-4. 00 (1H, m), 4. 57 (1H, bs), 4. 91 (2H, s), 6. 90 (1H, s), 7. 18-7. 25 (2H, m), 7. 63 (1H, dt. J = 4. 4Hz, J = 8. 0Hz), 8. 55 (1H, d, J = 4. 4Hz) |
| 249 | 3. 82-3. 85 (2H, m), 3. 92-3. 94 (2H, m), 4. 61 (2H, s), 4. 91(2H, s), 6. 8 4 (1H, s), 7. 16-7. 30 (4H, m), 7. 59-7. 72 (2H, m), 8.52-8. 58 (2H, m) |
| 254 | 4. 82 (2H, s), 4. 92 (2H, s), 6. 08 (1H, d, J=3.2Hz), 6. 31 (1H, d, J=3.2 Hz), 6. 68 (1H, s), 6. 83 (1H, s), 7. 21-7. 29 (2H, m), 7. 67 (1H, dt, J=8 , 2Hz), 8.59(1H, d, J=4.8Hz) |
| 255 | 1. 53-1. 62 (2H, m), 1. 89-1. 97 (2H, m), 3. 58-3. 64 (1H, m), 3. 73-3. 78 (1 H, m), 3. 85-3. 90 (2H, m), 4. 21-4. 25 (1H, m), 4. 92 (2H, s), 6.79(1H, s), 7.17-7. 23 (2H, m). 7. 63 (111, dt, J = 4.0Hz, J = 8.0Hz). 8. 56 (1H , d, J = 4.0Hz) |
| 256 | 3. 4-3. 85 (2H, m), 3. 88-3. 94 (2H, m), 3. 97-3. 98 (2H, m), 4. 94 (2H, s) , 5. 17 (1H, t, J = 4.0Hz). 6. 85 (1H, s), 7.17-7.25 (2H, m), 7. 63 (1H, dt, J = 4. 0Hz, J = 9. 2Hz), 8. 56 (1H, d, J 4.0Hz) |
| 257 | 1.65- 1. 70 (1H, m), 2. 00-2. 04 (1H, m), 2. 72-2. 75 (1H, m), 3.56-3.65 (2 H, m), 3. 72-3. 81 (3H, m), 3. 92-3. 98 (1H, m), 4.82(2H, s), 6. 70 (1H, s), 7. 15-7. 22 (2H, m), 7. 64 (1H, d t, J = 4. 4Hz, J = 8. 0Hz), 8. 56 (1H , d, J = 4.4Hz) |
| 258 | 1.28-1.35(1H, m). 1.48-1.65(4H, m), 1.85-1.88(1H, m), 3.29-3.34(1 II, m), 3. 55-3.64(2H, m), 3. 74-3. 77 (1H, m), 3. 90-3. 93 (1H, m), 4. 95 (2H, s), 6. 79 (1H, s), 7.16-7. 21 (2H, m), 7. 62(1H, d t, J = 4.0Hz, J = 8.0Hz), 8.55(1H, d, J = 4.0Hz) |
| 260 | 3. 05-3. 07 (2H, m), 3.71-3.76 (2H, m), 3. 88-3. 98 (2H, m), 4. 29-4.33 (1 H. m), 4. 91 (2H, s), 6. 77 (1H, s), 7. 19-7. 25 (2H, m), 7. 63 (1H, dt, J = 4. 4Hz, J = 8.0Hz), 8. 57 (1H, d, J = 4.4Hz) |

(continued)

| No. | $^1$H-NMR (δ ppm (Solvent : $CDCl_3$ /400MHz) |
|---|---|
| 261 | 2. 84-2. 89 (1H, m), 3.06-3.12 (1H, m), 3.31-3. 37 (1H, m), 4.10-4.16(2 H, m), 4.38-4.42(III, m). 4.59-4.62(1H, m), 4.86(2H, s), 6. 78 (1H, s), 7. 20-7. 25 (2H, m), 7.67(1H, dt, J = 4. 4Hz, J = 8.0Hz), 8. 56 (1H d, J = 4.0Hz) |
| 262 | 1. 89 (3H, s), 2. 92-2. 95 (2H, m), 4. 20-4. 22 (2H, m), 4.39(2H, s), 4-8 3(2H, s), 6.78(1H, s), 7.1 7-7. 24 (1H, m), 7.43-7.47(1H, m). 7.63(1 H. dt, J = 4.0Hz, J = 8.0Hz), 8.56 (1H, d, J=4.0Hz) |
| 263 | 1.99-2.05(2H, m), 2. 75-2. 82 (2H, m), 2. 95-3. 01 (2H, m), 4. 10 (2H, d, J=7.2Hz). 4.39 (1H, t, J = 7. 2Hz). 4. 86 (2H, s), 6. 72 (1H, s), 7. 19-7.22 (2H, m), 7. 65 (1H, dt, J=4.4Hz, J = 8.0Hz). 8. 57 (1H, d, J = 4.4Hz) |
| 264 | 3.56(3H, s), 4.71 (2H, s), 4.95(2H, s), 6. 04 (1H, dd, J = 2. 8Hz, J = 3. 2Hz). 6. 09 (1H, s), 6. 60 (1H, d, J=2.0Hz), 6. 67 (1H, s), 6. 75 ( 1H, s), 6. 90 (1H, d, J = 7.6Hz), 7.16 (1H, dd, J = 5.2Hz. J = 7. 2Hz ), 7. 58 (1H, dt, J= 4. 8Hz, J = 8.0Hz), 8. 54 (1H, d, J = 4. 8Hz) |
| 265 | 3.46(3H, s). 4. 68 (2H, s), 9. 94 (2H, s). 5. 97 (1H, d, J = 3. 2Hz), 6. 04 (1H, d, J = 3.2Hz). 6. 78 (1H, s), 6. 83 (1H, s) . 6. 91 (1H, d, J = 7 2Hz), 7.17 (III. dd, J = 5. 2Hz, J = 7. 6Hz), 7. 58 (1 H, dt, J = 5. 2Hz , J = 8.0Hz), 8. 54 (1H, d, J = 4. 8Hz) |
| 267 | 4.61(2H, s), 4. 90 (2H, s), 6. 29(1H, s), 6. 59 (1H, s), 6. 91 (1H, d, J = 1. 6Hz), 7. 08 (1H, d. J= 7. 6Hz), 7.16-7.21 (2H, m), 7. 60 (1H, dt, J = 4. 8Hz, J = 8.0Hz), 7.76 (1H, d, J = 8. 0Hz), 8.30(1H, d, J = 5 .2Hz), 8. 50 (1H, d, J = 4. 8Hz) |
| 268 | 2. 13 (3H, s). 3. 80 (3H, s), 4. 71 (2H, s), 5.03(2H, s), 6. 64 (1H, s), 6 77 (1H, s), 6. 96 (1H, d, J=8.0Hz), 7.14 (1H, dd, J=6.8. 5. 6Hz), 7.5 8 (1H, dd, J=7.2, 6.8Hz), 6. 50 (1H. d, J=4. 4Hz) |
| 269 | 3. 06(1H, dd, J = 8.0Hz, J = 16. 8Hz), 3. 44-3. 52 (1H, m), 3. 95 (1H, d d, J = 8.0Hz. J = 15. 2Hz). 4. 08-4. 14 (1H. m), 4. 96 (2H, s), 5. 23-5. 29 (1H, m), 6. 83 (1H, s), 7. 19-7. 22 (1H, m), 7. 28-7. 32 (2H, m), 7. 36-7.38 (2H, m), 7. 66 (1H, dt, J= 4. 4Hz, J = 8.0Hz), 8. 56 (1H, d, J = 4. 4Hz) |
| 270 | 1. 19-1. 33 (2H, m), 1. 35- 1. 39 (1H, m). 1.56-1.61 (2H, m), 1.74-1. 77(2 H, m), 2.13-2.20 (1H, m), 2. 40 (3H, s), 2. 85-2. 88 (1H, m), 3. 47-3. 52 (1H, m), 4.09-4.16(1H, m), 4.77(1H, d, J = 16.8Hz), 4.93(1H, d, J = 16.8Hz), 6. 75 (1H, s), 7.15-7.20(2H, m), 7.63 (1H, dt, J = 5.2Hz J = 8. 0Hz), 8. 55 (1H, d, J = 5. 2Hz) |
| 271 | 5. 29 (2H, s), 7.09(1H, s), 7. 24-7. 26 (1H, m), 7. 45 (1H, d, J = 8. 0Hz ), 7. 72 (1H, dt, J = 4. 8Hz, J = 8.0Hz), 8. 32 (1H, s), 8. 54 (1H, d, J = 4. 8Hz) |
| 272 | 7. 16 (1H, dd, J = 4. 8Hz, J = 7.2Hz), 7. 30-7.36 (3H, m), 7.42-7.450 H, m), 7. 50-7. 54 (3H, m), 7. 64 (1H, dt, J = 4. 8Hz, J = 8. 0Hz), 8. 49 (1H, d, J = 4.8Hz) |
| 273 | 5.35(2H, s), 6. 86-6. 90 (2H, m), 7. 00-7. 05 (1H, m), 7.15-7.18 (1H, m) 7. 31-7.38 (1H, m), 7. 51-7.57 (1H, m), 7. 64 (1H, dt, t, J = 4.0Hz, J = 8.0Hz), 8. 97 (1H, d, J = 4. 0Hz) |
| 279 | 1. 67 (3H, d, J = 6. 8Hz), 4. 77 (1H, d, J = 17. 2Hz), 4.82(1H, d, J = 17. 2Hz), 5. 97(1H, d, J = 6. 8Hz), 6. 57 (1H, s), 7.16-7.20 (2H, m), 7 .31(1H. d, J = 8.0Hz), 7.43 (1H, d, J = 7. 6Hz), 7. 62 (1H, dt, J = 4 . 4Hz, J = 8. 0Hz), 8. 39 (1H, s), 8. 47 (1H, d, J = 4.4Hz), 8. 55 (1H, d , J = 4.0z) |
| 280 | 1. 69 (3H, t, J = 6. 8Hz) , 4. 77-4. 97 (2H, m) , 6. 67 (1H, brs), 6.95-7.0 0 (2H, m), 7. 15-7. 20 (2H, m), 7. 34 (1H, brs), 7. 57 (1H, d, J = 7. 8Hz) , 7. 62 (1H, td, J= 7. 6, 1. 7Hz), 8. 51 (1H, d, J = 4.8Hz), 8. 54 (1H, d, J=4.4Hz) |
| 283 | 1. 65 (3H, d. J= 7. 2Hz) , 2. 96 (3H, s), 6. 07 (1H, q, J = 6. 9Hz), 6. 93 (1H, s), 7. 18 (1H, dd, J = 7. 6, 4. 8Hz), 7. 28 (1H, d, J = 8.0Hz), 7. 63 (1H, td, J = 7. 8, 2.0Hz),8.58(1H, dt, J =4.0, 0.9Hz) |
| 287 | 1. 71 (3H, t, J = 6. 8Hz). 4. 72 (1H, d, J = 17. 8Hz), 5. 05 (1H, d, J = 17.8Hz). 6. 26(1H, q, J = 7.2Hz), 6. 88 (1H, s), 7.10(1H, d, J = 8.0 Hz), 7.18 (1H, dd, J = 6. 8, 4.8Hz), 7.61 (1H, td, J = 8. 0, 1. 9Hz). 8. 44 (1H, d, J = 2. 4Hz), 8. 48 (1H, t, J =2. 2Hz), 8. 54-8. 69 (1H, m). 8. 69 (1H, s) |
| 288 | 1. 69 (3H, t, J = 7. 2Hz) 4. 70 (1H, d, J = 17. 8Hz), 4. 93 (1H, d, J = 17. 8Hz), 6. 23 (1H, q, J = 7. 2Hz), 6. 60 (1H, s), 7.06 (1H, d, J = 8.0 Hz) , 7. 17 (1H, dd, J = 6. 8, 4. 8Hz), 7. 59 (1H, td, J=7. 8. 1. 6Hz). 8.44 (1H, d, J = 3.2Hz), 8. 48 (1H, t, J = 2. 0Hz), 8. 52 (1H, d, J=4. 0Hz), 8. 70 (1H, d, J = 1.6Hz) |
| 289 | 4. 98 (1H, brs), 5. 98 (1H. brs), 6. 13 (1H, brs), 7. 16 (1H, dd, J = 7. 4 . 5.0Hz), 7. 49-7. 53 (2H. m), 7. 57-7. 66 (2H, m), 7. 74 (1H, dd, J = 7. 2, 0. 8Hz), 7. 92 (1H, dd, J= 8. 4, 0. 8Hz), 8. 26 (1H, dd, J = 8. 4, 2. 0Hz), 8. 48 (1H, d, J = 4. 4Hz), 8. 89 (1H, dd, J = 4. 2, 1. 8Hz) |

(continued)

| No. | $^{1}$H-NMR (δ ppm (Solvent : $CDCl_3$ /400MHz) |
|---|---|
| 291 | 4. 3 (2H, s), 4. 95 (2H, s), 7. 19-7. 38 (4H, m), 7. 26 (1H, s). 7. 65 (1H, td, J = 8. 0, 2. 0Hz), 8. 40 (1H, d, J = 2.4Hz). 8. 56 (1H, m) |
| 292 | 4. 99 (4H, s), 7. 07 (1H, s), 7. 19-7. 36 (4H, m), 7. 65 (1H, td. J = 7. 6. 2. 0Hz), 8. 40 (1H, d, J = 2. 0Hz), 8. 56 (1H, d. J = 4. 4Hz) |
| 293 | 2. 51 (3H, s), 4. 75 - 4. 91 (2H, m), 5. 15 (2H, br. s), 6. 95 (1H, br. s), 7. 08 (1H, d, J = 8.0Hz), 7. 20-7. 33 (3H, m), 7. 54 (1H, t, J = 8.0Hz), 7 . 67 (1H, td, J = 8. 0, 1.6Hz), 8. 56 (1H, br. s) |
| 294 | 3. 79(3H, s), 3. 83 (3H, s), 4. 78 (2H, s), 5. 01 (2H, s), 6. 55 (1H, s), 6. 62 (1H, d, J = 8.0Hz), 6.78(1H, d, J = 7. 2Hz) , 7. 17-7.22 (2H, m), 7. 48-7. 53 (1H, m), 7. 61 (1H, dt, J = 5. 2Hz, J = 9. 2Hz). 8. 57 (1H, d , J = 5. 2Hz) |
| 295 | 3.83(3H, s), 4. 77 (2H, s), 5. 01 (2H, s), 6. 63 (1H, d. J = 8. 4Hz), 6. 80 (1H, d, J =6. 8Hz), 7.00 (1H, s), 7. 18-7. 25 (2H, m), 7. 51 (1H, d, J = 8.0Hz), 7. 63 (1H, dt, J = 4.8Hz, J = 8.0Hz), 8.57 (1H, d. J = 4.8Hz) |
| 296 | 3.86 (6H, s), 4.83 (4H, s), 6.63 (2H, d, J = 7.6Hz), 6.81 (2H, d, J = 7. 2Hz), 7. 04(1H, s), 7. 49-7.54 (2H, m) |
| 297 | 4.93 (2H, s), 4.94 (2H, s), 6.79 (1H, s), 7.19-7.36 (3H, m), 7.39 (1H, d, J = 7.2Hz, 7.50 (1H, d, J = 7.2Hz), 7.66 (1H, dt, J = 5.2Hz, J = 8.0Hz), 8.55 (1H, d. J = 4.8Hz) |
| 298 | 4.91 (2H, s), 4.95 (2H, s), 6.77 (1H, s), 6. 84 (1H, dd, J = 8.4. 2.4H z), 7.14-7. 32 (3H, m), 7. 63-7. 77 (2H, m), 8.56 (1H, m) |
| 299 | 4.95 (2H, s), 5.00 (2H, s), 6.84 (1H, dd, J = 8.4, 2. 4Hz), 7. 05 (1H, s). 7. 19-7. 33 (3H, m), 7. 64-7. 77 (2H. m), 8.56 (1H, m) |
| 301 | 5.05 (2H, s), 5. 10 (2H, s), 7.03 (1H, s), 7. 17-7.20 (2H, m), 7.37-7.4 8 (4H, m), 7.57-7.67 (3H, m), 7. 94-7. 99 (2H, m), 8. 58 (1H, d, J = 4. 8 hz) |
| 302 | 4.96 (2H, s), 4.98 (2H, s), 6.26 (1H, t, J = 6.4Hz), 6.63 (1H, d, J = 9.2Hz), 6. 89 (1H, s), 7. 22-7.31 (3H, m), 7.36-7.41 (1H, m), 7.64 (1H t, J = 7. 2Hz). 7.74-7.81 (2H, m), 7.89 (1H, d, J = 8. 0Hz), 8.58 (1 H, d, J = 4.8Hz) |
| 304 | 4.00 (3H, s), 4.95 (2H, s), 5.08 (2H, s), 6. 85 (1H, s), 7. 19-7.22 (1H, m), 7.26-7.29 (1H, m), 7.44 (1H, d, J= 8.0Hz), 7.64 (1H, dt, J = 4 . 4Hz, J = 8.0Hz), 7.80 (1H, t, J = 7.6Hz) , 8.04 (1H, d, J = 8.0Hz), 8.55 (1H, d, J = 4.4Hz) |
| 305 | 5.04 (2H, s), 5.06 (2H, s), 6. 79 (1H, s), 7. 27-7. 35 (2H. m), 7. 56 (1H, d, J = 7.6Hz), 7.73 (1H, dt, J = 3.6Hz, J = 8.0Hz), 7.89 (1H, t, J = 8.0Hz), 8.10 (1H, d, J = 7.6Hz), 8.41 (1H, bs), 8.62 (1H, d. J = 3.6Hz) |
| 306 | 4.93 (2H, s), 5.01 (2H, s), 6.81 (1H, s), 7.21-7.24 (1H, m), 7.27-7.3 0 (1H, m), 7.56-7.61,(2H, m), 7.65-7.70 (1H, m), 7.79 (1H, t, J = 7.6 Hz), 8.55 (1H, d, J = 3.6Hz) |
| 308 | 2.54 (3H, s), 4.81 (2H, s), 4. 88 (2H. s), 7.09 (1H, dd, J = 8.0, 4.8H z), 7.21 (1H, d, J = 7.6Hz), 7.22 (1H, d, J = 8.0Hz), 7.26 (1H, s), 7.36 (1H, d, J = 7.6Hz), 1.65 (1H, td, J = 8.0, 2.0Hz), 8.42 (1H, d d, J = 4.8, I. 6Hz), 8.55 (1H, dd, J = 5. 2, 2. 4Hz) |
| 310 | 2.55 (3H, s), 4.83 (2H, s), 4.96 (2H, s), 6. 91 (1H, s), 7. 09 (1H, dd, J = 8.0, 4.8Hz), 7.20-7.24 (2H. m), 7.36 (1H, d, J = 8.0Hz). 7. 65 ( 1H, td, J = 7.6, 1.6Hz), 8.43 (1H. dd, J = 5.2, 1.6Hz), 8.55 (1H, m ) |
| 311 | 2.53 (3H, s), 4.83 (2H, s), 4.87(2H. s). 6.71 (1H, s), 7.12 (1H, d, J = 8.0Hz), 7. 17-7. 23 (2H, m), 7.51 (1H, dd, J = 8.0, 2.0Hz), 7.65 (1 H, td, J = 7.6, 1.2Hz), 8.42 (1H, d, J = 2.4Hz), 8.57 (1H, d, J = 4 0Hz) |
| 312 | 2.54 (3H, s), 4.83(2H, s), 4.94 (2H, s), 6.96 (1H, s), 7.12 (1H, d. J = 8.4Hz), 7.19-7.24 (2H, m), 7.53 (1H. dd, J=7.6, 2.4Hz), 7.65 (1 H, td, J = 8.0, 1.6Hz), 8.44 (1H, d, J = 2.0Hz), 8.57 (1H, d, J = 4. 8Hz) |
| 313 | 2.54 (3H, s), 4.79 (2H, s), 4.88 (2H, s), 6.69 (1H, s), 6.96 (1H, d, J = 4.8Hz), 7.00 (1H, s), 7.21 (1H, d, J =7.6Hz), 7.23 (1H, d, J = 7 .6Hz), 7.66 (1H, td, J = 8.0, 2.0Hz), 8.43 (1H, d, J = 5.6Hz), 8. 56 (1H, td, J = 4.8, 1.2Hz) |
| 318 | 4.84 (2H, s), 4.99 (2H, s). 6.97 (1H, s), 7.19-7. 28 (3H, m), 7.63-7.6 8 (2H, m), 8.54-8.58 (3H, m) |
| 320 | 1.70 (3H, brs), 4.77-5.11 (2H, m), 6.46 (1H, brs), 6.95 (2H, brs), 7. 17-7.20 (2H, m), 7.35 (1H, s), 7. 57-7. 70 (2H, m), 8.51-8.55 (2H, m) |
| 321 | 3.14 (6H, s), 4.97 (4H, brs), 6.32 (1H, s), 7.18-7.22 (4H, m), 7.65 (2 H, td, J = 7.8, 1.9Hz), 8. 56 (2H, d, J = 4. 4Hz) |
| 324 | (solvent : DMSO-$d_6$) 1.83 (4H, br, s), 3.40-3.50 (4H, m), 4.91 (2H, b r.s), 4.99 (2H, br. s), 6.66 (1H, br. s), 7.03 (1H, s), 7.20-7. 32 (2H, m ), 7.34 (1H, d, 1=7.6Hz) 7. 76 (1H, t, 8. 0Hz), 8. 02 (1H, br. s), 8. 50 (1H, d, J = 4.4Hz) |

(continued)

| No. | $^{1}$H-NMR (δ ppm (Solvent : $CDCl_3$ /400MHz) |
|---|---|
| 329 | 1.68 (3H, d, J= 6.8Hz), 2.47 (3H, s), 4.78 (2H, br. s) . 4. 98-5.03 (1H, m), 7.01 (1H, d, J = 3. 8Hz), 6.43 (1H, br. s), 6.60 (1H, br. s), 6.9 5-7. 11 (1H, m), 7.17 (1H, dd, J = 6.8, 4. 8Hz), 7. 50 (1H, t, J = 8.0H z). 7.57 (1H, m), 8.52 (1H, m) |

Now, Test Examples will be described.

TEST EXAMPLE 1

Test on controlling effects against green peach aphid (Myzus persicae)

A Japanese radish leaf was inserted in a test tube in which water was put, and about 20 first instar nymphs of green peach aphid were released on the leaf. On the next day, the number of nymphs parasitic on the leaf was counted, and then the leaf was dipped for about 10 seconds in an insecticidal solution prepared to bring the concentration of the compound of the present invention to 800 ppm, dried in air and left in a constant temperature chamber at 25°C with lightening. Survived nymphs were counted 5 days after the treatment, and the mortality was calculated by the following equation. The insects that dropped from the leaf or were moribund were included in the number of dead. The test was carried out with respect to the above-mentioned compound Nos. 1, 2, 4, 6, 9, 10, 13, 16, 17, 18, 19, 20, 22, 24, 27, 32, 34, 35, 38, 40, 42, 43, 45, 48, 53-55, 57, 62-64, 68, 70, 71, 73, 74, 77-81, 83, 86-107, 112, 114, 116-118, 121, 123, 124, 128, 133, 134, 136, 137, 139-142, 145-149, 151, 152, 154, 155, 157, 161, 162, 164, 165, 167, 168, 173-175, 177, 178, 182-186, 191, 192, 200-206, 208, 210, 211, 213-215, 217-220, 222, 223, 225-230, 231, 233-235, 239, 240, 242, 248, 255-264, 270, 271, 280, 284, 287, 288, 290-293, 295, 297-300, 303, 304, 306-314, 316-321, 329, 331 and 332, whereby all compounds showed a mortality of at least 90%. Mortality (%)=(1-(number of survived insects/number of treated insects))×100

TEST EXAMPLE 2

Test on controlling effects against common cutworm (Spodoptera litura)

A cabbage leaf disk was dipped for about 10 seconds in an insecticidal solution prepared to bring the concentration of the compound of the present invention to 800 ppm and dried in air. In a Petri dish having a diameter of 9 cm, a wet filter paper was placed, and the dried cabbage leaf fragment was placed thereon. Then, 10 second-third instar larvae of common cutworm were released thereon and after putting a cover on the Petri dish, left in a constant temperature chamber at 25°C with lightening. On the fifth day after the release, dead larvae were counted, and the mortality was calculated by the following equation. Moribund larvae were included in the number of dead. The test was carried out with respect to the above-mentioned Compound Nos. 1, 2, 4, 6, 9-11, 16, 17, 19, 20, 22-24, 42, 43, 45, 48, 50, 53-57, 59, 63, 64, 67-71, 73, 74, 76, 77, 79, 80, 83, 86, 89, 92, 93, 95-100, 102, 103, 105, 106, 109, 112, 114, 116-118, 121, 124, 125, 133, 135, 137, 139, 140, 142, 145-149, 151, 154, 155, 157, 161, 162, 164-168, 173-180, 182-186, 191, 192, 194, 198, 201, 202, 204-208, 210, 211, 213, 214, 217-225, 228, 229, 231-235, 238-240, 242, 248, 250, 251, 252, 255-257, 259-263, 266, 270, 277, 280, 285, 287-293, 295, 297-300, 303-306, 308-314, 316-321, 326, 327, 329, 331 and 332, whereby all compounds showed a mortality of at least 90%.

Mortality (%) = (Number of dead larvae/number of released larvae) × 100

TEST EXAMPLE 3

Test on adults of two-spotted spider mite (Tetranychus urticae)

An insecticidal solution was prepared to bring the concentration of the compound of the present invention to 800 ppm. A kidney bean having only one primordial leaf left, was transplanted to a pot (diameter: 8 cm, height: 7 cm), and 20 adults of two-spotted spider mite were released thereon. Together with the kidney bean leaf, they were dipped in the above insecticidal solution, dried in air and then left in a constant temperature chamber at 25°C with lightening.

On the second or third day after the treatment, dead adults were counted, and the mortality of adults was calculated by the following equation. Adults that dropped from the leaf or were moribund were included in the number of dead. The test was carried out with respect to the above-mentioned Compound No. 1-4, 6, 9, 10, 13, 16-19, 24, 27, 35, 42, 43, 45, 48, 53-55, 57, 62-64, 68, 70-74, 77-80, 83, 86-90, 92-107, 109, 110, 112, 114, 116, 118, 121, 133-135, 137, 139, 140, 141, 142, 146-149, 151, 152, 154, 155, 157, 161, 162, 164-168, 171, 173-176, 180-187, 191-193, 196, 198, 199-201, 204-206, 208, 210, 211, 213-215, 217-223, 225-228, 230, 231, 234-236, 240-242, 248, 255-263, 270, 271, 272, 279, 280, 284, 285, 287-293, 295, 297-300, 303-306, 308-314, 316-321, 324-326, 329, 331 and 332, whereby all compounds showed a mortality of adults of at least 90%.

Mortality of adults (%) = (Number of dead two-spotted spider mites/Number of treated two-spotted spider mites) $\times$ 100

TEST EXAMPLE 4

Test on controlling effects against Haemaphysalis longicornis

On an inner surface of Petri dish having a diameter of 9 cm, 1 ml of a solution of the compound of the present invention in acetone (concentration: 10 μg/ml) is dropped by a micro pipette. After the inner surface of the Petri dish is dried, 60 to 180 Larval ticks are put, and the Petri dish is covered with a polyethylene sheet and sealed by a rubber band. The number of ticks knocked down after contact with the compound is counted, whereby most of the compounds of the present invention will knock down Haemaphysalis longicornis.

TEST EXAMPLE 5

Test on controlling effects against Haemaphysalis longicornis employing a dog

50 Young mites of Haemaphysalis longicornis are released on the auricle of a dog (Beagle, 8 month old) and artificially parasitized. On the second day after being parasitized, the number of parasitic ticks is counted, and then a formulated compound of the present invention is spotted on the back of neck in an amount of 10 mg/kg. After administration of the drug, observation is continued up to the fifth day, whereby the number of parasitic ticks, the number of dropped ticks and the life or death of the dropped ticks are determined. The dog is independently taken care in a separate cage, permitted to freely drink tap water and fed with a predetermined amount of a dog food once a day. As a result, the compounds of the present invention are effective to kill or drop the parasitic Haemaphysalis longicornis.

TEST EXAMPLE 6

Test on controlling effects against cat flea (Ctenocephalides felis)employing a dog

100 Non-blood sucked adults of cat flea within three days after adult emergence, are released on the dorsal fur of a dog (Beagle, 8 month old) and artificially parasitized, and on the back of neck, a formulated compound of the present invention is spotted on at a dose of 10 mg/kg. On the third day after administration of the compound, the cat flea is recovered by means of a flea catching comb, and the parasitized number is counted. The dog is individually taken care in a separate cage, permitted to freely drink tap water and fed with a predetermined amount of a dog food once a day. As a result, the compound of the present invention is effective to control the parasitizing of cat flea.

Now, Formulation Examples are described below.

FORMULATION EXAMPLE 1

| | | |
|---|---|---|
| (1) | Compound of the present invention | 20 parts by weight |
| (2) | Clay | 70 parts by weight |
| (3) | White carbon | 5 parts by weight |
| (4) | Sodium polycarboxylate | 3 parts by weight |
| (5) | Sodium alkylnaphthalene sulfonate | 2 parts by weight |

The above components are uniformly mixed to obtain a wettable powder.

FORMULATION EXAMPLE 2

| | | |
|---|---|---|
| (1) | Compound of the present invention | 5 parts by weight |
| (2) | Talc | 60 parts by weight |
| (3) | Calcium carbonate | 34.5 parts by weight |
| (4) | Liquid paraffin | 0.5 part by weight |

The above components are uniformly mixed to obtain a dust.

FORMULATION EXAMPLE 3

| | | |
|---|---|---|
| (1) | Compound of the present invention | 20 parts by weight |
| (2) | N,N-dimethylacetamide | 20 parts by weight |
| (3) | Polyoxyethylene tristyryl phenyl ether | 10 parts by weight |
| (4) | Calcium dodecylbenzene sulfonate | 2 parts by weight |
| (5) | Xylene | 48 parts by weight |

The above components are uniformly mixed and dissolved to obtain an emulsifiable concentrate.

FORMULATION EXAMPLE 4

| | | |
|---|---|---|
| (1) | Clay | 68 parts by weight |
| (2) | Sodium lignin sulfonate | 2 parts by weight |
| (3) | Polyoxyethylene alkylaryl sulfate | 5 parts by weight |
| (4) | White carbon | 25 parts by weight |

The mixture of the above components is mixed with compound of the present invention in a weight ratio of 4: 1 to obtain a wettable powder.

FORMULATION EXAMPLE 5

| | | |
|---|---|---|
| (1) | Compound of the present invention | 50 parts by weight |
| (2) | Sodium alkylnaphthalene sulfonate condensation product of formaldehyde | 2 parts by weight |
| (3) | Silicone oil | 0.2 part by weight |
| (4) | Water | 47.8 parts by weight |

The above components are uniformly mixed and pulverized to obtain a base liquid, and

| | | |
|---|---|---|
| (5) | Sodium polycarboxylate | 5 parts by weight |
| (6) | Anhydrous sodium sulfate | 42.8 parts by weight |

are added, and the mixture is uniformly mixed, granulated and dried to obtain water-dispersible granules.

FORMULATION EXAMPLE 6

| | | |
|---|---|---|
| (1) | Compound of the present invention | 5 parts by weight |
| (2) | Polyoxyethylene octyl phenyl ether | 1 part by weight |
| (3) | Polyoxyethylene alkyl ether phosphoric acid ester | 0.1 part by weight |
| (4) | Granular calcium carbonate | 93.9 parts by weight |

The above components (1) to (3) are preliminarily uniformly mixed and diluted with a proper amount of acetone, and then the mixture is sprayed onto the component (4), and acetone is removed to obtain granules.

FORMULATION EXAMPLE 7

| | | |
|---|---|---|
| (1) | Compound of the present invention | 2.5 parts by weight |
| (2) | N,N-dimethylacetamide | 2.5 parts by weight |
| (3) | Soybean oil | 95.0 parts by weight |

The above components are uniformly mixed and dissolved to obtain an ultra low volume formulation.

FORMULATION EXAMPLE 8

| | | |
|---|---|---|
| (1) | Compound of the present invention | 40 parts by weight |
| (2) | Potassium polyoxyethylene styryl phenyl ether phosphate | 4 parts by weight |
| (3) | Silicone oil | 0.2 part by weight |
| (4) | Xanthan gum | 0.1 part by weight |
| (5) | Ethylene glycol | 5 parts by weight |
| (6) | Water | 50.7 parts by weight |

The above components are uniformly mixed and pulverized to obtain a water-based suspension concentrate.

FORMULATION EXAMPLE 9

| | | |
|---|---|---|
| (1) | Compound of the present invention | 10 parts by weight |
| (2) | Diethylene glycol monoethyl ether | 80 parts by weight |
| (3) | Polyoxyethylene alkyl ether | 10 parts by weight |

The above components are uniformly mixed to obtain a soluble concentrate.

INDUSTRIAL APPLICABILITY

The pesticide containing a novel pyridyl-methanamine derivative or its salt as an active ingredient of the present invention is excellent in the effect, the dosage, etc. as compared with conventional products, and has a very high controlling effect with a low dosage and is thereby applicable to control of pests, and particularly it is highly industrially applicable as an agricultural and horticultural pesticide and as a pesticide against parasites on animals.

The entire disclosure of Japanese Patent Application No. 2006-170283 filed on June 20, 2006 including specification, claims, drawings and summary is hereby referred to.

## Claims

1. A pyridyl-methanamine derivative represented by the formula (I) or its salt:

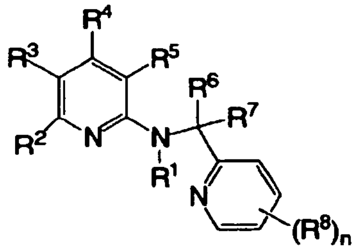

( I )

wherein $R^1$ is hydrogen, alkyl which may be substituted by $R^b$, alkenyl which may be substituted by $R^b$, alkynyl which may be substituted by $R^b$, aryl, cyano, N=CH$R^c$, O$R^c$, $S(O)_pR^c$=, COS$R^c$, COO$R^c$, CO$R^c$, or a heterocyclic group which may be substituted by alkyl or haloalkyl; each of $R^2$ and $R^3$ which are independent of each other, is hydrogen, halogen, cyano, nitro, alkyl which may be substituted by $R^8$, cycloalkyl, alkenyl, alkynyl, aryl, a heterocyclic group, $NR^aR^c$, $OR^a$, $SR^a$, $COR^a$, $COOR^a$, $CONR^aR^c$, CH=NO$R^a$, $SO_2R^a$ or $SOR^a$ ; $R^4$ is trifluoromethyl or chlorodifluoromethyl; $R^5$ is hydrogen, halogen, cyano, nitro, alkyl which may be substituted by $R^8$, alkenyl which may be substituted by $R^3$, alkynyl which may be substituted by $R^8$, $OR^a$, $SR^a$, $NR^aR^c$, $COOR^a$ or $COR^a$; each of $R^6$ and $R^7$ which are independent of each other, is hydrogen, cyano, alkyl, haloalkyl or cycloalkyl, or $R^6$ and $R^7$ may together form $C_{3-6}$ cycloalkyl which may be substituted by halogen; $R^8$ is alkyl, cycloalkyl, alkoxyalkyl, alkoxyalkoxyalkyl, hydroxyalkyl, halogen, haloalkyl, cyano, nitro, aryl which may be substituted by halogen, a heterocyclic group which may be substituted by halogen, heterocyclic oxy which may be substituted by halogen, $CONR^aR^c$, $COR^c$, $COOR^c$, $NR^aR^c$ or $OR^a$; $R^a$ is hydrogen, alkyl, cycloalkyl, haloalkyl or heterocyclic alkyl; $R^b$ is halogen, aryl which may be substituted by $R^8$, a heterocyclic group which may be substituted by $R^8$, heterocyclic oxy which may be substituted by $R^8$, heterocyclic thio which may be substituted by $R^8$, cyano, $NR^aR^c$, NHCOO$R^a$, $COR^c$, $COOR^c$, $CONR^aR^c$, alkoxyalkoxy, $OR^a$ or S(O)p$R^a$; $R^c$ is hydrogen, alkyl, haloalkyl, cycloalkyl, alkoxyalkyl, hydroxyalkyl, aryl which may be substituted by halogen, or a heterocyclic group which may be substituted by haloalkyl; n is an integer of from 0 to 4, p is an integer of from 0 to 2, in the $NR^aR^c$ moiety in each of the above substituents, $R^a$ and $R^c$ may together form a 5- or 6-membered heterocyclic ring together with the nitrogen atom to which they are bonded, provided that N-(2-pyridylmethyl)-3,5,6-trichloro-4-(trifluoromethyl)-2-pyridylamine is excluded.

2. The pyridyl-methanamine derivative or its salt according to Claim 1, wherein $R^1$ is hydrogen, alkyl which may be substituted by $R^b$, alkenyl which may be substituted by $R^b$, alkynyl which may be substituted by $R^b$, aryl, a heterocyclic group which may be substituted by haloalkyl, N=CH$R^c$, $OR^c$, COS$R^c$ or $COR^c$; each of $R^2$ and $R^3$ which are independent of each other, is hydrogen, halogen, cyano, nitro, alkyl which may be substituted by $R^8$, cycloalkyl, alkenyl, alkynyl, aryl, a heterocyclic group, $NR^aR^c$, $OR^a$, $SR^a$, $COR^A$, $COOR^a$, $CONR^aR^c$, $SO_2R^a$ or $SOR^a$; $R^5$ is hydrogen, halogen, cyano, nitro, alkyl which may be substituted by $R^8$, $COOR^a$ or $COR^a$; each of $R^6$ and $R^7$ which are independent of each other, is hydrogen, cyano, alkyl, haloalkyl or cycloalkyl; $R^8$ is alkyl, cycloalkyl, alkoxyalkyl, alkoxyalkoxyalkyl, hydroxyalkyl, halogen, haloalkyl, cyano, nitro, $NR^aR^c$ or $OR^a$; $R^a$ is hydrogen, alkyl, cycloalkyl or haloalkyl; $R^b$ is halogen, aryl which may be substituted by $R^8$, a heterocyclic group which may be substituted by $R^8$, cyano, $NR^aR^c$, NHCOO$R^a$, $OR^a$ or $SR^a$; and $R^c$ is hydrogen, alkyl, cycloalkyl, aryl or a heterocyclic group which may be substituted by haloalkyl.

3. The pyridyl-methanamine derivative or its salt according to Claim 2, wherein each of $R^2$ and $R^3$ which are independent of each other, is hydrogen, halogen, cyano, nitro, alkyl, haloalkyl, alkenyl, alkynyl, aryl, a heterocyclic group, $NR^aR^c$, $OR^a$, $SR^a$, $COR^a$ or $SOR^a$; $R^5$ is hydrogen, halogen or $COR^a$; $R^8$ is alkyl, halogen, haloalkyl, alkoxyalkoxyalkyl, hydroxyalkyl, cyano, $NR^aR^c$ or $OR^a$; $R^a$ is hydrogen, alkyl or haloalkyl; $R^b$ is halogen, aryl, a heterocyclic group which may be substituted by $R^8$, cyano, $NR^aR^c$, $OR^a$ or $SR^a$; and $R^c$ is hydrogen, alkyl, aryl or a heterocyclic group.

4. The pyridyl-methanamine derivative or its salt according to Claim 2, wherein $R^1$ is hydrogen, alkyl which may be substituted by $R^b$, alkenyl which may be substituted by $R^b$, alkynyl, aryl, a heterocyclic group which may be substituted by haloalkyl, $OR^c$ or $COR^c$; $R^2$ is hydrogen, halogen, cyano, alkyl, haloalkyl, alkynyl, aryl, $NR^aR^c$, $OR^a$ or $SR^a$; $R^3$ is hydrogen, halogen, cyano, nitro, haloalkyl, aryl, $NR^aR^c$, $SR^a$ or $COR^a$; $R^4$ is trifluoromethyl; $R^5$ is hydrogen, halogen or $COR^a$; each of $R^6$ and $R^7$ which are independent of each other, is hydrogen, alkyl or cycloalkyl; $R^8$ is

alkyl, halogen, haloalkyl, alkoxyalkoxyalkyl, hydroxyalkyl, cyano or $OR^a$; $R^a$ is hydrogen or alkyl; $R^b$ is halogen, aryl, a heterocyclic group which may be substituted by $R^8$, $OR^a$ or $NR^aR^c$; $R^c$ is hydrogen, alkyl, aryl or a heterocyclic group.

**5.** A method for producing a pyridyl-methanamine derivative or its salt as defined in Claim 1, which comprises

(1) reacting a compound represented by the formula (II):

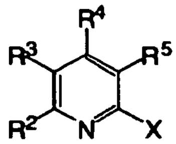

(II)

wherein $R^2$, $R^3$ , $R^4$ and $R^5$ are as defined above, and X is halogen, with a compound represented by the formula (III):

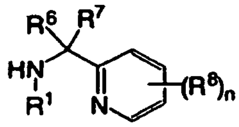

(III)

wherein $R^1$, $R^6$ , $R^7$, $R^8$ and n are as defined above ; or
(2) reacting a compound represented by the formula (V-1):

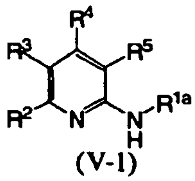

(V-1)

wherein $R^{1a}$ is alkyl which may be substituted by $R^b$, alkenyl which may be substituted by $R^b$, alkynyl which may be substituted by $R^b$, aryl, a heterocyclic group which may be substituted by alkyl or haloalkyl, $N=CHR^c$, $OR^c$, $S(O)_pR^c$, $COSR^c$, $COOR^c$ or $COR^c$, and $R^b$, $R^c$, p, $R^2$, $R^3$, $R^4$ and $R^5$ are as defined above, with a compound represented by the formula (VI):

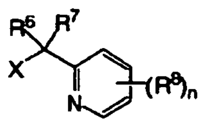

(VI)

wherein $R^6$, $R^7$, $R^8$, X and n are as defined above; or
(3) reacting a compound represented by the formula (I-1):

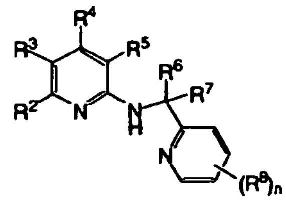

(I-1)

wherein $R^2$, $R^3$, $R^4$, $R^5$, $R^6$, $R^7$, $R^8$ and n are as defined above, with a compound represented by the formula (VII): $R^{1a}$-X, wherein $R^{1a}$ and X are as defined above.

**6.** A pesticide comprising, as an active ingredient, a pyridyl-methanamine derivative represented by the formula (I) or its salt:

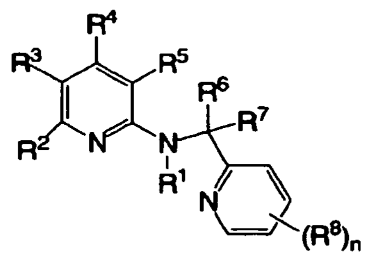

( I )

wherein $R^1$ is hydrogen, alkyl which may be substituted by $R^b$, alkenyl which may be substituted by $R^b$, alkynyl which may be substituted by $R^b$, aryl, cyano, N=CHR$^c$, OR$^c$, $S(O)_pR^c$=, COSR$^c$, COOR$^c$, COR$^c$, or a heterocyclic group which may be substituted by alkyl or haloalkyl; each of $R^2$ and $R^3$ which are independent of each other, is hydrogen, halogen, cyano, nitro, alkyl which may be substituted by $R^8$, cycloalkyl, alkenyl, alkynyl, aryl, a heterocyclic group, $NR^aR^c$, $OR^a$, $SR^a$, $COR^a$, $COOR^a$, $CONR^aR^c$, CH=NOR$^a$, $SO_2R^a$ or $SOR^a$; $R^4$ is trifluoromethyl or chlorodifluoromethyl; $R^5$ is hydrogen, halogen, cyano, nitro, alkyl which may be substituted by $R^8$, alkenyl which may be substituted by $R^8$, alkynyl which may be substituted by $R^8$, $OR^a$, $SR^a$, $NR^aR^c$, $COOR^a$ or $COR^a$; each of $R^6$ and $R^7$ which are independent of each other, is hydrogen, cyano, alkyl, haloalkyl or cycloalkyl, or $R^6$ and $R^7$ may together form $C_{3-6}$ cycloalkyl which may be substituted by halogen; $R^8$ is alkyl, cycloalkyl, alkoxyalkyl, alkoxyalkoxyalkyl, hydroxyalkyl, halogen, haloalkyl, cyano, nitro, aryl which may be substituted by halogen, a heterocyclic group which may be substituted by halogen, heterocyclic oxy which may be substituted by halogen, $CONR^aR^c$, $COR^c$, $COOR^c$, $NR^aR^c$ or $OR^a$; $R^a$ is hydrogen, alkyl, cycloalkyl, haloalkyl or heterocyclic alkyl; $R^b$ is halogen, aryl which may be substituted by $R^8$, a heterocyclic group which may be substituted by $R^8$, heterocyclic oxy which may be substituted by $R^8$, heterocyclic thio which may be substituted by $R^8$, cyano, $NR^aR^c$, $NHCOOR^a$, $COR^c$, $COOR^c$, $CONR^aR^c$, alkoxyalkoxy, $OR^a$ or $S(O)_pR^a$; $R^c$ is hydrogen, alkyl, haloalkyl, cycloalkyl, alkoxyalkyl, hydroxyalkyl, aryl which may be substituted by halogen, or a heterocyclic group which may be substituted by haloalkyl; n is an integer of from 0 to 4, p is an integer of from 0 to 2, in the $NR^aR^c$ moiety in each of the above substituents, $R^a$ and $R^c$ may together form a 5- or 6-membered heterocyclic ring together with the nitrogen atom to which they bond.

**7.** The pesticide according to Claim 6, wherein $R^1$ is hydrogen, alkyl which may be substituted by $R^b$, alkenyl which may be substituted by $R^b$, alkynyl which may be substituted by $R^b$, aryl, a heterocyclic group which may be substituted by haloalkyl, N=CHR$^c$, $OR^c$, $COSR^c$ or $COR^c$; each of $R^2$ and $R^3$ which are independent of each other, is hydrogen, halogen, cyano, nitro, alkyl which may be substituted by $R^8$, cycloalkyl, alkenyl, alkynyl, aryl, a heterocyclic group, $NR^aR^c$, $OR^a$, $SR^a$, $COR^a$, $COOR^a$, $CONR^aR^c$, $SO_2R^a$ or $SOR^a$; $R^5$ is hydrogen, halogen, cyano, nitro, alkyl which may be substituted by $R^8$, $COOR^a$ or $COR^a$; each of $R^6$ and $R^7$ which are independent of each other, is hydrogen, cyano, alkyl, haloalkyl or cycloalkyl; $R^8$ is alkyl, cycloalkyl, alkoxyalkyl, alkoxyalkoxyalkyl, hydroxyalkyl, halogen, haloalkyl, cyano, nitro, $NR^aR^c$ or $OR^a$; $R^b$ is halogen, aryl which may be substituted by $R^8$, a heterocyclic group which may be substituted by $R^8$, cyano, $NR^aR^c$, $NHCOOR^a$, $OR^a$ or $SR^a$; and $R^c$ is hydrogen, alkyl, cycloalkyl, aryl or a heterocyclic group which may be substituted by haloalkyl.

**8.** An agricultural and horticultural pesticide containing, as an active ingredient, the pyridyl-methanamine derivative represented by the formula (I) or its salt as defined in Claim 6.

9. An insecticide, miticide or nematicide containing, as an active ingredient, the pyridyl-methanamine derivative represented by the formula (I) or its salt as defined in Claim 6.

10. An agricultural or horticultural use of an effective amount of the pyridyl-methanamine derivative represented by the formula (I) or its salt as defined in Claim 6 for controlling a pest.

**Patentansprüche**

1. Pyridyl-Methanaminderivat der folgenden Formel (I) oder dessen Salz:

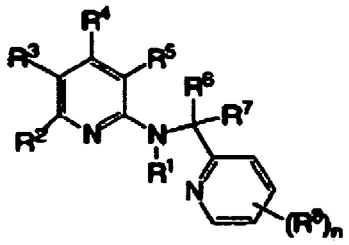

(I)

wobei $R^1$ für Wasserstoff, Alkyl, das mit $R^b$ substituiert sein kann, Alkenyl, das mit $R^b$ substituiert sein kann, Alkinyl, das mit $R^b$ substituiert sein kann, Aryl, Cyano, N=$CHR^c$, $OR^c$, $S(O)_pR^c$, $COSR^c$, $COOR^c$, $COR^c$, oder eine heterocyclische Gruppe, die mit Alkyl oder Haloalkyl substituiert sein kann, steht; jedes $R^2$ und $R^3$, die unabhängig voneinander sind, für Wasserstoff, Halogen, Cyano, Nitro, Alkyl, das mit $R^8$ substituiert sein kann, Cycloalkyl, Alkenyl, Alkinyl, Aryl, eine heterocyclische Gruppe, $NR^aR^c$, $OR^a$, $SR^a$, $COR^a$, $COOR^a$, $CONR^aR^c$, $CH=NOR^a$, $SO_2R^a$ oder $SOR^a$ steht; $R^4$ für Trifluormethyl oder Chlordifluormethyl steht; $R^5$ für Wasserstoff, Halogen, Cyano, Nitro, Alkyl, das mit $R^8$ substituiert sein kann, Alkenyl, das mit $R^8$ substituiert sein kann, Alkinyl, das mit $R^8$ substituiert sein kann, $OR^a$ $SR^a$, $NR^aR^c$, $COOR^a$ oder $COR^a$ steht; jedes $R^6$ und $R^7$, die unabhängig voneinander sind, für Wasserstoff, Cyano, Alkyl, Haloalkyl oder Cycloalkyl steht, oder $R^6$ und $R^7$ können zusammen ein $C_{3-6}$ Cycloalkyl bilden, das mit Halogen stubstituiert sein kann; $R^8$ für Alkyl, Cycloalkyl, Alkoxyalkyl, Alkoxyalkoxyalkyl, Hydroxyalkyl, Halogen, Haloalkyl, Cyano, Nitro, Aryl, das mit Halogen substituiert sein kann, eine heterocyclische Gruppe, die mit Halogen substituiert sein kann, Heterocyclusoxy, der mit Halogen substituiert sein kann, $CONR^aR^c$, $COR^c$, $COOR^c$, $NR^aR^c$ oder $OR^a$; $R^a$ für Wasserstoff, Alkyl, Cycloalkyl, Haloalkyl oder heterocyclisches Alkyl steht; $R^b$ für Halogen, Aryl, das mit $R^8$ substituiert sein kann, Heterocyclusoxy, der substituiert sein kann mit $R^8$, Heterocyclusthio, der mit $R^8$ substituiert sein kann, Cyano, $NR^aR^c$, $NHCOOR^a$, $COR^c$, $COOR^c$, $CONR^aR^c$, Alkoxyalkoxy, $OR^a$ oder $S(O)_pR^a$ steht; $R^c$ für Wasserstoff, Alkyl, Haloalkyl, Cycloalkyl, Alkoxyalkyl, Hydroxyalkyl, Aryl, das mit Halogen substituiert sein kann, oder eine heterocyclische Gruppe, die mit Haloalkyl substituiert sein kann, steht; n für eine ganze Zahl von 0 bis 4 steht, p für eine ganze Zahl von 0 bis 2 steht, wobei in der $NR^aR^c$ Gruppe in jedem der oben erwähnten Substituenten, $R^a$ und $R^c$ zusammen mit dem Stickstoffatom an das sie gebunden sind einen 5- oder 6-gliedrigen heterocyclischen Ring bilden können, mit der Maßgabe, dass N-(2-pyridylmethyl)-3,5,6-trichloro-4-(trifluoromethyl)-2-pyridylamin ausgeschlossen ist.

2. Das Pyridyl-Methanaminderivat oder dessen Salz nach Anspruch 1, wobei $R^1$ für Wasserstoff, Alkyl, das mit $R^b$ substituiert sein kann, Alkenyl, das mit $R^b$ substituiert sein kann, Alkinyl, das mit $R^b$ substituiert sein kann, Aryl, eine heterocyclische Gruppe, die mit Haloalkyl substituiert sein kann, N=$CHR^c$, $OR^c$, $COSR^c$, oder $COR^c$ steht; jedes aus $R^2$ und $R^3$, die unabhängig voneinander sind, für Wasserstoff, Halogen, Cyano, Nitro, Alkyl, das mit $R^8$ substituiert sein kann, Cycloalkyl, Alkenyl, Alkinyl, Aryl, eine heterocyclische Gruppe, $NR^aR^c$, $OR^a$, $SR^a$, $COR^a$, $CO0R^a$, $CONR^aR^c$, $SO_2R^a$ oder $SOR^a$ steht; $R^5$ für Wasserstoff, Halogen, Cyano, Nitro, Alkyl, das mit $R^8$ substituiert sein kann, $COOR^a$ oder $COR^a$ steht; jedes aus $R^6$ und $R^7$, die unabhängig voneinander sind für Wasserstoff, Cyano, Alkyl, Haloalkyl oder Cycloalkyl steht; $R^8$ für Alkyl, Cycloalkyl, Alkoxyalkyl, Alkoxyalkoxyalkyl, Hydroxyalkyl, Halogen, Haloalkyl, Cyano, Nitro, $NR^aR^c$ oder $OR^a$ steht; $R^a$ für Wasserstoff, Alkyl, Cycloalkyl oder Haloalkyl steht; $R^b$ für Halogen, Aryl, das mit $R^8$ substituiert sein kann, eine heterocyclische Gruppe, die mit $R^8$ substituiert sein kann, Cyano, $NR^aR^c$, $NHCOOR^a$ $OR^a$ oder $SR^a$ steht; und $R^c$ für Wasserstoff, Alkyl, Cycloalkyl, Aryl oder eine heterocyclische Gruppe, die mit Haloalkyl substituiert sein kann, steht.

3. Das Pyridyl-Methanaminderivat oder dessen Salz nach Anspruch 2, wobei jedes $R^2$ und $R^3$, die unabhängig voneinander sind, für Wasserstoff, Halogen, Cyano, Nitro, Alkyl, Haloalkyl, Alkenyl, Alkinyl, Aryl, eine heterocyclische

Gruppe, $NR^aR^c$, $OR^a$, $SR^a$, $COR^a$, oder $SOR^a$ steht; $R^5$ für Wasserstoff, Halogen, oder $COR^a$ steht; $R^8$ für Alkyl, Halogen, Haloalkyl, Alkoxyalkoxyalkyl, Hydroxyalkyl, Cyano, $NR^aR^c$ oder $OR^a$ steht; $R^a$ für Wasserstoff, Alkyl, oder Haloalkyl steht; $R^b$ für Halogen, Aryl, eine heterocyclische Gruppe, die mit $R^8$ substituiert sein kann, Cyano, $NR^aR^c$, $OR^a$ oder $SR^a$ steht; und $R^c$ für Wasserstoff, Alkyl, Aryl oder eine heterocyclische Gruppe steht.

**4.** Das Pyridyl-Methanaminderivat oder dessen Salz nach Anspruch 2, wobei $R^1$ für Wasserstoff, Alkyl, das mit $R^b$ substituiert sein kann, Alkenyl, das mit $R^b$ substituiert sein kann, Alkinyl, Aryl, eine heterocyclische Gruppe, die mit Haloalkyl substituiert sein kann, $OR^c$ oder $COR^c$ steht; $R^2$ für Wasserstoff, Halogen, Cyano, Alkyl, Haloalkyl, Alkinyl, Aryl, $NR^aR^c$, $OR^a$ oder $SR^a$ steht; $R^3$ für Wasserstoff, Halogen, Cyano, Nitro, Haloalkyl, Aryl, $NR^aR^c$, $SR^a$ oder $COR^a$ steht; $R^4$ für Trifluoromethyl steht; $R^5$ für Wasserstoff, Halogen oder $COR^a$ steht; jedes aus $R^6$ und $R^7$, die unabhängig voneinander sind, für Wasserstoff, Alkyl oder Cycloalkyl steht; $R^8$ für Alkyl, Halogen, Haloalkyl, Alkoxyalkoxyalkyl, Alkoxyalkyl, Cyano oder $OR^a$ steht; $R^a$ für Wasserstoff oder Alkyl steht; $R^b$ für Halogen, Aryl, eine heterocyclische Gruppe, die mit $R^8$ substituiert sein kann, $OR^a$ oder $NR^aR^c$ steht; $R^c$ für Wasserstoff, Alkyl, Aryl oder eine heterocyclische Gruppe steht.

**5.** Verfahren zur Herstellung eines Pyridyl-Methanaminderivats oder dessen Salz, wie es in Anspruch 1 definiert wird, das umfasst

(1) Umsetzung einer Verbindung der Formel (II):

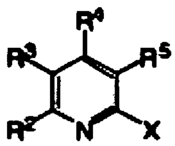

(II)

wobei $R^2$, $R^3$, $R^4$ und $R^5$, wie oben definiert sind, und X für Halogen steht, mit einer Verbindung der Formel (III):

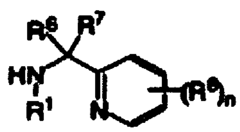

(III)

wobei $R^1$, $R^6$, $R^7$, $R^8$ und n wie oben definiert sind; oder
(2) Umsetzung einer Verbindung der Formel (V-1):

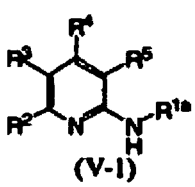

(V-1)

wobei $R^{1a}$ für Alkyl, das mit $R^b$ substituiert sein kann, Alkenyl, das mit $R^b$ substituiert sein kann, Alkinyl, das mit $R^b$ substituiert sein kann, Aryl, eine heterocyclische Gruppe, die mit Alkyl oder Haloalkyl substituiert sein kann, $N{=}CHR^c$, $OR^c$, $S(O)_pR^c$, $COSR^c$, $COOR^c$ oder $COR^c$, und $R^b$, $R^c$, p, $R^2$, $R^3$, $R^4$ und $R^5$ wie oben definiert sind, mit einer Verbindung der Formel (V1):

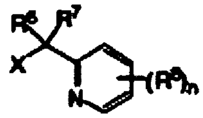

(VI)

wobei $R^6$, $R^7$, $R^8$, X und n wie oben definiert sind; oder
(3) Umsetzung einer Verbindung der Formel (I-1):

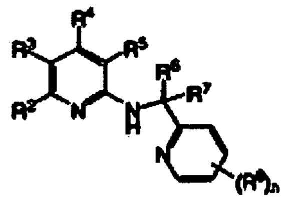

(I-1)

wobei $R^2$, $R^3$, $R^4$, $R^5$, $R^6$, $R^7$, $R^8$ und n wie oben definiert sind, mit einer Verbindung der Formel (VII): $R^{1a}$-X, wobei $R^{1a}$ und X wie oben definiert sind.

**6.** Pestizid, umfassend als wirksamen Bestandteil ein Pyridyl-Methanaminderivat der Formel (1) oder dessen Salz:

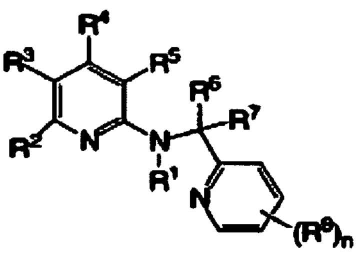

(1)

wobei $R^1$ für Wasserstoff, Alkyl, das mit $R^b$ substituiert sein kann, Alkenyl, das mit $R^b$ substituiert sein kann, Alkinyl, das mit $R^b$ substituiert sein kann, Aryl, Cyano, N=CHR$^c$, OR$^c$, S(O)$_p$R$^c$, COSR$^c$, COOR$^c$, COR$^c$, oder eine heterocyclische Gruppe, die mit Alkyl oder Haloalkyl substituiert sein kann, steht; jedes $R^2$ und $R^3$, die unabhängig voneinander sind, für Wasserstoff, Halogen, Cyano, Nitro, Alkyl, das mit $R^8$ substituiert sein kann, Cycloalkyl, Alkenyl, Alkinyl, Aryl, eine heterocyclische Gruppe, NR$^a$R$^c$, OR$^a$, SR$^a$, COR$^a$, COOR$^a$, CONR$^a$R$^c$, CH=NOR$^a$, SO$_2$R$^a$ oder SOR$^a$ steht; $R^4$ für Trifluormethyl oder Chlordifluormethyl steht; $R^5$ für Wasserstoff, Halogen, Cyano, Nitro, Alkyl, das mit $R^8$ substituiert sein kann, Alkenyl, das mit $R^8$ substituiert sein kann, Alkinyl, das mit $R^8$ substituiert sein kann, OR$^a$, SR$^a$, NR$^a$R$^c$, COOR$^a$ oder COR$^a$ steht; jedes $R^6$ und $R^7$, die unabhängig voneinander sind, für Wasserstoff, Cyano, Alkyl, Haloalkyl oder Cycloalkyl steht, oder $R^6$ und $R^7$ können zusammen ein $C_{3-6}$ Cycloalkyl bilden, das mit Halogen stubstituiert sein kann; $R^8$ für Alkyl, Cycloalkyl, Alkoxyalkyl, Alkoxyalkoxyalkyl, Hydroxyalkyl, Halogen, Haloalkyl, Cyano, Nitro, Aryl, das mit Halogen substituiert sein kann, eine heterocyclische Gruppe, die mit Halogen substituiert sein kann, Heterocyclusoxy, der mit Halogen substituiert sein kann, CONR$^a$R$^c$, COR$^c$, COOR$^c$, NR$^a$R$^c$ oder OR$^a$; $R^a$ für Wasserstoff, Alkyl, Cycloalkyl, Haloalkyl oder heterocyclisches Alkyl steht; $R^b$ für Halogen, Aryl, das mit $R^8$ substituiert sein kann, Heterocyclusoxy, der substituiert sein kann mit $R^8$, Heterocyclusthio, der mit $R^8$ substituiert sein kann, Cyano, NR$^a$R$^c$, NHCOOR$^a$, COR$^c$, COOR$^c$, CONR$^a$R$^c$, Alkoxyalkoxy, OR$^a$ oder S(O)$_p$R$^a$ steht; $R^c$ für Wasserstoff, Alkyl, Haloalkyl, Cycloalkyl, Alkoxyalkyl, Hydroxyalkyl, Aryl, das mit Halogen substituiert sein kann, oder eine heterocyclische Gruppe, die mit Haloalkyl substituiert sein kann, steht; n für eine ganze Zahl von 0 bis 4 steht, p für eine ganze Zahl von 0 bis 2 steht, wobei in der NR$^a$R$^c$ Gruppe in jedem der oben erwähnten Substituenten, $R^a$ und $R^c$ zusammen mit dem Stickstoffatom an das sie gebunden sind einen 5- oder 6-gliedrigen heterocyclischen Ring bilden können.

**7.** Das Pestizid nach Anspruch 6, wobei $R^1$ für Wasserstoff, Alkyl, das mit $R^b$ substituiert sein kann, Alkenyl, das mit $R^b$ substituiert sein kann, Alkinyl, das mit $R^b$ substituiert sein kann, Aryl, eine heterocyclische Gruppe, die mit Haloalkyl substituiert sein kann, N=CH$R^c$, O$R^c$, COS$R^c$, oder CO$R^c$ steht; jedes aus $R^2$ und $R^3$, die unabhängig voneinander sind, für Wasserstoff, Halogen, Cyano, Nitro, Alkyl, das mit $R^8$ substituiert sein kann, Cycloalkyl, Alkenyl, Alkinyl, Aryl, eine heterocyclische Gruppe, N$R^aR^c$, O$R^a$, S$R^a$, CO$R^a$, COO$R^a$, CON$R^aR^c$, $SO_2R^a$ oder SO$R^a$ steht; $R^5$ für Wasserstoff, Halogen, Cyano, Nitro, Alkyl, das mit $R^8$ substituiert sein kann, COO$R^a$ oder CO$R^a$ steht; jedes aus $R^6$ und $R^7$, die unabhängig voneinander sind für Wasserstoff, Cyano, Alkyl, Haloalkyl oder Cycloalkyl steht; $R^8$ für Alkyl, Cycloalkyl, Alkoxyalkyl, Alkoxyalkoxyalkyl, Hydroxyalkyl, Halogen, Haloalkyl, Cyano, Nitro, N$R^aR^c$ oder O$R^a$ steht; $R^8$ für Wasserstoff, Alkyl, Cycloalkyl oder Haloalkyl steht; $R^b$ für Halogen, Aryl, das mit $R^8$ substituiert sein kann, eine heterocyclische Gruppe, die mit $R^8$ substituiert sein kann, Cyano, N$R^aR^c$, NHCOO$R^a$, O$R^a$ oder S$R^a$ steht; und $R^c$ für Wasserstoff, Alkyl, Cycloalkyl, Aryl oder eine heterocyclische Gruppe, die mit Haloalkyl substituiert sein kann, steht.

**8.** Landwirtschaftliches bzw. gartenbauliches Pestizid, enthaltend das Pyridyl-Methanaminderivat der Formel (1), wie in Anspruch 6 definiert, oder dessen Salz als wirksamen Bestandteil.

**9.** Insektizid, Mitizid oder Nematizid, enthaltend das Pyridyl-Methanaminderivat der Formel (1), wie es in Anspruch 6 definiert wird, oder dessen Salz als aktiven Bestandteil.

**10.** Landwirtschaftliche oder gartenbauliche Verwendung einer wirksamen Menge des Pyridyl-Methanaminderivats der Formel (I), wie es in Anspruch 6 definiert wird, oder dessen Salzes zur Bekämpfung eines Schädlings.

## Revendications

**1.** Dérivé de pyridyl-méthanamine représenté par la formule (I) ou son sel :

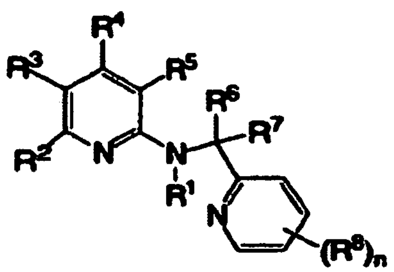

(I)

dans laquelle $R^1$ est l'hydrogène, un alkyle qui peut être substitué par $R^b$, un alcényle qui peut être substitué par $R^b$, un alcynyle qui peut être substitué par $R^b$, aryle, cyano, N=CH$R^c$, O$R^c$, $S(O)_pR^c$, COS$R^c$, COO$R^c$, CO$R^c$, ou un groupe hétérocyclique qui peut être substitué par un alkyle ou halogénoalkyle ; chacun de $R^2$ et $R^3$, qui sont indépendants l'un de l'autre, est l'hydrogène, un halogène, cyano, nitro, un alkyle qui peut être substitué par $R^8$, cycloalkyle, alcényle, alcynyle, aryle, un groupe hétérocyclique, N$R^aR^c$, O$R^a$, S$R^a$, CO$R^a$, COO$R^a$, CON$R^aR^c$, CH=NO$R^a$, $SO_2R^a$ ou SO$R^a$ ; $R^4$ est trifluorométhyle ou chlorodifluorométhyle ; $R^5$ est l'hydrogène, un halogène, cyano, nitro, un alkyle qui peut être substitué par $R^8$, un alcényle qui peut être substitué par $R^8$, un alcynyle qui peut être substitué par $R^8$, O$R^a$, S$R^a$, N$R^aR^c$, COO$R^a$ ou CO$R^a$ ; chacun de $R^6$ et $R^7$, qui sont indépendants l'un de l'autre, est l'hydrogène, cyano, alkyle, halogénoalkyle ou cycloalkyle, ou bien $R^6$ et $R^7$ peuvent former ensemble un cycloalkyle en $C_3$ à $C_6$ qui peut être substitué par un halogène ; $R^8$ est un alkyle, cycloalkyle, alcoxyalkyle, alcoxyalcoxyalkyle, hydroxyalkyle, halogène, halogénoalkyle, cyano, nitro, aryle qui peut être substitué par un halogène, un groupe hétérocyclique qui peut être substitué par un halogène, oxy hétérocyclique qui peut être substitué par un halogène, CON$R^aR^c$, CO$R^c$, COO$R^c$, N$R^aR^c$ ou O$R^a$ ; $R^a$ est l'hydrogène, un alkyle, cycloalkyle, halogénoalkyle ou alkyle hétérocyclique ; $R^b$ est un halogène, un aryle qui peut être substitué par $R^8$, un groupe hétérocyclique qui peut être substitué par $R^8$, un oxy hétérocyclique qui peut être substitué par $R^8$, un thio hétérocyclique qui peut être substitué par $R^8$, cyano, N$R^aR^c$, NHCOO$R^a$, CO$R^c$, COO$R^c$, CON$R^aR^c$, alcoxyalcoxy, O$R^a$ ou $S(O)_pR^a$ ; $R^c$ est l'hydrogène, un alkyle, halogénoalkyle, cycloalkyle, alcoxyalkyle, hydroxyalkyle, aryle qui peut être substitué par un halogène, ou un groupe hétérocyclique qui peut être substitué par un halogénoalkyle ; n est un entier de 0 à 4, p est un entier de 0 à 2, dans le fragment N$R^aR^c$ dans chacun des substituants ci-dessus, $R^a$ et

$R^c$ peuvent former ensemble un hétérocycle à 5 ou 6 chaînons conjointement avec l'atome d'azote auquel ils sont liés, à l'exclusion de la N-(2-pyridylméthyl)-3,5,6-trichloro-4-(trifluorométhyl)-2-pyridylamine.

**2.** Dérivé de pyridyl-méthanamine ou son sel selon la revendication 1, dans lequel $R^1$ est l'hydrogène, un alkyle qui peut être substitué par $R^b$, un alcényle qui peut être substitué par $R^b$, un alcynyle qui peut être substitué par $R^b$, aryle, un groupe hétérocyclique qui peut être substitué par un halogénoalkyle, N=CH$R^c$, O$R^c$, COS$R^c$ ou CO$R^c$ ; chacun de $R^2$ et $R^3$, qui sont indépendants l'un de l'autre, est l'hydrogène, un halogène, cyano, nitro, alkyle qui peut être substitué par $R^8$, cycloalkyle, alcényle, alcynyle, aryle, un groupe hétérocyclique, N$R^aR^c$, O$R^a$, S$R^a$, CO$R^a$, COO$R^a$, CON$R^aR^c$, $SO_2R^a$, ou SO$R^a$ ; $R^5$ est l'hydrogène, un halogène, cyano, nitro, alkyle qui peut être substitué par $R^8$ , COO$R^a$ ou CO$R^a$ ; chacun de $R^6$ et $R^7$, qui sont indépendants l'un de l'autre, est l'hydrogène, cyano, alkyle, halogénoalkyle ou cycloalkyle ; $R^8$ est un alkyle, cycloalkyle, alcoxyalkyle, alcoxyalcoxyalkyle, hydroxyalkyle, halogène, halogénoalkyle, cyano, nitro, N$R^aR^c$ ou O$R^a$ ; $R^a$ est l'hydrogène, alkyle, cycloalkyle ou halogénoalkyle ; $R^b$ est un halogène, un aryle qui peut être substitué par $R^8$, un groupe hétérocyclique qui peut être substitué par $R^8$, cyano, NR $R^c$, NHCOO$R^a$, O$R^a$ ou S$R^a$ ; et $R^c$ est l'hydrogène, alkyle, cycloalkyle, aryle ou un groupe hétérocyclique qui peut être substitué par un halogénoalkyle.

**3.** Dérivé de pyridyl-méthanamine ou son sel selon la revendication 2, dans lequel chacun de $R^2$ et $R^3$, qui sont indépendants l'un de l'autre, est l'hydrogène, un halogène, cyano, nitro, alkyle, halogénoalkyle, alcényle, alcynyle, aryle, un groupe hétérocyclique, N$R^aR^c$, O$R^a$, S$R^a$, CO$R^a$ ou SO$R^a$ ; $R^5$ est l'hydrogène, un halogène ou CO$R^a$ ; $R^8$ est un alkyle, halogène, halogénoalkyle, alcoxyalcoxyalkyle, hydroxyalkyle, cyano, N$R^aR^c$ ou O$R^a$ ; $R^a$ est l'hydrogène, alkyle ou halogénoalkyle ; $R^b$ est un halogène, aryle, un groupe hétérocyclique qui peut être substitué par $R^8$, cyano, N$R^aR^c$, O$R^a$ ou S$R^a$ ; et $R^c$ est l'hydrogène, alkyle, aryle, ou un groupe hétérocyclique.

**4.** Dérivé de pyridyl-méthanamine ou son sel selon la revendication 2, dans lequel $R^1$ est l'hydrogène, un alkyle qui peut être substitué par $R^b$, un alcényle qui peut être substitué par $R^b$ alcynyle, aryle, un groupe hétérocyclique qui peut être substitué par un halogénoalkyle, O$R^c$ ou CO$R^c$ ; $R^2$ est l'hydrogène, un halogène, cyano, alkyle, halogénoalkyle, alcynyle, aryle, N$R^aR^c$, O$R^a$ ou S$R^a$ ; $R^3$ est l'hydrogène, un halogène, cyano, nitro, halogénoalkyle, aryle, N$R^aR^c$, S$R^a$ ou CO$R^a$ ; $R^4$ est trifluorométhyle ; $R^5$ est l'hydrogène, un halogène ou CO$R^a$ ; chacun de $R^6$ et $R^7$ qui sont indépendants l'un de l'autre, est l'hydrogène, alkyle ou cycloalkyle ; $R^3$ est un alkyle, halogène, halogénoalkyle, alcoxyalcoxyalkyle, hydroxyalkyle, cyano ou O$R^a$ ; $R^a$ est l'hydrogène ou alkyle ; $R^b$ est un halogène, aryle, un groupe hétérocyclique qui peut être substitué par $R^8$, O$R^a$ ou N$R^aR^c$ ; $R^c$ est l'hydrogène, alkyle, aryle ou un groupe hétérocyclique.

**5.** Procédé pour produire un dérivé de pyridyl-méthanamine ou son sel tel que défini dans la revendication 1, qui comprend

(1) la réaction d'un composé représenté par la formule (II) :

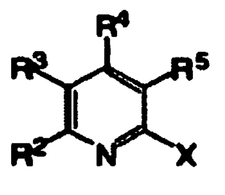

(II)

dans laquelle $R^2$, $R^3$, $R^4$ et $R^5$ sont tels que définis ci-dessus, et X est un halogène, avec un composé représenté par la formule (III) :

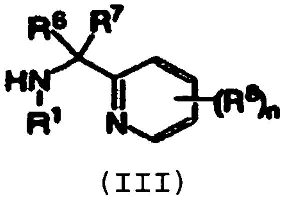

(III)

dans laquelle $R^1$, $R^6$, $R^7$, $R^8$ et n sont tels que définis ci-dessus ; ou
(2) la réaction d'un composé représenté par la formule (V-1) :

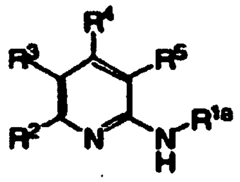

(V-I)

dans laquelle $R^{1a}$ est un alkyle qui peut être substitué par $R^b$, un alcényle qui peut être substitué par $R^b$, un alcynyle qui peut être substitué par $R^b$, aryle, un groupe hétérocyclique qui peut être substitué par alkyle ou halogénoalkyle, N=CHR$^c$, OR$^c$, S(O)$_p$R$^c$, COSR$^c$, COOR$^c$ ou COR$^c$, et $R^b$, $R^c$, p, $R^2$, $R^3$, $R^4$ et $R^5$ sont tels que définis ci-dessus,
avec un composé représenté par la formule (VI) :

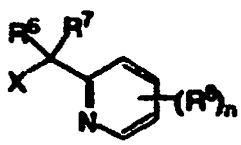

(VI)

dans laquelle $R^6$, $R^7$, $R^8$, X et n sont tels que définis ci-dessus ; ou
(3) la réaction d'un composé représenté par la formule (I-1) :

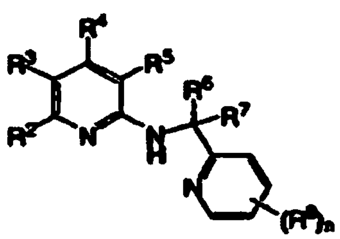

(I-1)

dans laquelle $R^2$, $R^3$, $R^4$, $R^5$, $R^6$, $R^7$, $R^8$ et n sont tels que définis ci-dessus,
avec un composé représenté par la formule (VII) : $R^{1a}$-X, dans laquelle $R^{1a}$ et X sont tels que définis ci-dessus.

**6.** Pesticide comprenant, à titre d'ingrédient actif, un dérivé de pyridyl-méthanamine représenté par la formule (I) ou son sel :

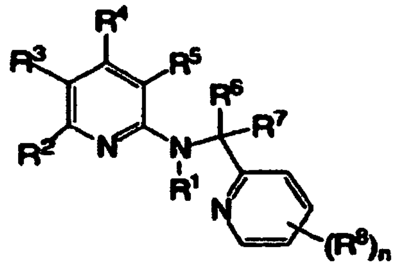

(I)

dans laquelle $R^1$ est l'hydrogène, un alkyle qui peut être substitué par $R^b$, un alcényle qui peut être substitué par $R^b$, un alcynyle qui peut être substitué par $R^b$, aryle, cyano, N=CHR$^c$ , OR$^c$, S(O)pR$^c$, COSR$^c$, COOR$^c$, COR$^c$, ou un groupe hétérocyclique qui peut être substitué par un alkyle ou halogénoalkyle ; chacun de $R^2$ et $R^3$, qui sont indépendants l'un de l'autre, est l'hydrogène, un halogène, cyano, nitro, un alkyle qui peut être substitué par $R^8$, cycloalkyle, alcényle, alcynyle, aryle, un groupe hétérocyclique, NR$^a$R$^c$, OR$^a$, SR$^a$, COR$^a$, COOR$^a$, CONR$^a$R$^c$, CH=NOR$^a$, $SO_2R^a$ ou SOR$^a$ ; $R^4$ est trifluorométhyle ou chlorodifluorométhyle ; $R^5$ est l'hydrogène, un halogène, cyano, nitro, un alkyle qui peut être substitué par $R^8$, un alcényle qui peut être substitué par $R^8$, un alcynyle qui peut être substitué par $R^8$, OR$^a$, SR$^a$, NR$^a$R$^c$, COOR$^a$ ou COR$^a$ ; chacun de $R^6$ et $R^7$ qui sont indépendants l'un de l'autre, est l'hydrogène, cyano, alkyle, halogénoalkyle ou cycloalkyle, ou bien $R^6$ et $R^7$ peuvent former ensemble un cycloalkyle en $C_3$ à $C_6$ qui peut être substitué par un halogène ; $R^8$ est un alkyle, cycloalkyle, alcoxyalkyle, alcoxyalcoxyalkyle, hydroxyalkyle, halogène, halogénoalkyle, cyano, nitro, aryle qui peut être substitué par un halogène, un groupe hétérocyclique qui peut être substitué par un halogène, oxy hétérocyclique qui peut être substitué par un halogène, CONR$^a$R$^c$, COR$^c$, COOR$^c$, NR$^a$R$^c$ ou OR$^a$ ; $R^a$ est l'hydrogène, un alkyle, cycloalkyle, halogénoalkyle ou alkyle hétérocyclique ; $R^b$ est un halogène, un aryle qui peut être substitué par $R^8$, un groupe hétérocyclique qui peut être substitué par $R^8$, un oxy hétérocyclique qui peut être substitué par $R^8$, un thio hétérocyclique qui peut être substitué par $R^8$ cyano, NR$^a$R$^c$, NHCOOR$^a$ , COR$^c$, COOR$^c$, CONR$^a$R$^c$, alcoxyalcoxy, OR$^a$ ou $S(O)_pR^a$ ; $R^c$ est l'hydrogène, un alkyle, halogénoalkyle, cycloalkyle, alcoxyalkyle, hydroxyalkyle, aryle qui peut être substitué par un halogène, ou un groupe hétérocyclique qui peut être substitué par un halogénoalkyle ; n est un entier de 0 à 4, p est un entier de 0 à 2, dans le fragment NR$^a$R$^c$ dans chacun des substituants ci-dessus, $R^a$ et $R^c$ peuvent former ensemble un hétérocycle à 5 ou 6 chaînons conjointement avec l'atome d'azote auquel ils sont liés.

**7.** Pesticide selon la revendication 6, dans lequel $R^1$ est l'hydrogène, un alkyle qui peut être substitué par $R^b$, un alcényle qui peut être substitué par $R^b$, un alcynyle qui peut être substitué par $R^b$, aryle, un groupe hétérocyclique qui peut être substitué par un halogénoalkyle, N=CHR$^c$, OR$^c$, COSR$^c$ ou COR$^c$ ; chacun de $R^2$ et $R^3$, qui sont indépendants l'un de l'autre, est l'hydrogène, un halogène, cyano, nitro, alkyle qui peut être substitué par $R^8$, cycloalkyle, alcényle, alcynyle, aryle, un groupe hétérocyclique, NR$^a$R$^c$, OR$^a$, SR$^a$, COR$^a$, COOR$^a$ , CONR$^a$R$^c$, $SO_2R^a$ ou SOR$^a$ ; $R^5$ est l'hydrogène, un halogène, cyano, nitro, alkyle qui peut être substitué par $R^8$, COOR$^a$ ou COR$^a$ ; chacun de $R^6$ et $R^7$, qui sont indépendants l'un de l'autre, est l'hydrogène, cyano, alkyle, halogénoalkyle ou cycloalkyle ; $R^8$ est un alkyle, cycloalkyle, alcoxyalkyle, alcoxyalcoxyalkyle, hydroxyalkyle, halogène, halogénoalkyle, cyano, nitro, NR$^a$R$^c$ ou OR$^a$ ; $R^b$ est un halogène, un aryle qui peut être substitué par $R^8$, un groupe hétérocyclique qui peut être substitué par $R^8$, cyano, NR$^a$R$^c$, NHCOOR$^a$, OR$^a$ ou SR$^a$ ; et $R^c$ est l'hydrogène, alkyle, cycloalkyle, aryle ou un groupe hétérocyclique qui peut être substitué par un halogénoalkyle.

**8.** Pesticide à usage agricole ou horticole contenant, à titre d'ingrédient actif, le dérivé de pyridyl-méthanamine représenté par la formule (I) ou son sel, tel que défini dans la revendication 6.

**9.** Insecticide, acaricide ou nématicide contenant, à titre d'ingrédient actif, le dérivé de pyridyl-méthanamine représenté par la formule (I) ou son sel, tel que défini dans la revendication 6.

**10.** Utilisation agricole ou horticole d'une quantité efficace du dérivé de pyridyl-méthanamine représenté par la formule (I) ou de son sel, tel que défini dans la revendication 6, pour maîtriser un nuisible.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0162233 A
- WO 0266470 A
- WO 0491518 A
- WO 9112228 A
- JP 3197460 A
- JP 2006170283 A

### Non-patent literature cited in the description

- *Chemistry Express,* 1992, vol. 7, 473-476